(19) Europäisches Patentamt
European Patent Office
Office européen des brevets

(11) EP 4 772 633 A1

(12) EUROPEAN PATENT APPLICATION
published in accordance with Art. 153(4) EPC

(43) Date of publication:
**08.07.2026 Bulletin 2026/28**

(21) Application number: **24853915.7**

(22) Date of filing: **15.10.2024**

(51) International Patent Classification (IPC):
***C12N 15/113*** *(2010.01)*

(52) Cooperative Patent Classification (CPC):
**C12N 15/113**

(86) International application number:
**PCT/CN2024/124919**

(87) International publication number:
**WO 2025/036511 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.10.2023 CN 202311341678**
**17.11.2023 CN 202311543411**

(71) Applicant: **Chengdu Gowell Biopharmaceutical Co., Ltd.**
**Chengdu, Sichuan 610093 (CN)**

(72) Inventors:
- **HUANG, Yi**
**Chengdu, Sichuan 610093 (CN)**
- **WEI, Chuanlai**
**Chengdu, Sichuan 610093 (CN)**
- **ZHOU, Wei**
**Chengdu, Sichuan 610093 (CN)**
- **CHEN, Youjin**
**Chengdu, Sichuan 610093 (CN)**
- **WU, Yueqi**
**Chengdu, Sichuan 610093 (CN)**
- **LV, Feilong**
**Chengdu, Sichuan 610093 (CN)**

(74) Representative: **Müller Schupfner & Partner Patent- und Rechtsanwaltspartnerschaft mbB (Muc)**
**Bavariaring 11**
**80336 München (DE)**

Remarks:
The complete document including Reference Table(s) and the Sequence Listing(s) can be downloaded from the EPO website

# (54) SIRNA, SIRNA CONJUGATE, PHARMACEUTICAL COMPOSITION AND USE THEREOF

(57) An siRNA that inhibits the expression of the angiotensinogen (AGT) gene, an siRNA conjugate, a pharmaceutical composition and the use thereof. The siRNA contains a sense strand and an antisense strand, wherein the antisense strand contains at least 19 consecutive nucleotides that differ by no more than three nucleotides from any one of the antisense strand sequences as shown in table 1. Further provided is a method for preventing and/or treating diseases associated with angiotensinogen dysregulation using at least one of the siRNA, the siRNA conjugate, and the pharmaceutical composition.

EP 4 772 633 A1

## Description

### TECHNICAL FIELD

The present disclosure pertains to the field of medical technology, in particular to an siRNA for inhibiting the expression of angiotensinogen (AGT) gene, a conjugate thereof, and a pharmaceutical composition. The present disclosure also relates to a kit comprising at least one of the siRNA, the conjugate thereof, and the pharmaceutical composition, a method of preventing and/or treating a disease associated with angiotensinogen dysregulation using at least one of the siRNA, the conjugate thereof, and the pharmaceutical composition, and use of at least one of the siRNA, the conjugate thereof, and the pharmaceutical composition in preparing a medicament for preventing and/or treating a disease associated with angiotensinogen dysregulation.

### BACKGROUND

The renin-angiotensin-aldosterone system (RAAS) plays a crucial role in the regulation of blood pressure, and the RAAS cascade begins with the secretion of renin by the juxtaglo-merular cells of the kidney into the circulation. AGT (Angiotensinogen) is the most upstream precursor in the renin-angiotensin-aldosterone system (RAAS), and its cascade effect has been confirmed in the regulation of blood pressure, for example, inhibiting the expression of AGT can exert a good antihypertensive effect.

Hypertension is a disease of persistently high blood pressure, which is also a major risk factor for inducing related diseases such as stroke, heart disease, hemangioma, and renal failure, having become one of the leading causes of death worldwide. Therefore, using antihypertensive drugs to treat hypertension, improve blood pressure control rates, and reduce patients' blood pressure levels have become an urgent need for hypertensive patients. However, despite the large number of antihypertensive drugs on the market, most patients cannot effectively lower their blood pressure levels by taking a single antihypertensive drug, but need to take multiple antihypertensive drugs together to achieve the therapeutic effect. Therefore, the development of more effective antihypertensive drugs is extremely urgent.

Small interfering RNA (siRNA) is a newly emerging and highly potent candidate research object that inhibits or blocks the expression of the target gene of interest in a sequence-specific manner based on the RNA interference mechanism, thereby achieving the purpose of treating diseases. Since siRNA recognizes the target RNA through complementary base pairing and sequence specifically, thereby it can achieve the cleavage of the target RNA, and achieve the purpose of downregulating the target RNA level and inhibiting the expression of the target gene. Therefore, siRNA has extremely broad application prospects. However, compared with traditional drugs, siRNA has poor stability and has the disadvantage of being easily degraded by nucleases when being administered systematically. In addition, it is necessary to avoid side effects such as off-target effects, immune stimulation and cytotoxicity while further improving the activity. Therefore, it has become an urgent problem needed to be solved to develop more candidate siRNAs that inhibit the expression of the AGT gene with good biological activity and/or stability in the blood and low cytotoxicity. At the same time, using the candidate siRNAs that inhibit the expression of the AGT gene to develop drugs capable of effectively preventing and/or treating diseases associated with angiotensinogen dysregulation has the necessity for clinical research and the practicality for commercialization.

### SUMMARY OF THE INVENTION

Based on the above status quo, the purpose of the present disclosure is to provide an siRNA capable of effectively inhibiting the expression of the AGT gene, or an siRNA conjugate obtained by conjugating the siRNA with a conjugate molecule, or a pharmaceutical composition comprising the siRNA as an active ingredient, or a kit comprising at least one of the siRNA, the siRNA conjugate or the pharmaceutical composition, use of at least one of the siRNA, the siRNA conjugate or the pharmaceutical composition in the preparation of a drug for preventing and/or treating diseases associated with angiotensinogen dysregulation, and a method of preventing and/or treating diseases associated with angiotensinogen dysregulation using at least one of the siRNA, the siRNA conjugate or the pharmaceutical composition. The AGT gene may be in cells, for example, cells in the body of a subject (e.g., a human).

In the first aspect, the present disclosure provides an siRNA that inhibits the expression of the AGT gene, comprising a sense strand and an antisense strand, wherein the antisense strand comprises at least 19 contiguous nucleotides that differ by no more than 3 nucleotides from any one of antisense strand sequences shown in Table 1. Unless otherwise specified, at least partial sequences of the sense strand and the antisense strand herein can complementarily bind.

In the second aspect, the present disclosure provides an siRNA conjugate, which is obtained by conjugating the siRNA described in the first aspect with a conjugate molecule. In the third aspect, the present disclosure provides a pharmaceutical composition comprising the siRNA described in the first aspect as an active ingredient and a pharmaceutically acceptable carrier.

In the fourth aspect, the present disclosure provides a kit comprising at least one of the siRNA described in the first aspect, the siRNA conjugate described in the second aspect, and the pharmaceutical composition described in the third aspect.

In the fifth aspect, the present disclosure provides use of at least one of the siRNA described in the first aspect, the siRNA conjugate described in the second aspect, and the pharmaceutical composition described in the third aspect in the preparation of a medicament for preventing and/or treating diseases associated with angiotensinogen dysregulation. Alternatively, the present disclosure provides use of at least one of the siRNA described in the first aspect, the siRNA conjugate described in the second aspect, and the pharmaceutical composition described in the third aspect for preventing and/or treating diseases associated with angiotensinogen dysregulation.

In the sixth aspect, the present disclosure provides a method of preventing and/or treating diseases associated with angiotensinogen dysregulation, comprising administering to a subject in need thereof at least one of the siRNA described in the first aspect, the siRNA conjugate described in the second aspect, and the pharmaceutical composition described in the third aspect.

In the seventh aspect, the present disclosure provides the siRNA described in the first aspect and/or the siRNA conjugate described in the second aspect and/or the pharmaceutical composition described in the third aspect for use in preventing and/or treating diseases associated with angiotensinogen dysregulation.

The present disclosure provides an siRNA, a conjugate thereof, a pharmaceutical composition and use thereof, which have the following beneficial effects: the siRNA, siRNA conjugate and pharmaceutical composition provided in the present disclosure have acceptable AGT gene inhibitory activity and are expected to have good druggability.

Other features and advantages of the present disclosure will be described in details in the subsequent specific embodiments.

## EMBODIMENTS OF THE INVENTION

Hereinafter, the specific embodiments of the present disclosure are described in details. It should be understood that the specific embodiments described herein are only used to illustrate and explain the present disclosure, and are not intended to limit the present disclosure. Instead, various modifications, alternations or changes can be made based on the spirit and concept of the present disclosure, and the contents after these modifications, alternations or changes still fall within the scope of the present disclosure.

Unless otherwise specified, the term "AGT" in the present disclosure includes human AGT, the amino acid and complete coding sequence of which can be found, for example, in GenBank Accession No. GI: 188595658 (NM_001382817.3; SEQ ID NO: 1); cynomolgus monkey (*Macaca fascicularis*) AGT, the amino acid and complete coding sequence of which can be found, for example, in GenBank Accession No. GI: 90075391 (AB170313.1; SEQ ID NO: 2); mouse (*Mus musculus*) AGT, the amino acid and complete coding sequence of which can be found, for example, in GenBank Accession No. GI: 113461997 (NM_007428.3; SEQ ID NO: 3); and rat (*Rattus norvegicus*) AGT, the amino acid and complete coding sequence of which can be found, for example, in GenBank Accession No. GI: 51036672 (NM_134432; SEQ ID NO: 4). Other instances of AGT mRNA sequences can also be easily obtained using publicly available databases (e.g., GenBank, UniProt, OMIM, and the *Macaca* Genome Project website).

Unless otherwise specified, capital letters C, G, U, A in the present disclosure represent the base composition of ribonucleotides; dC, dG, dT, dA represent the base composition of deoxyribonucleotides; lowercase letter m represents that the nucleotide adjacent to the right side of the letter m is a 2'-methoxy modified nucleotide; the identifier i2F represents that the nucleotide adjacent to the right side of the identifier i2F is a 2'-fluoro modified nucleotide; the identifier * represents that the two nucleotides adjacent to the left and right sides of the identifier * are linked via a phosphorothioate group; the identifier # represents that the two nucleotides adjacent to the left and right sides of the identifier # are linked via a mesyl phosphoramidate (MsPA) bond; the identifier (E)-VP represents that the nucleotide adjacent to the right side of the identifier (E)-VP is a (E)-vinylphosphonate modified nucleotide; the underline "_" represents that the nucleotide shown by the underline is a glycerol nucleic acid (GNA); "T" is the S-isomer of thymidine-glycol nucleic acid (GNA), and their structures can be shown as follows (wherein Base represents a base):

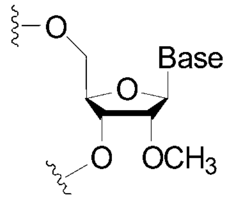

2'-methoxy modified nucleotide

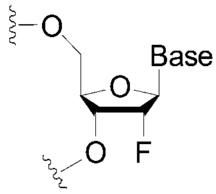

2'-fluoro modified nucleotide

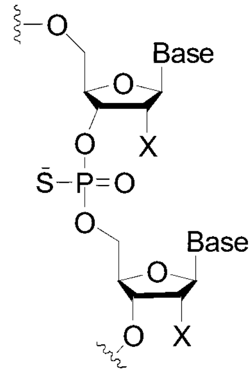

phosphorothioate linkage

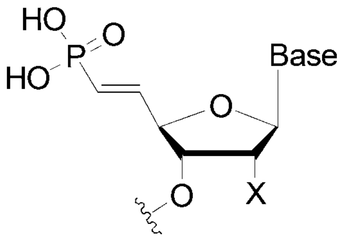

(*E*)-vinylphosphonate modified nucleotide

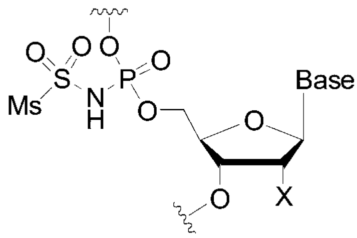

MsPA

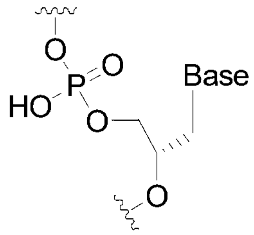

glycerol nucleic acid (GNA).

Unless otherwise specified, in the context of siRNA, "complementary" in the present disclosure means that in a siRNA duplex molecule, the bases of one strand are each paired with the bases on the other strand in a complementary manner, or the bases of the antisense strand of the siRNA are each paired with the bases on the target gene or target sequence in a complementary manner. That is, when A/dA is paired with U/dT, and C/dC is paired with G/dG, the two strands are considered to be complementary. "Complementary" as described in the present disclosure may include base pairing formed by non-Watson-Crick base pairing and/or non-natural or modified nucleotides, as long as they hybridize and are capable of forming a double-stranded structure. Correspondingly, unless otherwise specified, in the context of siRNA, "mismatch" in the present disclosure means that in a siRNA duplex molecule, the bases at corresponding positions are not paired in a complementary form, or the bases of the antisense strand of the siRNA are not paired with the bases at corresponding positions on the target gene or target sequence in a complementary form.

Unless otherwise specified, "substantially complementary pairing" in the present disclosure means that the number of mismatched nucleotides is not more than 3, for example, the number of mismatched nucleotides is 2, the number of mismatched nucleotides is 1.

Unless otherwise specified, "conjugation" in the present disclosure means that two or more chemical moieties are linked to each other by means of covalent bonding. "Conjugate" means a compound formed by covalent bonding between two or more chemical moieties; and "siRNA conjugate" means a compound formed by covalent bonding of one or more chemical moieties to an siRNA. It should be clarified here that each chemical moiety can be directly linked to the siRNA or linked to the siRNA through a linker. For example, the conjugate molecule may include a linker moiety optionally used for linking to the siRNA and a functional moiety (e.g., a targeting moiety for targeting specific tissues or cells, such as GalNAc).

In the present disclosure, unless otherwise specified, "-" in "linker-targeting ligand" means that the linker is covalently bonded to the targeting ligand.

Unless otherwise specified, the term "pharmaceutically acceptable" in the present disclosure means that carriers, vehicles, diluents, excipients and/or salts/esters/hydrates formed therefrom etc. are generally chemically or physically compatible with other ingredients constituting a certain pharmaceutical dosage form and physiologically compatible with the recipient.

Unless otherwise specified, the term "being a 2’-fluoro nucleotide" in the present disclosure refers to a nucleotide containing a 2’-fluoro modification, which may also contain modifications at positions other than the 2’ position in the nucleotide structure. Unless otherwise specified, the term "being a 2’--methoxy nucleotide" in the present disclosure refers

to a nucleotide containing a 2'-methoxy modification, which may also contain modifications at positions other than the 2' position in the nucleotide structure.

Unless otherwise specified, "target sequence" in the present disclosure refers to a contiguous portion of the nucleotide sequence of an mRNA molecule formed during the transcription of the AGT gene, including mRNA that is an RNA processing product of the primary transcription product.

Unless otherwise specified, the term "inhibition" in the present disclosure refers to the situation of down-regulation of a target gene expression due to siRNA-mediated degradation of the mRNA of the target gene. The "down-regulation" refers to the situation where the target gene expression level decreases by 5%, 10%, 15%, 20%, 25%, 30%, 35%, 40%, 45%, 50%, 55%, 60%, 65%, 70%, 75%, 80%, 85%, 90%, 95%, or 99% or more, or even 100% compared to the absence of siRNA treatment. Among others, a 100% decrease of the target gene expression level means that there is no detectable level of target gene expression.

Unless otherwise specified, the terms "comprising", "including" and "containing" or equivalents in the present disclosure are open-ended mode expressions, meaning that in addition to the listed elements, components or steps, other unspecified elements, components or steps may also be encompassed.

Unless otherwise specified, the term "identity" in the present disclosure refers to the similarity between two nucleotide sequences or between two amino acid sequences. The percentage of identity between sequences can be determined by algorithms known to the skilled in the art (e.g., Needleman-Wunsch algorithm, Smith-Waterman algorithm, BLAST algorithm).

Unless otherwise specified, the term "substantially complementary" in the present disclosure means that two nucleic acid sequences are completely complementary or at least 90% (e.g., 91%, 92%, 93%, 94%, 95%, 96%, 97%, 98%, 99% or 100%) of the nucleotides therein are complementary.

Unless otherwise specified, parameter values representing amounts of ingredients, physicochemical properties, or reaction conditions etc. herein should be understood as being modified by the term "about" in all cases. When the term "about" is used to describe the present application, the term "about" indicates the existence of error values, for example, it indicates a variation within a range of ±5%, such as ±1% or 0.1% of a specific value.

The terms "subject", "patient", and "individual" are used interchangeably herein, including mammals or non-mammalian vertebrates (such as chicken, emu, fish). The mammals include but are not limited to domesticated animals (e.g., cattle, sheep, cat, dog, pig, and horse), primates (e.g., human, non-human primates such as monkey), rabbit, and rodents (e.g., mouse, rat, guinea pig, hamster), preferably human.

The terms "treating", "treatment" or "treat" herein refer to relieving or alleviating a certain disease or symptom, reducing the rate of onset or development of a certain disease or symptom, reducing the risk of developing a certain disease or symptom, or delaying the development of symptoms related to a certain disease or symptom, reducing or terminating symptoms related to a certain disease or symptom, achieving complete or partial reversal of a certain disease or symptom, curing a certain disease or symptom, or a combination of the above.

The various aspects of the present disclosure will be described in details below.

1. siRNA of the present disclosure

The term "siRNA" in the present disclosure means an RNA molecule that can sequence-specifically induce RNAi (RNA interference) phenomenon, comprises a sense strand and an antisense strand, and has a partially or completely complementary double-stranded structure.

The present disclosure provides an siRNA that inhibits the expression of the AGT gene, comprising a sense strand and an antisense strand, wherein the antisense strand comprises at least 19 contiguous nucleotides that differ by no more than 3 nucleotides from any one of the antisense strand sequences shown in Table 1. In some embodiments of the present disclosure, the region where the sense strand and the antisense strand are complementarily bound comprises at least 15 contiguous nucleotides, for example 20-21 contiguous nucleotides.

In some embodiments of the present disclosure, the antisense strand comprises at least 19 contiguous nucleotides that differ by no more than 2 nucleotides from any one of the antisense strand sequences shown in Table 1. In some embodiments of the present disclosure, the antisense strand comprises at least 19 contiguous nucleotides that differ by no more than 1 nucleotide from any one of the antisense strand sequences shown in Table 1.

In some embodiments of the present disclosure, the antisense strand comprises no more than 27, for example no more than 25 contiguous nucleotides.

In the present disclosure, the "difference" of the nucleotide sequence may include changes occurring in the nucleotide sequence caused by the addition, deletion or substitution of nucleotides.

The present disclosure provides an siRNA that inhibits the expression of the AGT gene, comprising a sense strand and an antisense strand, wherein the antisense strand comprises at least 19 contiguous nucleotides that differ by no more than 3 nucleotides from any one of antisense strand sequences selected from SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86,

88, 90, 92, 94, 96, 98, 100, 102, 104, 106, 109, 111, 113, 115, 117, 119, 121, 123, 125, 127, 129, 131, 133, 135, 137, 139, 141, and 143.

In some embodiments of the present disclosure, the antisense strand comprises at least 19 contiguous nucleotides that differ by no more than 2 nucleotides from any one of the antisense strand sequences of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

In some embodiments of the present disclosure, the antisense strand comprises at least 19 contiguous nucleotides that differ by no more than 1 nucleotide from any one of the antisense strand sequences of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

The present disclosure provides an siRNA that inhibits the expression of the AGT gene, comprising a sense strand and an antisense strand, wherein the antisense strand comprises at least 19 contiguous nucleotides that differ by no more than 3 nucleotides from any one of the antisense strand sequences selected from SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143.

In some embodiments of the present disclosure, the antisense strand comprises at least 19 contiguous nucleotides that differ by no more than 2 nucleotides from any one of the antisense strand sequences of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143. In some embodiments of the present disclosure, the antisense strand comprises at least 19 contiguous nucleotides that differ by no more than 1 nucleotide from any one of the antisense strand sequences of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143. In some embodiments of the present disclosure, the antisense strand comprises at least 19 contiguous nucleotides that differ by no more than 3 nucleotides, no more than 2 nucleotides, or no more than 1 nucleotide from any one of the antisense strand sequences of SEQ ID NOs: 6, 8, 10, 12, 14, 16, 18, 20, 22, 24, 30, 92, 113, and 131.

In some embodiments of the present disclosure, the antisense strand in the siRNA comprises a terminal overhang of 1-5 nucleotides in length at the 3' end. In some embodiments of the present disclosure, the antisense strand in the siRNA comprises a terminal overhang of 1-4 nucleotides in length at the 3' end. In some embodiments of the present disclosure, the antisense strand in the siRNA comprises a terminal overhang of 1-3 nucleotides in length at the 3' end. In some embodiments of the present disclosure, the antisense strand in the siRNA comprises a terminal overhang of 2 nucleotides in length at the 3' end.

In some embodiments of the present disclosure, the 5' end of the antisense strand in the siRNA is blunt.

In some embodiments of the present disclosure, the number of nucleotides of the sense strand in the siRNA is 16-25. In some embodiments of the present disclosure, the number of nucleotides of the sense strand in the siRNA is 16. In some embodiments of the present disclosure, the number of nucleotides of the sense strand in the siRNA is 17. In some embodiments of the present disclosure, the number of nucleotides of the sense strand in the siRNA is 18. In some embodiments of the present disclosure, the number of nucleotides of the sense strand in the siRNA is 19. In some embodiments of the present disclosure, the number of nucleotides of the sense strand in the siRNA is 20. In some embodiments of the present disclosure, the number of nucleotides of the sense strand in the siRNA is 21. In some embodiments of the present disclosure, the number of nucleotides of the sense strand in the siRNA is 22. In some embodiments of the present disclosure, the number of nucleotides of the sense strand in the siRNA is 23. In some embodiments of the present disclosure, the number of nucleotides of the sense strand in the siRNA is 24. In some embodiments of the present disclosure, the number of nucleotides of the sense strand in the siRNA is 25. In some embodiments of the present disclosure, the number of nucleotides of the antisense strand in the siRNA is 19-27. In some embodiments of the present disclosure, the number of nucleotides of the antisense strand in the siRNA is 19. In some embodiments of the present disclosure, the number of nucleotides of the antisense strand in the siRNA is 21. In some embodiments of the present disclosure, the number of nucleotides of the antisense strand in the siRNA is 22. In some embodiments of the present disclosure, the number of nucleotides of the antisense strand in the siRNA is 23. In some embodiments of the present disclosure, the number of nucleotides of the antisense strand in the siRNA is 24. In some embodiments of the present disclosure, the number of nucleotides of the antisense strand in the siRNA is 25. In some embodiments of the present disclosure, the number of nucleotides of the antisense strand in the siRNA is 26. In some embodiments of the present disclosure, the number of nucleotides of the antisense strand in the siRNA is 27.

In some embodiments of the present disclosure, the antisense strand in the siRNA differs by no more than 3 nucleotides from any one of the antisense strands listed in Table 1. In some embodiments of the present disclosure, the antisense strand in the siRNA differs by no more than 2 nucleotides from any one of the antisense strands listed in Table 1. In some embodiments of the present disclosure, the antisense strand in the siRNA differs by no more than 1 nucleotide from any one of the antisense strands listed in Table 1.

In some embodiments of the present disclosure, the antisense strand in the siRNA differs by no more than 3

nucleotides from any one of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

In some embodiments of the present disclosure, the antisense strand in the siRNA differs by no more than 2 nucleotides from any one of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

In some embodiments of the present disclosure, the antisense strand in the siRNA differs by no more than 1 nucleotide from any one of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

In some embodiments of the present disclosure, the antisense strand in the siRNA differs by no more than 3 nucleotides from any one of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143. In some embodiments of the present disclosure, the antisense strand in the siRNA differs by no more than 2 nucleotides from any one of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143.

In some embodiments of the present disclosure, the antisense strand in the siRNA differs by no more than 1 nucleotide from any one of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143.

In some embodiments of the present disclosure, the antisense strand in the siRNA is any one of the antisense strands shown in Table 1.

In some embodiments of the present disclosure, the antisense strand in the siRNA is any one of the antisense strands of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 32, 34, 36, 38, 40, 44, 60, 66, 68, 92, 94, 104, 113, 131, 137, 139, 141, and 143.

In some embodiments of the present disclosure, the antisense strand in the siRNA is any one of the antisense strands of SEQ ID NOs: 6, 8, 10, 12, 14, 16, 18, 20, 22, 24, 30, 32, 34, 36, 38, 40, 44, 60, 66, 68, 92, 94, 104, 113, 131, 137, 139, 141, and 143. Preferably, the antisense strand in the siRNA is any one of the antisense strands of SEQ ID NOs: 6, 8, 10, 12, 14, 16, 18, 20, 22, 24, 30, 92, 113, and 131.

Table 1 Unmodified siRNA sequences that inhibit the expression of the AGT gene

| Sense strand (5' to 3') | SEQ ID NOs | Antisense strand (5' to 3') | SEQ ID NOs |
|---|---|---|---|
| ACCGACCAGCUUGUUUGUGAA | SEQ ID NO: 5 | UUCACAAACAAGCUGGUCGGUUG | SEQ ID NO: 6 |
| CCAGCUUGUUUGUGAAACAAA | SEQ ID NO: 7 | UUUGUUUCACAAACAAGCUGGUC | SEQ ID NO: 8 |
| GACCAGCUUGUUUGUGAAACA | SEQ ID NO: 9 | UGUUUCACAAACAAGCUGGUCGG | SEQ ID NO: 10 |
| AGUGUUCCCUUUUCAAGUUGA | SEQ ID NO: 11 | UCAACUUGAAAAGGGAACACUUU | SEQ ID NO: 12 |
| GAGGUGCUGAACAGCAUUUUU | SEQ ID NO: 13 | AAAAAUGCUGUUCAGCACCUCCC | SEQ ID NO: 14 |
| AACAAAAAAGUGUUCCCUUUU | SEQ ID NO: 15 | AAAAGGGAACACUUUUUUGUUUC | SEQ ID NO: 16 |
| AACCGACCAGCUUGUUUGUGA | SEQ ID NO: 17 | UCACAAACAAGCUGGUCGGUUGG | SEQ ID NO: 18 |

(continued)

| Sense strand (5' to 3') | SEQ ID NOs | Antisense strand (5' to 3') | SEQ ID NOs |
|---|---|---|---|
| CCUGUUUGCUGUGUAUGAUCA | SEQ ID NO: 19 | UGAUCAUACACAGCAAACAGGAA | SEQ ID NO: 20 |
| AGCUUGUUUGUGAAACAAAAA | SEQ ID NO: 21 | UUUUUGUUUCACAAACAAGCUGG | SEQ ID NO: 22 |
| UCCCACCUUUUCUUCUAAUGA | SEQ ID NO: 23 | UCAUUAGAAGAAAAGGUGGGAGA | SEQ ID NO: 24 |
| GUUCCCUUUUCAAGUUGAGAA | SEQ ID NO: 25 | UUCUCAACUUGAAAAGGGAACAC | SEQ ID NO: 26 |
| AAAAAAGUGUUCCCUUUUCAA | SEQ ID NO: 27 | UUGAAAAGGGAACACUUUUUUGU | SEQ ID NO: 28 |
| ACCAGCUUGUUUGUGAAACAA | SEQ ID NO: 29 | UUGUUUCACAAACAAGCUGGUCG | SEQ ID NO: 30 |
| CUGUUCCAAAAAGAAUUCCAA | SEQ ID NO: 31 | UUGGAAUUCUUUUUGGAACAGUA | SEQ ID NO: 32 |
| UUUGUGAAACAAAAAAGUGUU | SEQ ID NO: 33 | AACACUUUUUUGUUUCACAAACA | SEQ ID NO: 34 |
| GUCUCCCACCUUUUCUUCUAA | SEQ ID NO: 35 | UUAGAAGAAAAGGUGGGAGACUG | SEQ ID NO: 36 |
| GAGGGUCUCACUUUCCAGCAA | SEQ ID NO: 37 | UUGCUGGAAAGUGAGACCCUCCA | SEQ ID NO: 38 |
| CCCAUUCCUGUUUGCUGUGUA | SEQ ID NO: 39 | UACACAGCAAACAGGAAUGGGCG | SEQ ID NO: 40 |
| GCUGGGUUUAUUUUAGAGAAU | SEQ ID NO: 41 | AUUCUCUAAAAUAAACCCAGCAA | SEQ ID NO: 42 |
| CCCAGUUUGCUGGGUUUAUUU | SEQ ID NO: 43 | AAAUAAACCCAGCAAACUGGGAG | SEQ ID NO: 44 |
| CCAGUCUCCCACCUUUUCUUU | SEQ ID NO: 45 | AAAGAAAAGGUGGGAGACUGGGG | SEQ ID NO: 46 |
| CAGUCUCCCACCUUUUCUUCU | SEQ ID NO: 47 | AGAAGAAAAGGUGGGAGACUGGG | SEQ ID NO: 48 |

(continued)

| Sense strand (5' to 3') | SEQ ID NOs | Antisense strand (5' to 3') | SEQ ID NOs |
|---|---|---|---|
| AGUCUCCCACCUUUUCUUCUA | SEQ ID NO: 49 | UAGAAGAAAAGGUGGGAGACUGG | SEQ ID NO: 50 |
| UCUCCCACCUUUUCUUCUAAU | SEQ ID NO: 51 | AUUAGAAGAAAAGGUGGGAGACU | SEQ ID NO: 52 |
| CUCCCACCUUUUCUUCUAAUU | SEQ ID NO: 53 | AAUUAGAAGAAAAGGUGGGAGAC | SEQ ID NO: 54 |
| CCCACCUUUUCUUCUAAUGAU | SEQ ID NO: 55 | AUCAUUAGAAGAAAAGGUGGGAG | SEQ ID NO: 56 |
| CCACCUUUUCUUCUAAUGAGU | SEQ ID NO: 57 | ACUCAUUAGAAGAAAAGGUGGGA | SEQ ID NO: 58 |
| CACCUUUUCUUCUAAUGAGUU | SEQ ID NO: 59 | AACUCAUUAGAAGAAAAGGUGGG | SEQ ID NO: 60 |
| ACCUUUUCUUCUAAUGAGUCU | SEQ ID NO: 61 | AGACUCAUUAGAAGAAAAGGUGG | SEQ ID NO: 62 |
| GACCUUUUCUUCUAAUGAGUU | SEQ ID NO: 63 | AACUCAUUAGAAGAAAAGGUCGG | SEQ ID NO: 64 |
| CUCCUUUUCUUCUAAUGAGUU | SEQ ID NO: 65 | AACUCAUUAGAAGAAAAGGAGGG | SEQ ID NO: 66 |
| CAGCUUUUCUUCUAAUGAGUU | SEQ ID NO: 67 | AACUCAUUAGAAGAAAAGCUGGG | SEQ ID NO: 68 |
| CACGUUUUCUUCUAAUGAGUU | SEQ ID NO: 69 | AACUCAUUAGAAGAAAACGUGGG | SEQ ID NO: 70 |
| CACCAUUUCUUCUAAUGAGUU | SEQ ID NO: 71 | AACUCAUUAGAAGAAAUGGUGGG | SEQ ID NO: 72 |
| CACCUAUUCUUCUAAUGAGUU | SEQ ID NO: 73 | AACUCAUUAGAAGAAUAGGUGGG | SEQ ID NO: 74 |
| GUCCUUUUCUUCUAAUGAGUU | SEQ ID NO: 75 | AACUCAUUAGAAGAAAAGGACGG | SEQ ID NO: 76 |
| GAGCUUUUCUUCUAAUGAGUU | SEQ ID NO: 77 | AACUCAUUAGAAGAAAAGCUCGG | SEQ ID NO: 78 |

(continued)

| Sense strand (5' to 3') | SEQ ID NOs | Antisense strand (5' to 3') | SEQ ID NOs |
|---|---|---|---|
| CUGCUUUUCUUCUAAUGAGUU | SEQ ID NO: 79 | AACUCAUUAGAAGAAAAGCAGGG | SEQ ID NO: 80 |
| GUGCUUUUCUUCUAAUGAGUU | SEQ ID NO: 81 | AACUCAUUAGAAGAAAAGCACGG | SEQ ID NO: 82 |
| ACCCACCUUUUCUUCUAAUGA | SEQ ID NO: 83 | UCAUUAGAAGAAAAGGUGGGUGA | SEQ ID NO: 84 |
| AGGCACCUUUUCUUCUAAUGA | SEQ ID NO: 85 | UCAUUAGAAGAAAAGGUGCCUGA | SEQ ID NO: 86 |
| UCCGACCAGCUUGUUUGUGAA | SEQ ID NO: 87 | UUCACAAACAAGCUGGUCGGAUG | SEQ ID NO: 88 |
| UGGGACCAGCUUGUUUGUGAA | SEQ ID NO: 89 | UUCACAAACAAGCUGGUCCCAUG | SEQ ID NO: 90 |
| CACCUUUUCUUCUAAUGAGUA | SEQ ID NO: 91 | UACUCAUUAGAAGAAAAGGUGGG | SEQ ID NO: 92 |
| CGCCUUUUCUUCUAAUGAGUA | SEQ ID NO: 93 | UACUCAUUAGAAGAAAAGGCGGG | SEQ ID NO: 94 |
| CUCCCACCUUUUCUUCUAAUGAGUA | SEQ ID NO: 95 | UACUCAUUAGAAGAAAAGGUGGGAGAC | SEQ ID NO: 96 |
| UCCCACCUUUUCUUCUAAUGAGUA | SEQ ID NO: 97 | UACUCAUUAGAAGAAAAGGUGGGAGA | SEQ ID NO: 98 |
| CCCACCUUUUCUUCUAAUGAGUA | SEQ ID NO: 99 | UACUCAUUAGAAGAAAAGGUGGGAG | SEQ ID NO: 100 |
| CCACCUUUUCUUCUAAUGAGUA | SEQ ID NO: 101 | UACUCAUUAGAAGAAAAGGUGGGA | SEQ ID NO: 102 |
| ACCUUUUCUUCUAAUGAGUA | SEQ ID NO: 103 | UACUCAUUAGAAGAAAAGGUGG | SEQ ID NO: 104 |
| CCUUUUCUUCUAAUGAGUA | SEQ ID NO: 105 | UACUCAUUAGAAGAAAAGGUG | SEQ ID NO: 106 |
| UUUCUUCUAAUGAGUA | SEQ ID NO: 107 | UACUCAUUAGAAGAAAAGGUG | SEQ ID NO: 106 |

(continued)

| Sense strand (5' to 3') | SEQ ID NOs | Antisense strand (5' to 3') | SEQ ID NOs |
|---|---|---|---|
| UUUUCUUCUAAUGAGUA | SEQ ID NO: 108 | UACUCAUUAGAAGAAAAGGUG | SEQ ID NO: 106 |
| UUUUCUUCUAAUGAGUA | SEQ ID NO: 108 | UACUCAUUAGAAGAAAAGGUGGG | SEQ ID NO: 109 |
| CUUUUCUUCUAAUGAGUA | SEQ ID NO: 110 | UACUCAUUAGAAGAAAAGGUGGG | SEQ ID NO: 109 |
| CCUUUUCUUCUAAUGAGUA | SEQ ID NO: 105 | UACUCAUUAGAAGAAAAGGUGGG | SEQ ID NO: 109 |
| UUUCUUCUAAUGAGUA | SEQ ID NO: 107 | UACUCAUUAGAAGAAAAGG | SEQ ID NO: 111 |
| UUUUCUUCUAAUGAGUA | SEQ ID NO: 108 | UACUCAUUAGAAGAAAAGG | SEQ ID NO: 111 |
| CUCCUUUUCUUCUAAUGAGUA | SEQ ID NO: 112 | UACUCAUUAGAAGAAAAGGAGGG | SEQ ID NO: 113 |
| UCCUUUUCUUCUAAUGAGUA | SEQ ID NO: 114 | UACUCAUUAGAAGAAAAGGAGG | SEQ ID NO: 115 |
| AGCUUUUCUUCUAAUGAGUA | SEQ ID NO: 116 | UACUCAUUAGAAGAAAAGCUGG | SEQ ID NO: 117 |
| ACGUUUUCUUCUAAUGAGUA | SEQ ID NO: 118 | UACUCAUUAGAAGAAAACGUGG | SEQ ID NO: 119 |
| GCCUUUUCUUCUAAUGAGUA | SEQ ID NO: 120 | UACUCAUUAGAAGAAAAGGCGG | SEQ ID NO: 121 |
| GCACCUUUUCUUCUAAUGAGUA | SEQ ID NO: 122 | UACUCAUUAGAAGAAAAGGUGCGA | SEQ ID NO: 123 |
| CGACCUUUUCUUCUAAUGAGUA | SEQ ID NO: 124 | UACUCAUUAGAAGAAAAGGUCGGA | SEQ ID NO: 125 |
| CCUCCUUUUCUUCUAAUGAGUA | SEQ ID NO: 126 | UACUCAUUAGAAGAAAAGGAGGGA | SEQ ID NO: 127 |
| CCGCCUUUUCUUCUAAUGAGUA | SEQ ID NO: 128 | UACUCAUUAGAAGAAAAGGCGGGA | SEQ ID NO: 129 |

(continued)

| Sense strand (5' to 3') | SEQ ID NOs | Antisense strand (5' to 3') | SEQ ID NOs |
|---|---|---|---|
| CAGCUUUUCUUCUAAUGAGUA | SEQ ID NO: 130 | UACUCAUUAGAAGAAAAGCUGGG | SEQ ID NO: 131 |
| UGCCACCUUUUCUUCUAAUGA | SEQ ID NO: 132 | UCAUUAGAAGAAAAGGUGGCAGA | SEQ ID NO: 133 |
| UCGCACCUUUUCUUCUAAUGA | SEQ ID NO: 134 | UCAUUAGAAGAAAAGGUGCGAGA | SEQ ID NO: 135 |
| GACCUUUUCUUCUAAUGAGUA | SEQ ID NO: 136 | UACUCAUUAGAAGAAAAGGUCGG | SEQ ID NO: 137 |
| CACGUUUUCUUCUAAUGAGUA | SEQ ID NO: 138 | UACUCAUUAGAAGAAAACGUGGG | SEQ ID NO: 139 |
| CACCAUUUCUUCUAAUGAGUA | SEQ ID NO: 140 | UACUCAUUAGAAGAAAUGGUGGG | SEQ ID NO: 141 |
| CACCUAUUCUUCUAAUGAGUA | SEQ ID NO: 142 | UACUCAUUAGAAGAAUAGGUGGG | SEQ ID NO: 143 |

In some embodiments of the present disclosure, there is at least one mismatch between the sense strand and the antisense strand in the siRNA. In some embodiments of the present disclosure, there are at most 3 mismatches between the sense strand and the antisense strand in the siRNA. In some embodiments of the present disclosure, there are at most 2 mismatches between the sense strand and the antisense strand in the siRNA. In some embodiments of the present disclosure, there is at most one mismatch between the sense strand and the antisense strand in the siRNA. In some embodiments of the present disclosure, there is no mismatch between the sense strand and the antisense strand in the siRNA. In some embodiments of the present disclosure, there are at most 4 mismatches between the antisense strand in the siRNA and the target sequence. In some embodiments of the present disclosure, there are at most 3 mismatches between the antisense strand in the siRNA and the target sequence. In some embodiments of the present disclosure, there are at most 2 mismatches between the antisense strand in the siRNA and the target sequence. In some embodiments of the present disclosure, there is at most one mismatch between the antisense strand in the siRNA and the target sequence.

In some embodiments of the present disclosure, the nucleotide at position 1 in the direction from the 5' end to the 3' end of the siRNA antisense strand is mismatched with the target sequence. In some embodiments of the present disclosure, the nucleotide at position 1 in the direction from the 5' end to the 3' end of the siRNA antisense strand is mismatched with the target sequence; and at the mismatch site between the antisense strand and the target sequence, the nucleotides between the antisense strand and the target sequence are different. In some embodiments of the present disclosure, the nucleotide at position 1 in the direction from the 5' end to the 3' end of the siRNA antisense strand is mismatched with the target sequence; and at the mismatch site between the antisense strand and the target sequence, the nucleotides between the antisense strand and the sense strand are complementary. In some embodiments of the present disclosure, the nucleotide at position 1 in the direction from the 5' end to the 3' end of the siRNA antisense strand is mismatched with the target sequence; and at the mismatch site between the antisense strand and the target sequence, the nucleotides between the antisense strand and the target sequence are different, and the nucleotides between the antisense strand and the sense strand are complementarily paired. In some embodiments of the present disclosure, the nucleotide at position 1 in the direction from the 5' end to the 3' end of the siRNA antisense strand is U or A. In some embodiments of the present disclosure, the nucleotide at position 1 in the direction from the 5' end to the 3' end of the siRNA antisense strand is U or A, and the corresponding nucleotide in the sense strand that is complementarily paired with the nucleotide is A or U. In some embodiments of the present disclosure, the nucleotides at positions 16 to 23 in the direction from the 5' end to the 3' end of the siRNA antisense strand have at most one mismatch with the target sequence. In some embodiments of the present disclosure, the nucleotide at position 16 in the direction from the 5' end to the 3' end of the

siRNA antisense strand is mismatched with the target sequence. In some embodiments of the present disclosure, the nucleotide at position 17 in the direction from the 5' end to the 3' end of the siRNA antisense strand is mismatched with the target sequence. In some embodiments of the present disclosure, the nucleotide at position 18 in the direction from the 5' end to the 3' end of the siRNA antisense strand is mismatched with the target sequence. In some embodiments of the present disclosure, the nucleotide at position 19 in the direction from the 5' end to the 3' end of the siRNA antisense strand is mismatched with the target sequence. In some embodiments of the present disclosure, the nucleotide at position 20 in the direction from the 5' end to the 3' end of the siRNA antisense strand is mismatched with the target sequence. In some embodiments of the present disclosure, the nucleotide at position 21 in the direction from the 5' end to the 3' end of the siRNA antisense strand is mismatched with the target sequence. In some embodiments of the present disclosure, the nucleotide at position 22 in the direction from the 5' end to the 3' end of the siRNA antisense strand is mismatched with the target sequence. In some embodiments of the present disclosure, the nucleotide at position 23 in the direction from the 5' end to the 3' end of the siRNA antisense strand is mismatched with the target sequence. In some embodiments of the present disclosure, the antisense strand in the siRNA has at least one mismatch with the target sequence. In some embodiments of the present disclosure, the nucleotides at positions 16 to 21 in the direction from the 5' end to the 3' end of the siRNA antisense strand have at most one mismatch with the target sequence. In some embodiments of the present disclosure, the nucleotides at positions 16 to 22 in the direction from the 5' end to the 3' end of the siRNA antisense strand have at most 3 mismatches with the target sequence. In some embodiments of the present disclosure, among the nucleotides at positions 16 to 21 in the direction from the 5' end to the 3' end of the siRNA antisense strand, there is optionally one mismatch with the target sequence. In some embodiments of the present disclosure, among the nucleotides at positions 16 to 21 in the direction from the 5' end to the 3' end of the siRNA antisense strand, there are optionally 2 mismatches with the target sequence. In some embodiments of the present disclosure, among the nucleotides at positions 16 to 21 in the direction from the 5' end to the 3' end of the siRNA antisense strand, there are optionally 3 mismatches with the target sequence. In some embodiments of the present disclosure, among the nucleotides at positions 19, 20 and 21 in the direction from the 5' end to the 3' end of the siRNA antisense strand, there is optionally one mismatch with the target sequence. In some embodiments of the present disclosure, among the nucleotides at positions 19, 20 and 21 in the direction from the 5' end to the 3' end of the siRNA antisense strand, there are optionally 2 mismatches with the target sequence. In some embodiments of the present disclosure, the nucleotides at positions 19 and 21 in the direction from the 5' end to the 3' end of the siRNA antisense strand are mismatched with the target sequence. In some embodiments of the present disclosure, the nucleotides at positions 19 and 20 in the direction from the 5' end to the 3' end of the siRNA antisense strand are mismatched with the target sequence. In some embodiments of the present disclosure, the nucleotides at positions 20 and 21 in the direction from the 5' end to the 3' end of the siRNA antisense strand are mismatched with the target sequence. In some embodiments of the present disclosure, the nucleotides at positions 19, 20 and 21 in the direction from the 5' end to the 3' end of the siRNA antisense strand are all mismatched with the target sequence. In some embodiments of the present disclosure, at site(s) where there is a mismatch between the siRNA antisense strand and the target sequence, the nucleotides of the antisense strand and the target sequence are identical. In some embodiments of the present disclosure, at site(s) where there is a mismatch between the siRNA antisense strand and the target sequence, the nucleotides of the target sequence and the siRNA sense strand are complementary. In some embodiments of the present disclosure, at site(s) where there is a mismatch between the siRNA antisense strand and the target sequence, the nucleotides of the antisense strand and the sense strand are interchanged. In some embodiments of the present disclosure, at site(s) where there is a mismatch between the siRNA antisense strand and the target sequence, the nucleotides of the antisense strand and the sense strand are complementary. In some embodiments of the present disclosure, at site(s) where there is a mismatch between the siRNA antisense strand and the target sequence, the nucleotides of the antisense strand and the sense strand are interchanged, and the nucleotides of the antisense strand and the sense strand are complementary.

In some embodiments of the present disclosure, the nucleotide at position 16 in the direction from the 5' end to the 3' end of the antisense strand is mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 17 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 18 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 19 in the direction from the 5' end to the 3' end of the antisense strand are mismatched with the target sequence; and at the site where the antisense strand and the target sequence are mismatched, the nucleotides of the antisense strand and the target sequence are identical, and the

nucleotides of the antisense strand and the sense strand are complementarily paired. In some embodiments of the present disclosure, the nucleotide at position 20 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 21 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 22 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the nucleotide at position 23 in the direction from the 5' end to the 3' end of the antisense strand are mismatched with the target sequence; and at the site where the antisense strand and the target sequence are mismatched, the nucleotides of the antisense strand and the target sequence are identical, and the nucleotides of the antisense strand and the sense strand are complementarily paired. In some embodiments of the present disclosure, the antisense strand has at most two mismatches with the target sequence, wherein the nucleotides at positions 1 and 20 in the direction from the 5' end to the 3' end of the antisense strand are mismatched with the target sequence.

In some embodiments of the present disclosure, the nucleotide at position 19 in the direction from the 5' end to the 3' end of the antisense strand is mismatched with the target sequence; and at the site where the antisense strand and the target sequence are mismatched, the nucleotides of the antisense strand and the target sequence are different, and the nucleotides of the antisense strand and the sense strand are complementarily paired. In some embodiments of the present disclosure, the nucleotide at position 20 in the direction from the 5' end to the 3' end of the antisense strand is mismatched with the target sequence; and at the site where the antisense strand and the target sequence are mismatched, the nucleotides of the antisense strand and the target sequence are different, and the nucleotides of the antisense strand and the sense strand are complementarily paired. In some embodiments of the present disclosure, the nucleotide at position 21 in the direction from the 5' end to the 3' end of the antisense strand is mismatched with the target sequence; and at the site where the antisense strand and the target sequence are mismatched, the nucleotides of the antisense strand and the target sequence are different, and the nucleotides of the antisense strand and the sense strand are complementarily paired.

In some embodiments of the present disclosure, the antisense strand of the siRNA has no mismatch with the target sequence.

It is known to the skilled in the art that mismatches are permissible for the activity of siRNA. The methods described herein and/or methods known in the art can be used to determine whether the AGT siRNA containing mismatches effectively inhibits the expression of the AGT gene.

In some embodiments of the present disclosure, the unmodified duplex of the siRNA is selected from duplexes consisting of the sense strand sequences and the antisense strand sequences in the same row in Table 1.

In some embodiments of the present disclosure, the unmodified duplex of the siRNA is selected from: a duplex consisting of SEQ ID NO: 5 and SEQ ID NO: 6, a duplex consisting of SEQ ID NO: 7 and SEQ ID NO: 8, a duplex consisting of SEQ ID NO: 9 and SEQ ID NO: 10, a duplex consisting of SEQ ID NO: 11 and SEQ ID NO: 12, a duplex consisting of SEQ ID NO: 13 and SEQ ID NO: 14, a duplex consisting of SEQ ID NO: 15 and SEQ ID NO: 16, a duplex consisting of SEQ ID NO: 17 and SEQ ID NO: 18, a duplex consisting of SEQ ID NO: 19 and SEQ ID NO: 20, a duplex consisting of SEQ ID NO: 21 and SEQ ID NO: 22, a duplex consisting of SEQ ID NO: 23 and SEQ ID NO: 24, a duplex consisting of SEQ ID NO: 25 and SEQ ID NO: 26, a duplex consisting of SEQ ID NO: 27 and SEQ ID NO: 28, a duplex consisting of SEQ ID NO: 29 and SEQ ID NO: 30, a duplex consisting of SEQ ID NO: 31 and SEQ ID NO: 32, a duplex consisting of SEQ ID NO: 33 and SEQ ID NO: 34, a duplex consisting of SEQ ID NO: 35 and SEQ ID NO: 36, a duplex consisting of SEQ ID NO: 37 and SEQ ID NO: 38, a duplex consisting of SEQ ID NO: 39 and SEQ ID NO: 40, a duplex consisting of SEQ ID NO: 41 and SEQ ID NO: 42, a duplex consisting of SEQ ID NO: 43 and SEQ ID NO: 44, a duplex consisting of SEQ ID NO: 45 and SEQ ID NO: 46, a duplex consisting of SEQ ID NO: 47 and SEQ ID NO: 48, a duplex consisting of SEQ ID NO: 49 and SEQ ID NO: 50, a duplex consisting of SEQ ID NO: 51 and SEQ ID NO: 52, a duplex consisting of SEQ ID NO: 53 and SEQ ID NO: 54, a duplex consisting of SEQ ID NO: 55 and SEQ ID NO: 56, a duplex consisting of SEQ ID NO: 57 and SEQ ID NO: 58, a duplex consisting of SEQ ID NO: 59 and SEQ ID NO: 60, a duplex consisting of SEQ ID NO: 61 and SEQ ID NO: 62, a duplex consisting of SEQ ID NO: 63 and SEQ ID NO: 64, a duplex consisting of SEQ ID NO: 65 and SEQ ID NO: 66, a duplex consisting of SEQ ID NO: 67 and SEQ ID NO: 68, a duplex consisting of SEQ ID NO: 69 and SEQ ID NO: 70, a duplex consisting of SEQ ID NO: 71 and SEQ ID NO: 72, a duplex consisting of SEQ ID NO: 73 and SEQ ID NO: 74, a duplex consisting of SEQ ID NO: 75 and SEQ ID NO: 76, a duplex consisting of SEQ ID NO: 77 and SEQ ID NO: 78, a duplex consisting of SEQ ID NO: 79 and SEQ ID NO: 80, a duplex consisting of SEQ ID NO: 81 and SEQ ID NO: 82, a duplex consisting of SEQ ID NO: 83 and SEQ ID NO: 84, a duplex consisting of SEQ ID NO: 85 and SEQ ID NO: 86, a

duplex consisting of SEQ ID NO: 87 and SEQ ID NO: 88, a duplex consisting of SEQ ID NO: 89 and SEQ ID NO: 90, a duplex consisting of SEQ ID NO: 91 and SEQ ID NO: 92, a duplex consisting of SEQ ID NO: 93 and SEQ ID NO: 94, a duplex consisting of SEQ ID NO: 95 and SEQ ID NO: 96, a duplex consisting of SEQ ID NO: 97 and SEQ ID NO: 98, a duplex consisting of SEQ ID NO: 99 and SEQ ID NO: 100, a duplex consisting of SEQ ID NO: 101 and SEQ ID NO: 102, a duplex consisting of SEQ ID NO: 103 and SEQ ID NO: 104, a duplex consisting of SEQ ID NO: 105 and SEQ ID NO: 106, a duplex consisting of SEQ ID NO: 107 and SEQ ID NO: 106, a duplex consisting of SEQ ID NO: 108 and SEQ ID NO: 106, a duplex consisting of SEQ ID NO: 108 and SEQ ID NO: 109, a duplex consisting of SEQ ID NO: 110 and SEQ ID NO: 109, a duplex consisting of SEQ ID NO: 105 and SEQ ID NO: 109, a duplex consisting of SEQ ID NO: 107 and SEQ ID NO: 111, a duplex consisting of SEQ ID NO: 108 and SEQ ID NO: 111, a duplex consisting of SEQ ID NO: 112 and SEQ ID NO: 113, a duplex consisting of SEQ ID NO: 114 and SEQ ID NO: 115, a duplex consisting of SEQ ID NO: 116 and SEQ ID NO: 117, a duplex consisting of SEQ ID NO: 118 and SEQ ID NO: 119, a duplex consisting of SEQ ID NO: 120 and SEQ ID NO: 121, a duplex consisting of SEQ ID NO: 122 and SEQ ID NO: 123, a duplex consisting of SEQ ID NO: 124 and SEQ ID NO: 125, a duplex consisting of SEQ ID NO: 126 and SEQ ID NO: 127, a duplex consisting of SEQ ID NO: 128 and SEQ ID NO: 129, a duplex consisting of SEQ ID NO: 130 and SEQ ID NO: 131, a duplex consisting of SEQ ID NO: 132 and SEQ ID NO: 133, a duplex consisting of SEQ ID NO: 134 and SEQ ID NO: 135, a duplex consisting of SEQ ID NO: 136 and SEQ ID NO: 137, a duplex consisting of SEQ ID NO: 138 and SEQ ID NO: 139, a duplex consisting of SEQ ID NO: 140 and SEQ ID NO: 141, and a duplex consisting of SEQ ID NO: 142 and SEQ ID NO: 143. Preferably, in some embodiments of the present disclosure, the unmodified duplex of the siRNA is selected from the duplex consisting of SEQ ID NO: 5 and SEQ ID NO: 6, the duplex consisting of SEQ ID NO: 7 and SEQ ID NO: 8, the duplex consisting of SEQ ID NO: 9 and SEQ ID NO: 10, the duplex consisting of SEQ ID NO: 11 and SEQ ID NO: 12, the duplex consisting of SEQ ID NO: 13 and SEQ ID NO: 14, the duplex consisting of SEQ ID NO: 15 and SEQ ID NO: 16, the duplex consisting of SEQ ID NO: 17 and SEQ ID NO: 18, the duplex consisting of SEQ ID NO: 19 and SEQ ID NO: 20, the duplex consisting of SEQ ID NO: 21 and SEQ ID NO: 22, the duplex consisting of SEQ ID NO: 23 and SEQ ID NO: 24, the duplex consisting of SEQ ID NO: 25 and SEQ ID NO: 26, the duplex consisting of SEQ ID NO: 27 and SEQ ID NO: 28, the duplex consisting of SEQ ID NO: 29 and SEQ ID NO: 30, the duplex consisting of SEQ ID NO: 31 and SEQ ID NO: 32, the duplex consisting of SEQ ID NO: 33 and SEQ ID NO: 34, the duplex consisting of SEQ ID NO: 35 and SEQ ID NO: 36, the duplex consisting of SEQ ID NO: 37 and SEQ ID NO: 38, the duplex consisting of SEQ ID NO: 39 and SEQ ID NO: 40, the duplex consisting of SEQ ID NO: 41 and SEQ ID NO: 42, the duplex consisting of SEQ ID NO: 43 and SEQ ID NO: 44, the duplex consisting of SEQ ID NO: 59 and SEQ ID NO: 60, the duplex consisting of SEQ ID NO: 65 and SEQ ID NO: 66, the duplex consisting of SEQ ID NO: 67 and SEQ ID NO: 68, the duplex consisting of SEQ ID NO: 91 and SEQ ID NO: 92, the duplex consisting of SEQ ID NO: 93 and SEQ ID NO: 94, the duplex consisting of SEQ ID NO: 103 and SEQ ID NO: 104, and the duplex consisting of SEQ ID NO: 112 and SEQ ID NO: 113. More preferably, in some embodiments of the present disclosure, the unmodified duplex of the siRNA is selected from the duplex consisting of SEQ ID NO: 5 and SEQ ID NO: 6, the duplex consisting of SEQ ID NO: 7 and SEQ ID NO: 8, the duplex consisting of SEQ ID NO: 9 and SEQ ID NO: 10, the duplex consisting of SEQ ID NO: 11 and SEQ ID NO: 12, the duplex consisting of SEQ ID NO: 13 and SEQ ID NO: 14, the duplex consisting of SEQ ID NO: 15 and SEQ ID NO: 16, the duplex consisting of SEQ ID NO: 17 and SEQ ID NO: 18, the duplex consisting of SEQ ID NO: 19 and SEQ ID NO: 20, the duplex consisting of SEQ ID NO: 21 and SEQ ID NO: 22, the duplex consisting of SEQ ID NO: 23 and SEQ ID NO: 24, the duplex consisting of SEQ ID NO: 29 and SEQ ID NO: 30, the duplex consisting of SEQ ID NO: 91 and SEQ ID NO: 92, and the duplex consisting of SEQ ID NO: 112 and SEQ ID NO: 113. In some embodiments of the present disclosure, the unmodified duplex of the siRNA is selected from at least one of duplex 1 to duplex 27;

Duplex 1

Sense strand: 5'-ACCGACCAGCUUGUUUGUGAA-3' (SEQ ID NO: 5)
Antisense strand: 5'-UUCACAAACAAGCUGGUCGGUUG-3' (SEQ ID NO: 6);

Duplex 2

Sense strand: 5'-CCAGCUUGUUUGUGAAACAAA-3' (SEQ ID NO: 7)
Antisense strand: 5'-UUUGUUUCACAAACAAGCUGGUC-3' (SEQ ID NO: 8);

Duplex 3

Sense strand: 5'-GACCAGCUUGUUUGUGAAACA-3' (SEQ ID NO: 9)
Antisense strand: 5'-UGUUUCACAAACAAGCUGGUCGG-3' (SEQ ID NO: 10);

Duplex 4

Sense strand: 5’-AGUGUUCCCUUUUCAAGUUGA-3’ (SEQ ID NO: 11)
Antisense strand: 5’-UCAACUUGAAAAGGGAACACUUU-3’ (SEQ ID NO: 12);

Duplex 5

Sense strand: 5’-GAGGUGCUGAACAGCAUUUUU-3’ (SEQ ID NO: 13)
Antisense strand: 5’-AAAAAUGCUGUUCAGCACCUCCC-3’ (SEQ ID NO: 14);

Duplex 6

Sense strand: 5’-AACAAAAAAGUGUUCCCUUUU-3’ (SEQ ID NO: 15)
Antisense strand: 5’-AAAAGGGAACACUUUUUUGUUUC-3’ (SEQ ID NO: 16);

Duplex 7

Sense strand: 5’-AACCGACCAGCUUGUUUGUGA-3’ (SEQ ID NO: 17)
Antisense strand: 5’-UCACAAACAAGCUGGUCGGUUGG-3’ (SEQ ID NO: 18);

Duplex 8

Sense strand: 5’-CCUGUUUGCUGUGUAUGAUCA-3’ (SEQ ID NO: 19)
Antisense strand: 5’-UGAUCAUACACAGCAAACAGGAA-3’ (SEQ ID NO: 20);

Duplex 9

Sense strand: 5’-AGCUUGUUUGUGAAACAAAAA-3’ (SEQ ID NO: 21)
Antisense strand: 5’-UUUUUGUUUCACAAACAAGCUGG-3’ (SEQ ID NO: 22);

Duplex 10

Sense strand: 5’-UCCCACCUUUUCUUCUAAUGA-3’ (SEQ ID NO: 23)
Antisense strand: 5’-UCAUUAGAAGAAAAGGUGGGAGA-3’ (SEQ ID NO: 24);

Duplex 11

Sense strand: 5’-GUUCCCUUUUCAAGUUGAGAA-3’ (SEQ ID NO: 25)
Antisense strand: 5’-UUCUCAACUUGAAAAGGGAACAC-3’ (SEQ ID NO: 26);

Duplex 12

Sense strand: 5’-AAAAAAGUGUUCCCUUUUCAA-3’ (SEQ ID NO: 27)
Antisense strand: 5’- UUGAAAAGGGAACACUUUUUUGU-3’ (SEQ ID NO: 28);

Duplex 13

Sense strand: 5’-ACCAGCUUGUUUGUGAAACAA-3’ (SEQ ID NO: 29)
Antisense strand: 5’-UUGUUUCACAAACAAGCUGGUCG-3’ (SEQ ID NO: 30);

Duplex 14

Sense strand: 5’-CUGUUCCAAAAAGAAUUCCAA-3’ (SEQ ID NO: 31)
Antisense strand: 5’-UUGGAAUUCUUUUUGGAACAGUA-3’ (SEQ ID NO: 32);

Duplex 15

Sense strand: 5’-UUUGUGAAACAAAAAAGUGUU-3’ (SEQ ID NO: 33)
Antisense strand: 5’-AACACUUUUUUGUUUCACAAACA-3’ (SEQ ID NO: 34);

Duplex 16

Sense strand: 5'-GUCUCCCACCUUUUCUUCUAA-3' (SEQ ID NO: 35)
Antisense strand: 5'-UUAGAAGAAAAGGUGGGAGACUG-3' (SEQ ID NO: 36);

Duplex 17

Sense strand: 5'-GAGGGUCUCACUUUCCAGCAA-3' (SEQ ID NO: 37)
Antisense strand: 5'-UUGCUGGAAAGUGAGACCCUCCA-3' (SEQ ID NO: 38);

Duplex 18

Sense strand: 5'-CCCAUUCCUGUUUGCUGUGUA-3' (SEQ ID NO: 39)
Antisense strand: 5'-UACACAGCAAACAGGAAUGGGCG-3' (SEQ ID NO: 40);

Duplex 19

Sense strand: 5'-GCUGGGUUUAUUUUAGAGAAU-3' (SEQ ID NO: 41)
Antisense strand: 5'-AUUCUCUAAAAUAAACCCAGCAA-3' (SEQ ID NO: 42);

Duplex 20

Sense strand: 5'-CCCAGUUUGCUGGGUUUAUUU-3' (SEQ ID NO: 43)
Antisense strand: 5'-AAAUAAACCCAGCAAACUGGGAG-3' (SEQ ID NO: 44);

Duplex 21

Sense strand: 5'-CACCUUUUCUUCUAAUGAGUU-3' (SEQ ID NO: 59)
Antisense strand: 5'-AACUCAUUAGAAGAAAAGGUGGG-3' (SEQ ID NO: 60);

Duplex 22

Sense strand: 5'-CUCCUUUUCUUCUAAUGAGUU-3' (SEQ ID NO: 65)
Antisense strand: 5'-AACUCAUUAGAAGAAAAGGAGGG-3' (SEQ ID NO: 66);

Duplex 23

Sense strand: 5'-CAGCUUUUCUUCUAAUGAGUU-3' (SEQ ID NO: 67)
Antisense strand: 5'-AACUCAUUAGAAGAAAAGCUGGG-3' (SEQ ID NO: 68);

Duplex 24

Sense strand: 5'-CACCUUUUCUUCUAAUGAGUA-3' (SEQ ID NO: 91)
Antisense strand: 5'-UACUCAUUAGAAGAAAAGGUGGG-3' (SEQ ID NO: 92);

Duplex 25

Sense strand: 5'-CGCCUUUUCUUCUAAUGAGUA-3' (SEQ ID NO: 93)
Antisense strand: 5'-UACUCAUUAGAAGAAAAGGCGGG-3' (SEQ ID NO: 94);

Duplex 26

Sense strand: 5'-CUCCUUUUCUUCUAAUGAGUA-3' (SEQ ID NO: 112)
Antisense strand: 5'-UACUCAUUAGAAGAAAAGGAGGG-3' (SEQ ID NO: 113);

Duplex 27

Sense strand: 5'-ACCUUUUCUUCUAAUGAGUA-3' (SEQ ID NO: 103)

Antisense strand: 5'-UACUCAUUAGAAGAAAAGGUGG-3' (SEQ ID NO: 104).

In some embodiments of the present disclosure, the unmodified duplex of the siRNA is a duplex that differs by no more than 2 nucleotides from any one of duplex 1 to duplex 27.

In some embodiments of the present disclosure, the unmodified duplex of the siRNA differs by no more than 2 nucleotides from duplex 21. In some embodiments of the present disclosure, the unmodified duplex of the siRNA differs by no more than 2 nucleotides from duplex 24. In some embodiments of the present disclosure, the unmodified duplex of the siRNA differs by 2 nucleotides from duplex 24. In some embodiments of the present disclosure, the sense strand of the unmodified duplex of the siRNA differs by no more than 1 nucleotide from the sense strand of duplex 24; and the antisense strand of the unmodified duplex of the siRNA differs by no more than 1 nucleotide from the antisense strand of duplex 24.

In some embodiments of the present disclosure, the sense strand of the unmodified duplex of the siRNA differs by 1 nucleotide from the sense strand of duplex 24, which is located within positions 1 to 3 in the direction from the 5' end to the 3' end of the sense strand; and the antisense strand of the unmodified duplex of the siRNA differs by 1 nucleotide from the antisense strand of duplex 24, which is located within positions 19 to 21 in the direction from the 5' end to the 3' end of the antisense strand. In some embodiments of the present disclosure, the difference between the sense strand of the unmodified duplex of the siRNA and the sense strand of duplex 24 is the nucleotide located at position 1 in the direction from the 5' end to the 3' end of the sense strand; and the difference between the antisense strand of the unmodified duplex of the siRNA and the antisense strand of duplex 24 is the nucleotide located at position 21 in the direction from the 5' end to the 3' end of the antisense strand. In some embodiments of the present disclosure, the difference between the sense strand of the unmodified duplex of the siRNA and the sense strand of duplex 24 is the nucleotide located at position 2 in the direction from the 5' end to the 3' end of the sense strand; and the difference between the antisense strand of the unmodified duplex of the siRNA and the antisense strand of duplex 24 is the nucleotide located at position 20 in the direction from the 5' end to the 3' end of the antisense strand. In some embodiments of the present disclosure, the difference between the sense strand of the unmodified duplex of the siRNA and the sense strand of duplex 24 is the nucleotide located at position 3 in the direction from the 5' end to the 3' end of the sense strand; and the difference between the antisense strand of the unmodified duplex of the siRNA and the antisense strand of duplex 24 is the nucleotide located at position 19 in the direction from the 5' end to the 3' end of the antisense strand.

In some embodiments of the present disclosure, the difference between the sense strand of the unmodified duplex of the siRNA and the sense strand of duplex 24 is the nucleotide located at position 2 in the direction from the 5' end to the 3' end of the sense strand; the difference between the antisense strand of the unmodified duplex of the siRNA and the antisense strand of duplex 24 is the nucleotide located at position 20 in the direction from the 5' end to the 3' end of the antisense strand; and the nucleotide at position 2 in the direction from the 5' end to the 3' end of the sense strand and the nucleotide at position 20 in the direction from the 5' end to the 3' end of the antisense strand of the unmodified duplex of the siRNA are complementary.

In some embodiments of the present disclosure, siRNA may also contain modified nucleotides or nucleotide analogs as needed, and the modified nucleotides or nucleotide analogs do not lead to a significant weakening or loss of the function of the siRNA in inhibiting the expression of the AGT gene. Currently, there are multiple methods in the art that can be used to modify siRNA, including, for example, backbone modification (such as phosphate group modification), ribose group modification, and base modification, etc. (Watts, J.K., G.F. Deleavey, and M.J. Damha, Chemically modified siRNA: tools and applications. Drug Discov Today, 2008. 13(19-20): p. 842-55).

In some embodiments of the present disclosure, at least one nucleotide in the sense strand or antisense strand of the siRNA is a modified nucleotide. For example, the modified nucleotide includes a nucleotide group modified at the ribose group and optionally at the phosphate group, but is not limited thereto. In some preferred embodiments of the present disclosure, the modified nucleotide is a nucleotide modified at 2' position of the ribose thereof, for example, a nucleotide with the following 2' position modification: 2'-deoxy, 2'-fluoro, 2'-amino, 2'-methyl, 2'-ethyl, 2'-methyl-O-methyl, 2'-ethyl-O-methyl, 2'-O-methyl (2'-OMe), 2'-O-ethyl, 2'-O-methoxyethyl (2'-O-MOE), 2'-methoxyethyl (2'-MOE), 2'-O-ethyl-O-methyl or 2'-O-allyl.

In some embodiments of the present disclosure, at least one nucleotide in the sense strand of the siRNA is a modified nucleotide. In some embodiments of the present disclosure, all nucleotides in the sense strand of the siRNA are modified nucleotides. In some embodiments of the present disclosure, at least one nucleotide in the antisense strand of the siRNA is a modified nucleotide. In some embodiments of the present disclosure, all nucleotides in the antisense strand of the siRNA are modified nucleotides.

In some embodiments of the present disclosure, the modified nucleotides are selected from at least one of 2'-methoxy nucleotides, 2'-fluoro nucleotides, 2'-deoxy nucleotides, 2'-methoxyethyl nucleotides, 2'-amino nucleotides, 2'-alkyl nucleotides, and 3'-methoxy nucleotides, but is not limited thereto. In some embodiments of the present disclosure, the modified nucleotides are selected from 2'-methoxy nucleotides, and 2'-fluoro nucleotides.

In some embodiments of the present disclosure, the modified nucleotide is a nucleotide modified with a phosphate analog. In some embodiments of the present disclosure, nucleotides modified with phosphate analogs

may include (E)-vinylphosphonate ((E)-VP) modified nucleotides, (Z)-vinylphosphonate ((Z)-VP) modified nucleotides, phosphorothioate (Ps) modified nucleotides, Sp-configured phosphorothioate modified nucleotides, Rp-configured phosphorothioate modified nucleotides (Anastasia Khvorova, Jonathan K. Watts, The chemical evolution of oligonucleotide therapies of clinical utility. Nature Biotechnology, 2017, 35(3): p. 238-248) or mesyl phosphoramidate (MsPA) modified nucleotides, etc.

In some embodiments of the present disclosure, the nucleotide analog refers to a group that can replace a nucleotide in a nucleic acid but has a base structure different from that of adenosine ribonucleotide, guanine ribonucleotide, cytosine ribonucleotide, uracil ribonucleotide or thymine deoxyribonucleotide. In some embodiments of the present disclosure, these nucleotide analogs may include locked nucleic acid (LNA), unlocked nucleic acid (UNA) or glycerol nucleic acid (GNA). In some embodiments of the present disclosure, the nucleotide analog includes locked nucleic acid (LNA) or glycerol nucleic acid (GNA). In the present disclosure, unless otherwise specified, each nucleotide may have one or more modifications at the same time, for example, one nucleotide may be a 2'-fluoro nucleotide and a (E)-vinylphosphonate ((E)-VP) modified nucleotide at the same time.

The inventors of the present disclosure find that the preferred siRNA of the present disclosure achieves surprising stability and gene silencing efficiency, satisfactory cytotoxicity and immunostimulatory activity in animal experiments.

In some embodiments of the present disclosure, all nucleotides in the sense strand and/or antisense strand of the siRNA are modified nucleotides or nucleotide analogs.

In some embodiments of the present disclosure, each nucleotide in the sense strand and antisense strand of the siRNA may independently be a 2'-methoxy nucleotide, a 2'-fluoro nucleotide or a 2'-deoxy nucleotide or a phosphorothioate nucleotide (a nucleotide containing a phosphorothioate group, such as an Rp-configured phosphorothioate group or an Sp-configured phosphorothioate group).

In some embodiments of the present disclosure, three, four, five, or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at position 11 and position 13 are both 2'-fluoro nucleotides.

In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 9, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 9, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 7, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 5, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 5, 11, 12 and 13 are all 2'-fluoro nucleotides.

In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 3, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 1, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand,

and the nucleotides at positions 1, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 1, 3, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 1, 5, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 1, 5, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 1, 7, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 1, 9, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 1, 9, 11, 12 and 13 are all 2'-fluoro nucleotides. In some embodiments of the present disclosure, four, five or six nucleotides in the sense strand are 2'-fluoro nucleotides; the 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 5, 9, 11, 12 and 13 are all 2'-fluoro nucleotides.

In some embodiments of the present disclosure, four nucleotides in the sense strand are 2'-fluoro nucleotides; the four 2'-fluoro nucleotides are at positions 11, 12, 13 and 15 in the direction from the 3' end to the 5' end of the sense strand.

In some embodiments of the present disclosure, five nucleotides in the sense strand are 2'-fluoro nucleotides; the five 2'-fluoro nucleotides are at positions 9, 11, 12, 13 and 15 in the direction from the 3' end to the 5' end of the sense strand.

In some embodiments of the present disclosure, six nucleotides in the sense strand are 2'-fluoro nucleotides; the six 2'-fluoro nucleotides are at positions 1, 5, 9, 11, 12 and 13 in the direction from the 3' end to the 5' end of the sense strand. In some embodiments of the present disclosure, six nucleotides in the sense strand are 2'-fluoro nucleotides; the six 2'-fluoro nucleotides are at positions 5, 9, 11, 12, 13 and 15 in the direction from the 3' end to the 5' end of the sense strand.

In some embodiments of the present disclosure, four, five, six or seven nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides. In some embodiments of the present disclosure, four, five, six or seven nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14, 16 and optionally 22 are all 2'-fluoro nucleotides. In some embodiments of the present disclosure, four, five, six or seven nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 23 in the direction from the 5' end to the 3' end of the antisense strand, and the nucleotides at positions 2, 6, 14, 16 and optionally 22 are all 2'-fluoro nucleotides. In some embodiments of the present disclosure, four, five, six or seven nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 24 in the direction from the 5' end to the 3' end of the antisense strand, and the nucleotides at positions 2, 6, 14, 16 and optionally 22 are all 2'-fluoro nucleotides. In some embodiments of the present disclosure, four, five, six or seven nucleotides in the antisense strand are 2'-fluoro nucleotides; the 2'-fluoro nucleotides are selected from the nucleotides at positions 2, 4, 6, 7, 8, 9, 10, 14, 16, 18, 20, 21, and optionally 25 in the direction from the 5' end to the 3' end of the antisense strand, and the nucleotides at positions 2, 6, 14, 16 and optionally 22 are all 2'-fluoro nucleotides. In some embodiments of the present disclosure, four, five, six or seven nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 26 in the direction from the 5' end to the 3' end of the antisense strand, and the nucleotides at positions 2, 6, 14 and 16 are all 2'-fluoro nucleotides.

In some embodiments of the present disclosure, five nucleotides in the antisense strand are 2'-fluoro nucleotides; the 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14,

and 16 are all 2'-fluoro nucleotides.

In some embodiments of the present disclosure, five nucleotides in the antisense strand are 2'-fluoro nucleotides; the five 2'-fluoro nucleotides are nucleotides at positions 2, 6, 14, 16, and optionally 22 in the direction from the 5' end to the 3' end of the antisense strand. In some embodiments of the present disclosure, five nucleotides in the antisense strand are 2'-fluoro nucleotides; the five 2'-fluoro nucleotides are nucleotides at positions 2, 6, 14, 16, and optionally 23 in the direction from the 5' end to the 3' end of the antisense strand. In some embodiments of the present disclosure, five nucleotides in the antisense strand are 2'-fluoro nucleotides; the five 2'-fluoro nucleotides are nucleotides at positions 2, 6, 14, 16, and optionally 24 in the direction from the 5' end to the 3' end of the antisense strand. In some embodiments of the present disclosure, five nucleotides in the antisense strand are 2'-fluoro nucleotides; the five 2'-fluoro nucleotides are nucleotides at positions 2, 6, 14, 16, and optionally 25 in the direction from the 5' end to the 3' end of the antisense strand. In some embodiments of the present disclosure, five nucleotides in the antisense strand are 2'-fluoro nucleotides; the five 2'-fluoro nucleotides are nucleotides at positions 2, 6, 14, 16, and optionally 26 in the direction from the 5' end to the 3' end of the antisense strand. In some embodiments of the present disclosure, five nucleotides in the antisense strand are 2'-fluoro nucleotides; the five 2'-fluoro nucleotides are at positions 2, 6, 14, 16 and 22 in the direction from the 5' end to the 3' end of the antisense strand.

In some embodiments of the present disclosure, six nucleotides in the antisense strand are 2'-fluoro nucleotides; the six 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14, 16 and optionally 22 are all 2'-fluoro nucleotides.

In some embodiments of the present disclosure, six nucleotides in the antisense strand are 2'-fluoro nucleotides; the six 2'-fluoro nucleotides are selected from the nucleotides at positions 2, 4, 6, 7, 8, 10, 14, 16, 18, 20, and optionally 22 in the direction from the 5' end to the 3' end of the antisense strand, and the nucleotides at positions 2, 6, 14, 16 and optionally 22 are all 2'-fluoro nucleotides. In some embodiments of the present disclosure, six nucleotides in the antisense strand are 2'-fluoro nucleotides; the nucleotides at positions 2, 6, 7, 14, 16 and 22 in the direction from the 5' end to the 3' end of the antisense strand are all 2'-fluoro nucleotides.

In some embodiments of the present disclosure, seven nucleotides in the antisense strand are 2'-fluoro nucleotides; the seven 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14, 16 and optionally 22 are all 2'-fluoro nucleotides.

In some embodiments of the present disclosure, four, five or six nucleotides in the antisense strand are 2'-fluoro nucleotides; the 2'-fluoro nucleotides are selected from the nucleotides at positions 2, 4, 6, 7, 8, 10, 14, 16, 18, 20, and optionally 22 in the direction from the 5' end to the 3' end of the antisense strand, and the nucleotides at positions 2, 6, 14 and 16 are all 2'-fluoro nucleotides. In some embodiments of the present disclosure, four, five or six nucleotides in the antisense strand are 2'-fluoro nucleotides; the 2'-fluoro nucleotides are selected from the nucleotides at positions 2, 4, 6, 7, 8, 10, 14, 16, 18, 20, and optionally 22 in the direction from the 5' end to the 3' end of the antisense strand, and the nucleotides at positions 2, 6, 14, 16 and optionally 22 are all 2'-fluoro nucleotides. In some embodiments of the present disclosure, four or five nucleotides in the antisense strand are 2'-fluoro nucleotides; the 2'-fluoro nucleotides are selected from the nucleotides at positions 2, 4, 6, 7, 8, 10, 14, 16, 18, 20, and optionally 22 in the direction from the 5' end to the 3' end of the antisense strand, and the nucleotides at positions 2, 6, 14 and 16 are all 2'-fluoro nucleotides.

In some embodiments of the present disclosure, four nucleotides in the antisense strand are 2'-fluoro nucleotides; the four 2'-fluoro nucleotides are at positions 2, 6, 14 and 16 in the direction from the 5' end to the 3' end of the antisense strand. In some embodiments of the present disclosure, five or six nucleotides in the antisense strand are 2'-fluoro nucleotides; the 2'-fluoro nucleotides are selected from the nucleotides at positions 2, 4, 6, 7, 8, 10, 14, 16, 18, 20, and optionally 22 in the direction from the 5' end to the 3' end of the antisense strand, and the nucleotides at position 20 and optionally position 22 are both 2'-fluoro nucleotides. In some embodiments of the present disclosure, five or six nucleotides in the antisense strand are 2'-fluoro nucleotides; the 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 4, 8, 20 and optionally position 22 are all 2'-fluoro nucleotides.

In some embodiments of the present disclosure, in the siRNA, the sense strand comprises at least four 2'-fluoro nucleotides at positions 11, 12, 13 and 15 in the direction from the 3' end to the 5' end thereof; the antisense strand comprises at least four 2'-fluoro nucleotides at positions 2, 6, 14 and 16 in the direction from the 5' end to the 3' end thereof.

In some embodiments of the present disclosure, four nucleotides in the sense strand are 2'-fluoro nucleotides, and the four 2'-fluoro nucleotides are at positions 11, 12, 13 and 15 in the direction from the 3' end to the 5' end of the sense strand; four nucleotides in the antisense strand are 2'-fluoro nucleotides, and the four 2'-fluoro nucleotides are at positions 2, 6, 14 and 16 in the direction from the 5' end to the 3' end of the antisense strand.

In some embodiments of the present disclosure, in the siRNA, the sense strand comprises at least four 2'-fluoro nucleotides, and the 2'-fluoro nucleotides are at positions 11, 12, 13 and 15 as well as optionally at one or more of positions 1, 3, 5, 7, 9 and 19 in the direction from the 3' end to the 5' end of the sense strand; the antisense strand comprises at least

five 2'-fluoro nucleotides, and the 2'-fluoro nucleotides are at positions 2, 6, 14 and 16 as well as at least one of positions 3, 4, 5, 7, 8, 9, 10, 18, 20, 21, 22, 23, 24, 25, and 26 in the direction from the 5' end to the 3' end of the antisense strand.

In some embodiments of the present disclosure, in the siRNA, the sense strand comprises at least five 2'-fluoro nucleotides, and the 2'-fluoro nucleotides are at positions 11, 12, 13 and 15 as well as at least one of positions 1, 3, 5, 6, 7, 9 and 19 (for example, at least one of positions 5 and 9) in the direction from the 3' end to the 5' end of the sense strand; the antisense strand comprises at least four 2'-fluoro nucleotides, and the 2'-fluoro nucleotides are at position 14 as well as at least three of positions 2, 3, 4, 5, 6, 7, 8, 9, 10, 12, 16, 18, 20, 21, 22, 23, 24, 25, and 26 in the direction from the 5' end to the 3' end of the antisense strand.

In some embodiments of the present disclosure, in the siRNA, the sense strand comprises at least five 2'-fluoro nucleotides, and the 2'-fluoro nucleotides are at positions 11, 12, 13 and 15 as well as at least one of positions 1, 3, 5, 6, 7, 9 and 19 (for example, at least one of positions 5 and 9) in the direction from the 3' end to the 5' end of the sense strand; the antisense strand comprises at least five 2'-fluoro nucleotides, and the 2'-fluoro nucleotides are at positions 2, 6, 14, and 16 as well as at least one of positions 3, 4, 5, 7, 8, 9, 10, 12, 18, 20, 21, 22, 23, 24, 25, and 26 in the direction from the 5' end to the 3' end of the antisense strand. In some embodiments of the present disclosure, in the siRNA, the sense strand comprises at least five 2'-fluoro nucleotides, and the 2'-fluoro nucleotides are at positions 9, 11, 12, 13 and 15 as well as optionally at one or more of positions 1, 3, 5, 7 and 19 in the direction from the 3' end to the 5' end of the sense strand; the antisense strand comprises at least five 2'-fluoro nucleotides, and the 2'-fluoro nucleotides are at positions 2, 6, 14, 16 and 22 as well as optionally at one or more of positions 3, 4, 5, 7, 8, 9, 10, 18, 20, 21, 23, 24, 25, and 26 in the direction from the 5' end to the 3' end of the antisense strand.

In some embodiments of the present disclosure, in the siRNA, the sense strand comprises at least six 2'-fluoro nucleotides; the 2'-fluoro nucleotides are at positions 11, 12, 13 and 15 as well as at least two of positions 1, 3, 5, 6, 7, 9 and 19 (for example, at least two of positions 1, 5, and 9) in the direction from the 3' end to the 5' end of the sense strand.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 22 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 22 in the direction from the 5' end to the 3' end of the antisense strand.

In some embodiments of the present disclosure, five nucleotides in the sense strand are 2'-fluoro nucleotides, and the five 2'-fluoro nucleotides are the nucleotides at positions 9, 11, 12, 13 and 15 in the direction from the 3' end to the 5' end of the sense strand; five nucleotides in the antisense strand are 2'-fluoro nucleotides, and the five 2'-fluoro nucleotides are the nucleotides at positions 2, 6, 14, 16 and 22 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 23 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 23 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 24 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 24 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 25 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 25 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 26 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are at positions 2, 6, 14, 16, and optionally 26 in the direction from the 5' end to the 3' end of the antisense strand.

In some embodiments of the present disclosure, five nucleotides in the sense strand are 2'-fluoro nucleotides, and the five 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; six nucleotides in the antisense strand are 2'-fluoro nucleotides, and the six 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 2, 6, 14 and 16 are all 2'-fluoro nucleotides.

In some embodiments of the present disclosure, five nucleotides in the sense strand are 2'-fluoro nucleotides, and the five 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; six nucleotides in the antisense strand are 2'-fluoro nucleotides, and the six 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 2, 6, 14, 16 and 22 are all 2'-fluoro

nucleotides. In some embodiments of the present disclosure, five nucleotides in the sense strand are 2'-fluoro nucleotides, and the five 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; six nucleotides in the antisense strand are 2'-fluoro nucleotides, and the six 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides.

In some embodiments of the present disclosure, six nucleotides in the sense strand are 2'-fluoro nucleotides, and the six 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides.

In some embodiments of the present disclosure, six nucleotides in the sense strand are 2'-fluoro nucleotides, and the six 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; five nucleotides in the antisense strand are 2'-fluoro nucleotides, and the five 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 2, 6, 14 and 16 are all 2'-fluoro nucleotides. In some embodiments of the present disclosure, six nucleotides in the sense strand are 2'-fluoro nucleotides, and the six 2'-fluoro nucleotides are the nucleotides at positions 5, 9, 11, 12, 13 and 15 in the direction from the 3' end to the 5' end of the sense strand; five nucleotides in the antisense strand are 2'-fluoro nucleotides, and the five 2'-fluoro nucleotides are the nucleotides at positions 2, 6, 14, 16 and 22 in the direction from the 5' end to the 3' end of the antisense strand.

In some embodiments of the present disclosure, six nucleotides in the sense strand are 2'-fluoro nucleotides, and the six 2'-fluoro nucleotides are selected from the nucleotides at positions 1, 3, 5, 6, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; six nucleotides in the antisense strand are 2'-fluoro nucleotides, and the six 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides. In some embodiments of the present disclosure, six nucleotides in the sense strand are 2'-fluoro nucleotides, and the six 2'-fluoro nucleotides are selected from the nucleotides at positions 1, 3, 5, 7, 9, 11, 12, 13, 15, 17, 19, and optionally 21 in the direction from the 3' end to the 5' end of the sense strand, and the nucleotides at positions 11, 12 and 13 are all 2'-fluoro nucleotides; six nucleotides in the antisense strand are 2'-fluoro nucleotides, and the six 2'-fluoro nucleotides are selected from the nucleotides at positions 2, 4, 6, 8, 10, 14, 16, 18, and optionally 22 in the direction from the 5' end to the 3' end of the antisense strand, and the nucleotides at positions 2, 6, 14 and 16 are all 2'-fluoro nucleotides.

In some embodiments of the present disclosure, six nucleotides in the sense strand are 2'-fluoro nucleotides, and the six 2'-fluoro nucleotides are at positions 5, 9, 11, 12, 13 and 15 in the direction from the 3' end to the 5' end of the sense strand; six nucleotides in the antisense strand are 2'-fluoro nucleotides, and the six 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides.

In some embodiments of the present disclosure, six nucleotides in the sense strand are 2'-fluoro nucleotides, and the six 2'-fluoro nucleotides are at positions 5, 9, 11, 12, 13 and 15 in the direction from the 3' end to the 5' end of the sense strand; six nucleotides in the antisense strand are 2'-fluoro nucleotides, and the six 2'-fluoro nucleotides are selected from the nucleotides at positions 2, 4, 6, 8, 10, 14, 16, 18, 20, and optionally 22 in the direction from the 5' end to the 3' end of the antisense strand, and the nucleotides at positions 2, 6, 14 and 16 are all 2'-fluoro nucleotides.

In some embodiments of the present disclosure, six nucleotides in the sense strand are 2'-fluoro nucleotides, and the six 2'-fluoro nucleotides are at positions 5, 9, 11, 12, 13 and 15 in the direction from the 3' end to the 5' end of the sense strand; six nucleotides in the antisense strand are 2'-fluoro nucleotides, and the six 2'-fluoro nucleotides are selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14, 16 and 22 are all 2'-fluoro nucleotides.

In some embodiments of the present disclosure, all nucleotides of the sense strand and the antisense strand of the siRNA are each independently 2'-methoxy nucleotides or 2'-fluoro nucleotides.

In some embodiments of the present disclosure, at least the nucleotides at positions 11, 12, and 13 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 11, 12, and 13 in the direction from the 3' end to the 5' end of the sense strand are 2'-fluoro nucleotides; the nucleotides at other positions of the sense strand may be 2'-methoxy nucleotides. In some embodiments of the present disclosure, the

nucleotides at positions 11, 12, 13 and 15 in the direction from the 3' end to the 5' end of the sense strand are 2'-fluoro nucleotides; the nucleotides at other positions of the sense strand may be 2'-methoxy nucleotides.

In some embodiments of the present disclosure, the nucleotides at positions 1, 3, 5, 6, 7, 9, 11, 12, 13, 15, 17, 19 and optionally 21 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 1, 3, 5, 7, 9, 11, 12, 13, 15, 17, 19 and optionally 21 in the direction from the 3' end to the 5' end of the sense strand are 2'-fluoro nucleotides; the nucleotides at other positions of the sense strand may be 2'-methoxy nucleotides.

In some embodiments of the present disclosure, the nucleotides at positions 1, 3, 5, 6, 7, 9, 11, 12, 13, 15, 17, 19, and 21 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 1, 3, 5, 7, 9, 11, 12, 13, 15, 17, 19, and 21 in the direction from the 3' end to the 5' end of the sense strand are 2'-fluoro nucleotides; the nucleotides at other positions of the sense strand may be 2'-methoxy nucleotides.

In some embodiments of the present disclosure, the nucleotides at positions 11, 12, 13, and 15 in the direction from the 3' end to the 5' end of the sense strand are 2'-fluoro nucleotides; at least one nucleotide at positions 1, 3, 5, 6, 7, 9, 17, 19, and 21 may be a 2'-fluoro nucleotide. In some embodiments of the present disclosure, the nucleotides at positions 11, 12, 13, and 15 in the direction from the 3' end to the 5' end of the sense strand are 2'-fluoro nucleotides; at least one nucleotide at positions 1, 3, 5, 7, and 9 may be a 2'-fluoro nucleotide. In some embodiments of the present disclosure, the nucleotides at positions 11, 12, 13, and 15 in the direction from the 3' end to the 5' end of the sense strand are 2'-fluoro nucleotides; one of the nucleotides at positions 1, 3, 5, 6, 7, 9, 17, 19, and 21 may be a 2'-fluoro nucleotide. In some embodiments of the present disclosure, the nucleotides at positions 11, 12, 13, and 15 in the direction from the 3' end to the 5' end of the sense strand are 2'-fluoro nucleotides; one of the nucleotides at positions 1, 3, 5, 7, and 9 may be a 2'-fluoro nucleotide. In some embodiments of the present disclosure, the nucleotides at positions 11, 12, 13, and 15 in the direction from the 3' end to the 5' end of the sense strand are 2'-fluoro nucleotides; at least two nucleotides at positions 1, 3, 5, 6, 7, 9, 17, 19, and 21 may be 2'-fluoro nucleotides. In some embodiments of the present disclosure, the nucleotides at positions 11, 12, 13, and 15 in the direction from the 3' end to the 5' end of the sense strand are 2'-fluoro nucleotides; at least two nucleotides at positions 1, 3, 5, 7, and 9 may be 2'-fluoro nucleotides. In some embodiments of the present disclosure, the nucleotides at positions 11, 12, 13, and 15 in the direction from the 3' end to the 5' end of the sense strand are 2'-fluoro nucleotides; two of the nucleotides at positions 1, 3, 5, 7, and 9 may be 2'-fluoro nucleotides.

In some embodiments of the present disclosure, the nucleotides at positions 9, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 6, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 11, 12, 13, 15, and optionally 21 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 11, 12, 13, 15, and 21 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 11, 12, 13, 15, and 19 in the direction from the 3' end to the 5' end of the sense strand are 2'-fluoro nucleotides; the nucleotides at other positions of the sense strand may be 2'-methoxy nucleotides. In some embodiments of the present disclosure, the nucleotides at positions 11, 12, 13, 15, and 17 in the direction from the 3' end to the 5' end of the sense strand are 2'-fluoro nucleotides; the nucleotides at other positions of the sense strand may be 2'-methoxy nucleotides.

In some embodiments of the present disclosure, at least the nucleotides at positions 2, 6, 14, and 16 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, and 16 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 8, 9, 14, and 16 in the direction from the 5' end to the 3' end of the antisense

strand are 2'-fluoro nucleotides; the nucleotides at other positions of the antisense strand may be 2'-methoxy nucleotides.

In some embodiments of the present disclosure, the nucleotides at positions 2, 3, 4, 5, 6, 7, 8, 9, 10, 14, 16, 18, 20, 21, 22, 23, 24, 25, and 26 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 6, 8, 10, 14, 16, 18, and optionally 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 4, 5, 6, 7, 8, 9, 10, 14, 16, 18, 20, 21, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 6, 8, 10, 14, 16, 18, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 4, 5, 6, 7, 8, 9, 10, 14, 16, 18, 20, 21, and 23 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 4, 5, 6, 7, 8, 9, 10, 14, 16, 18, 20, 21, and 24 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 4, 5, 6, 7, 8, 9, 10, 14, 16, 18, 20, 21, and 25 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 4, 5, 6, 7, 8, 9, 10, 14, 16, 18, 20, 21, and 26 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, and 16 in the direction from the 5' end to the 3' end of the antisense strand are 2'-fluoro nucleotides; at least one nucleotide at positions 3, 4, 5, 7, 8, 9, 10, 18, 20, 21, and 22 is a 2'-fluoro nucleotide. In some embodiments of the present disclosure, the nucleotides at positions 2, 6, 14, and 16 in the direction from the 5' end to the 3' end of the antisense strand are 2'-fluoro nucleotides; at least one nucleotide at positions 4, 8, 10, 18, 20, and 22 is a 2'-fluoro nucleotide. In some embodiments of the present disclosure, the nucleotides at positions 2, 6, 14, and 16 in the direction from the 5' end to the 3' end of the antisense strand are 2'-fluoro nucleotides; one of the nucleotides at positions 3, 4, 5, 7, 8, 9, 10, 18, 20, 21, and 22 is a 2'-fluoro nucleotide. In some embodiments of the present disclosure, the nucleotides at positions 2, 6, 14, and 16 in the direction from the 5' end to the 3' end of the antisense strand are 2'-fluoro nucleotides; one of the nucleotides at positions 4, 8, 10, 18, 20, and 22 is a 2'-fluoro nucleotide. In some embodiments of the present disclosure, the nucleotides at positions 2, 6, 14, and 16 in the direction from the 5' end to the 3' end of the antisense strand are 2'-fluoro nucleotides; at least two nucleotides at positions 3, 4, 5, 7, 8, 9, 10, 18, 20, 21, and 22 are 2'-fluoro nucleotides. In some embodiments of the present disclosure, the nucleotides at positions 2, 6, 14, and 16 in the direction from the 5' end to the 3' end of the antisense strand are 2'-fluoro nucleotides; at least two nucleotides at positions 4, 8, 10, 18, 20, and 22 are 2'-fluoro nucleotides. In some embodiments of the present disclosure, the nucleotides at positions 2, 6, 14, and 16 in the direction from the 5' end to the 3' end of the antisense strand are 2'-fluoro nucleotides; two of the nucleotides at positions 3, 4, 5, 7, 8, 9, 10, 18, 20, 21, and 22 are 2'-fluoro nucleotides. In some embodiments of the present disclosure, the nucleotides at positions 2, 6, 14, and 16 in the direction from the 5' end to the 3' end of the antisense strand are 2'-fluoro nucleotides; two of the nucleotides at positions 4, 8, 10, 18, 20, and 22 are 2'-fluoro nucleotides. In some embodiments of the present disclosure, the nucleotides at positions 2, 6, 14, and 16 in the direction from the 5' end to the 3' end of the antisense strand are 2'-fluoro nucleotides; at least three nucleotides at positions 3, 4, 5, 7, 8, 9, 10, 18, 20, 21, and 22 are 2'-fluoro nucleotides.

In some embodiments of the present disclosure, the nucleotides at positions 2, 6, 10, 14, and 16 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 18 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 20 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 21 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 23 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 24 in the direction from the 5' end to the 3' end of the antisense strand are 2'-fluoro nucleotides; the nucleotides at other positions of

the antisense strand may be 2'-methoxy nucleotides. In some embodiments of the present disclosure, the nucleotides at positions 2, 6, 14, 16, and 25 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, and 26 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 6, 14, and 16 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 8, 14, and 16 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 16, 20, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 8, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 6, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 10, 14, 16, and 22 in the direction from he 5' end to the 3' end of the antisense strand are 2'-fluoro nucleotides; the nucleotides at other positions of the antisense strand may be 2'-methoxy nucleotides. In some embodiments of the present disclosure, the nucleotides at positions 4, 6, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 6, 8, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 6, 14, 16, 18, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 6, 14, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 8, 14, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 14, 18, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 4, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 8, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 6, 9, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 9, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 9, 14, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 4, 6, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 12, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 8, 9, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 3, 6, 10, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 5, 6, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 14, 16, and 22 in the direction from the 5' end to the 3' end of the anti-sense strand are 2'-fluoro

nucleotides; the nucleotides at other positions of the antisense strand may be 2'-methoxy nucleotides. In some embodiments of the present disclosure, the nucleotides at positions 2, 6, 9, 10, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 10, 14, 16, and 22 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 14, 16, and 20 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 10, 14, 16, and 20 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 14, 16, and 21 in the direction from the 5' end to the 3' end of the antisense strand are 2'-fluoro nucleotides; the nucleotides at other positions of the antisense strand may be 2'-methoxy nucleotides. In some embodiments of the present disclosure, the nucleotides at positions 2, 6, 7, 14, 16, and 23 in the direction from the 5' end to the 3' end of the anti-sense strand are 2'-fluoro nucleotides; the nucleotides at other positions of the antisense strand may be 2'-methoxy nucleotides.

In some embodiments of the present disclosure, the nucleotides at positions 11, 12, and 13 in the direction from the 3' end to the 5' end of the sense strand are 2'-fluoro nucleotides, and the nucleotides at other positions of the sense strand may be 2'-methoxy nucleotides; the nucleotides at positions 2, 6, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 9, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 7, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 6, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at

positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxyl nucleotides. In some embodiments of the present disclosure, the nucleotides at positions 3, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure,

the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 12, 13, 15, and 21 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 1, 11, 12, 13, 15, and 21 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, 15, and 21 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 1, 11, 12, 13, 15, and 21 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, 15, and 21 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 12, 13, 15, and 19 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 1, 11, 12, 13, 15, and 19 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, 15, and 19 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 1, 11, 12, 13, 15, and 19 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some

embodiments of the present disclosure, the nucleotides at positions 1, 11, 12, 13, 15, and 19 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 12, 13, 15, and 17 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 1, 11, 12, 13, 15, and 17 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, 15, and 17 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 1, 11, 12, 13, 15, and 17 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 1, 11, 12, 13, 15, and 17 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 5, 6, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 9, 14, and 16 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, and 16 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-

methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, 20, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxyl nucleotides; the nucleotides at positions 2, 4, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 4, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 8, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 14, 16, 18, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 18, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 8, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 9, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 9, 14, 16, and 22 in the direction from the 5' end to the 3' end of the antisense strand are 2'-fluoro nucleotides, and the nucleotides at other positions of the antisense strand may be 2'-methy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at

positions 2, 6, 9, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 9, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 3, 6, 10, 14, 16, and 22 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 5, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 7, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 9, 10, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 26 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 25 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 24 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 23 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 7, 10, 14, 16, and 18 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 10, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in

the direction from the 3' end to the 5' end of the sense strand are 2'-fluoro nucleotides, and the nucleotides at other positions of the sense strand may be 2'-methoxy nucleotides; the nucleotides at positions 2, 6, 7, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 7, 10, 14, 16, and 20 in the direction from the 5' end to the 3' end of the anti-sense strand are 2'-fluoro nucleotides, and the nucleotides at other positions of the anti-sense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 7, 14, 16, and 21 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 7, 14, 16, and 23 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 4, 6, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 20 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 8, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 14, 16, 18, and 22 in the direction from the 5' end to the 3' end of the antisense strand are 2'-fluoro nucleotides, and the nucleotides at other positions of the anti-sense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 6, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 18, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 4, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 8, 14, 16, and 22 in the direction from the 5' end to the 3' end of the

antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 6, 9, 14, 16, and 22 in the direction from the 5' end to the 3' end of the antisense strand are 2'-fluoro nucleotides, and the nucleotides at other positions of the antisense strand may be 2'-methy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 9, 14, 16, and 22 in the direction from the 5' end to the 3' end of the antisense strand are 2'-fluoro nucleotides, and the nucleotides at other positions of the anti-sense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 9, 14, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 8, 9, 14, 16, and 22 in the direction from the 5' end to the 3' end of the antisense strand are 2'-fluoro nucleotides, and the nucleotides at other positions of the antisense strand may be 2'-methoxy nucleotides. In some embodiments of the present disclosure, the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 9, 10, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 1, 9, 11, 12, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 13, and 15 in the direction from the 3' end to the 5' end of the sense strand are 2'-fluoro nucleotides, and the nucleotides at other positions of the sense strand may be 2'-methoxy nucleotides; the nucleotides at positions 2, 6, 12, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 9, 11, and 13 in the direction from the 3' end to the 5' end of the sense strand are 2'-fluoro nucleotides, and the nucleotides at other positions of the sense strand may be 2'-methoxy nucleotides; the nucleotides at positions 2, 6, 12, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 5, 11, and 13 in the direction from the 3' end to the 5' end of the sense strand are 2'-fluoro nucleotides, and the nucleotides at other positions of the sense strand may be 2'-methoxy nucleotides; the nucleotides at positions 2, 6, 12, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 5, 11, and 13 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, the nucleotides at positions 11, 12, and 13 in the direction from the 3' end to the 5' end of the sense strand are 2'-fluoro nucleotides, and the nucleotides at other positions of the sense strand may be 2'-methoxy nucleotides; the nucleotides at positions 2, 6, 14, 16, and 22 in the direction from the 5' end to the 3' end of the antisense strand are 2'-fluoro nucleotides, and the nucleotides at other positions of the antisense strand may be 2'-methoxy nucleotides.

In some embodiments of the present disclosure, at least one of linkages between the nucleotide at position 1 and the nucleotide at position 2 at the 5' end of the sense strand, between the nucleotide at position 2 and the nucleotide at position 3 at the 5' end of the sense strand, between the nucleotide at position 1 and the nucleotide at position 2 at the 3' end of the antisense strand, between the nucleotide at position 2 and the nucleotide at position 3 at the 3' end of the antisense strand, between the nucleotide at position 1 and the nucleotide at position 2 at the 5' end of the antisense strand, and

between the nucleotide at position 2 and the nucleotide at position 3 at the 5' end of the antisense strand is a phosphorothioate linkage, an Rp-configured phosphorothioate linkage, or an Sp-configured phosphorothioate linkage. In some embodiments of the present disclosure, at least four or six of linkages between the nucleotide at position 1 and the nucleotide at position 2 at the 5' end of the sense strand, between the nucleotide at position 2 and the nucleotide at position 3 at the 5' end of the sense strand, between the nucleotide at position 1 and the nucleotide at position 2 at the 3' end of the antisense strand, between the nucleotide at position 2 and the nucleotide at position 3 at the 3' end of the antisense strand, between the nucleotide at position 1 and the nucleotide at position 2 at the 5' end of the antisense strand, and between the nucleotide at position 2 and the nucleotide at position 3 at the 5' end of the antisense strand are phosphorothioate linkages, Rp-configured phosphorothioate linkages, or Sp-configured phosphorothioate linkages.

In some embodiments of the present disclosure, the linkages between the nucleotide at position 1 and the nucleotide at position 2 at the 5' end of the sense strand and between the nucleotide at position 2 and the nucleotide at position 3 at the 5' end of the sense strand are phosphorothioate linkages, Rp-configured phosphorothioate linkages, or Sp-configured phosphorothioate linkages. In some embodiments of the present disclosure, the linkages between the nucleotide at position 1 and the nucleotide at position 2 at the 5' end of the sense strand and between the nucleotide at position 2 and the nucleotide at position 3 at the 5' end of the sense strand are phosphorothioate linkages, Rp-configured phosphorothioate linkages, or Sp-configured phosphorothioate linkages; and the linkages between the nucleotide at position 1 and the nucleotide at position 2 at the 3' end of the sense strand and between the nucleotide at position 2 and the nucleotide at position 3 at the 3' end of the sense strand are phosphorothioate linkages, Rp-configured phosphorothioate linkages, or Sp-configured phosphorothioate linkages. In some embodiments of the present disclosure, the linkages between the nucleotide at position 1 and the nucleotide at position 2 at the 5' end of the antisense strand and between the nucleotide at position 2 and the nucleotide at position 3 at the 5' end of the antisense strand are phosphorothioate linkages, Rp-configured phosphorothioate linkages, or Sp-configured phosphorothioate linkages. In some embodiments of the present disclosure, the linkages between the nucleotide at position 1 and the nucleotide at position 2 at the 3' end of the antisense strand and between the nucleotide at position 2 and the nucleotide at position 3 at the 3' end of the antisense strand are phosphorothioate linkages, Rp-configured phosphorothioate linkages, or Sp-configured phosphorothioate linkages. In some embodiments of the present disclosure, the linkages between the nucleotide at position 1 and the nucleotide at position 2 at the 3' end of the anti-sense strand and between the nucleotide at position 2 and the nucleotide at position 3 at the 3' end of the antisense strand are phosphorothioate linkages, Rp-configured phosphorothioate linkages, or Sp-configured phosphorothioate linkages; and the linkages between the nucleotide at position 1 and the nucleotide at position 2 at the 5' end of the antisense strand and between the nucleotide at position 2 and the nucleotide at position 3 at the 5' end of the antisense strand are phosphorothioate linkages, Rp-configured phosphorothioate linkages, or Sp-configured phosphorothioate linkages.

In some embodiments of the present disclosure, all the linkages between the nucleotide at position 1 and the nucleotide at position 2 at the 3' end of the antisense strand, between the nucleotide at position 2 and the nucleotide at position 3 at the 3' end of the antisense strand, between the nucleotide at position 3 and the nucleotide at position 4 at the 3' end of the antisense strand, and between the nucleotide at position 4 and the nucleotide at position 5 at the 3' end of the antisense strand are phosphorothioate linkages, Rp-configured phosphorothioate linkages, or Sp-configured phosphorothioate linkages; and the linkages between the nucleotide at position 1 and the nucleotide at position 2 at the 5' end of the anti-sense strand and between the nucleotide at position 2 and the nucleotide at position 3 at the 5' end of the antisense strand are phosphorothioate linkages, Rp-configured phosphorothioate linkages, or Sp-configured phosphorothioate linkages.

In some embodiments of the present disclosure, the nucleotide at position 2 at the 3' end of the sense strand may be a thermally destabilizing nucleotide. In some embodiments of the present disclosure, the nucleotide at position 2 at the 3' end of the sense strand is a thermally destabilizing nucleotide.

In some embodiments of the present disclosure, at least one nucleotide among the nucleotides at positions 6, 7 and 8 at the 5' end of the antisense strand is a thermally destabilizing nucleotide. In some embodiments of the present disclosure, the nucleotide at position 6 at the 5' end of the antisense strand is a thermally destabilizing nucleotide. In some embodiments of the present disclosure, the nucleotide at position 7 at the 5' end of the antisense strand is a thermally destabilizing nucleotide. In some embodiments of the present disclosure, the nucleotide at position 8 at the 5' end of the antisense strand is a thermally destabilizing nucleotide. In some embodiments of the present disclosure, the thermally destabilizing nucleotide may be selected from LNA, UNA or GNA. In some embodiments of the present disclosure, the thermally destabilizing nucleotide may be GNA.

In some embodiments of the present disclosure, the nucleotide at position 1 at the 5' end of the antisense strand may be an (E)-VP modified nucleotide. In some embodiments of the present disclosure, the nucleotide at position 1 at the 5' end of the antisense strand is an (E)-VP modified nucleotide.

In some embodiments of the present disclosure, the sense strand has the following modified nucleotides:

(1) The nucleotides at positions 9, 11, 12, 13, and 15 in the direction from the 3' end to the 5' end of the sense strand are

2'-fluoro nucleotides; the nucleotides at other positions of the sense strand may be 2'-methoxy nucleotides;
(2) The nucleotides at positions 11, 12, 13, and 15 in the direction from the 3' end to the 5' end of the sense strand are 2'-fluoro nucleotides; one of the nucleotides at positions 1, 3, 5, 6, 7, and 19 in the direction from the 3' end to the 5' end of the sense strand may further be a 2'-fluoro nucleotide; the nucleotides at other positions of the sense strand may be 2'-methoxy nucleotides;
(3) The nucleotides at positions 1, 9, 11, 12, 13, and 15 in the direction from the 3' end to the 5' end of the sense strand are 2'-fluoro nucleotides; the nucleotides at other positions of the sense strand may be 2'-methoxy nucleotides;
(4) The nucleotides at positions 9, 11, 12, 13, and 15 in the direction from the 3' end to the 5' end of the sense strand are 2'-fluoro nucleotides, and the nucleotide at position 2 is GNA; the nucleotides at other positions of the sense strand may be 2'-methoxy nucleotides;
(5) The nucleotides at positions 5, 9, 11, 12, 13, and 15 in the direction from the 3' end to the 5' end of the sense strand are 2'-fluoro nucleotides; the nucleotides at other positions of the sense strand may be 2'-methoxy nucleotides (preferably, the nucleotides at positions 9, 11, 12, 13, and 15 in the direction from the 3' end to the 5' end of the sense strand are 2'-fluoro nucleotides, and the nucleotides at other positions of the sense strand may be 2'-methoxy nucleotides; or the nucleotides at positions 5, 11, 12, 13, and 15 in the direction from the 3' end to the 5' end of the sense strand are 2'-fluoro nucleotides, and the nucleotides at other positions of the sense strand may be 2'-methoxy nucleotides);
(6) The nucleotides at positions 11 and 13 in the direction from the 3' end to the 5' end of the sense strand are 2'-fluoro nucleotides; one of the nucleotides at positions 5, 9, 12, 15, and 19 in the direction from the 3' end to the 5' end of the sense strand may further be a 2'-fluoro nucleotide; the nucleotides at other positions of the sense strand may be 2'-methoxy nucleotides.

In some embodiments of the present disclosure, the antisense strand has the following modified nucleotides:

(1) The nucleotides at positions 2, 6, 7, 14, 16, and 22 in the direction from the 5' end to the 3' end of the antisense strand are 2'-fluoro nucleotides; the nucleotides at other positions of the antisense strand may be 2'-methoxy nucleotides; the nucleotide at position 1 in the direction from the 5' end to the 3' end of the antisense strand further has an (E)-VP modification;
(2) The nucleotides at positions 2, 6, 14, and 16 in the direction from the 5' end to the 3' end of the antisense strand are 2'-fluoro nucleotides; one or two of the nucleotides at positions 4, 8, 9, 10, 18, and 20 in the direction from the 5' end to the 3' end of the antisense strand may further be 2'-fluoro nucleotides; the nucleotides at other positions of the antisense strand may be 2'-methoxy nucleotides;
(3) The nucleotides at positions 2, 6, 14, and 16 in the direction from the 5' end to the 3' end of the antisense strand and the nucleotide at position 2 in the direction from the 3' end to the 5' end of the antisense strand are 2'-fluoro nucleotides; one or two of the nucleotides at positions 3, 4, 5, 7, 8, 9, 10, 12, and 20 in the direction from the 5' end to the 3' end of the antisense strand may further be 2'-fluoro nucleotides; the nucleotides at other positions of the antisense strand may be 2'-methoxy nucleotides; the nucleotide at position 1 in the direction from the 5' end to the 3' end of the antisense strand may further have an (E)-VP modification;
(4) The nucleotides at positions 2, 6, 7, 14, and 16 in the direction from the 5' end to the 3' end of the antisense strand and the nucleotide at position 2 in the direction from the 3' end to the 5' end of the antisense strand are 2'-fluoro nucleotides; the nucleotide at position 10 in the direction from the 5' end to the 3' end of the antisense strand may further be a 2'-fluoro nucleotide; the nucleotides at other positions of the antisense strand may be 2'-methoxy nucleotides; the nucleotide at position 1 in the direction from the 5' end to the 3' end of the antisense strand further has an (E)-VP modification;
(5) The nucleotides at positions 2, 14, and 16 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, and 9 in the direction from the 5' end to the 3' end of the antisense strand may further be a 2'-fluoro nucleotide; the nucleotides at other positions of the antisense strand may be 2'-methoxy nucleotides;
(6) The nucleotides at positions 2, 6, and 14 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, 9, and 18 in the direction from the 5' end to the 3' end of the antisense strand may further be a 2'-fluoro nucleotide; the nucleotides at other positions of the antisense strand may be 2'-methoxy nucleotides; or
(7) The nucleotides at positions 6, 14, and 16 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, 9, and 18 in the direction from the 5' end to the 3' end of the antisense strand may further be a 2'-fluoro nucleotide; the nucleotides at other positions of the antisense strand may be 2'--methoxy

nucleotides.

In some embodiments of the present disclosure, the siRNA comprises the following sense strand and antisense strand:

The nucleotides at positions 9, 11, 12, 13, and 15 in the direction from the 3' end to the 5' end of the sense strand are 2'-fluoro nucleotides, the nucleotides at other positions of the sense strand are 2'-methoxy nucleotides, and the nucleotides at positions 19 and 20, as well as the nucleotides at positions 20 and 21 in the direction from the 3' end to the 5' end of the sense strand are linked via phosphorothioate groups, and

The nucleotides at positions 2, 6, 7, 14, 16, and 22 in the direction from the 5' end to the 3' end of the antisense strand are 2'-fluoro nucleotides, the nucleotides at other positions of the antisense strand are 2'-methoxy nucleotides, the nucleotide at position 1 in the direction from the 5' end to the 3' end of the antisense strand further has an (E)-VP modification, and the nucleotides at positions 1 and 2, the nucleotides at positions 2 and 3, the nucleotides at positions 21 and 22, as well as the nucleotides at positions 22 and 23 in the direction from the 5' end to the 3' end of the antisense strand are linked via phosphorothioate groups.

In some embodiments of the present disclosure, the siRNA comprises the following sense strand and antisense strand:

The nucleotides at positions 5, 9, 11, 12, 13, and 15 in the direction from the 3' end to the 5' end of the sense strand are 2'-fluoro nucleotides; the nucleotides at other positions of the sense strand may be 2'-methoxy nucleotides, and the nucleotides at positions 19 and 20, as well as positions 20 and 21 in the direction from the 3' end to the 5' end of the sense strand are linked via phosphorothioate groups;

The nucleotides at positions 2, 6, 14, and 16 in the direction from the 5' end to the 3' end of the antisense strand and the nucleotide at position 2 in the direction from the 3' end to the 5' end of the antisense strand are 2'-fluoro nucleotides (alternatively, the nucleotides at positions 2, 6, 14, 16, and 22 in the direction from the 5' end to the 3' end of the antisense strand are 2'-fluoro nucleotides); and one or two of the nucleotides at positions 3, 4, 5, 7, 8, 9, and 10 in the direction from the 5' end to the 3' end of the antisense strand may further be 2'-fluoro nucleotides; the nucleotides at other positions of the antisense strand may be 2'-methoxy nucleotides; the nucleotide at position 1 in the direction from the 5' end to the 3' end of the antisense strand may further have an (E)-VP modification; the nucleotides at positions 1 and 2, the nucleotides at positions 2 and 3, the nucleotides at positions 21 and 22, as well as the nucleotides at positions 22 and 23 in the direction from the 5' end to the 3' end of the antisense strand are linked via phosphorothioate groups.

In some embodiments of the present disclosure, the siRNA may be selected from at least one of modified duplex 46 to modified duplex 50, modified duplex 60 to modified duplex 125, modified duplex 129 to modified duplex 130, modified duplex 133 to modified duplex 149, modified duplex 152 to modified duplex 201:

Modified duplex 46

Sense strand: 5'-mU*mC*mCmCmAmCi2FCmUi2FUi2FUi2FUmCmUmUmCmUmAmA mUmGmA-3' (SEQ ID NO: 144)
Anti-sense strand: 5'-mU*i2FC*mAmUmUi2FAmGmAmAmGmAmAmAi2FAmGi2FGm UmGmGmGmA*mG*-mA-3' (SEQ ID NO: 145);

Modified duplex 47

Sense strand: 5'-mU*mC*mCmCmAmCi2FCmUi2FUi2FUi2FUmCi2FUmUmCmUmAm AmUmGmA-3' (SEQ ID NO: 146)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Modified duplex 48

Sense strand: 5'-mU*mC*mCmCmAmCi2FCmUi2FUi2FUi2FUmCmUmUi2FCmUmAm AmUmGmA-3' (SEQ ID NO: 148)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Modified duplex 49

Sense strand: 5'-mU*mC*mCmCmAmCi2FCmUi2FUi2FUi2FUmCmUmUmCmUi2FAm AmUmGmA-3' (SEQ ID NO: 149)
Anti-sense strand: 5'-mU*i2FC*mAmUmUi2FAmGmAmAmGmAmAmAi2FAmGi2FGm UmGmGi2FGmA*mG*-mA-3' (SEQ ID NO: 150);

Modified duplex 50

Sense strand: 5'-mU*mC*i2FCmCmAmCi2FCmUi2FUi2FUi2FUmCmUmUmCmUmAm AmUmGmA-3' (SEQ ID NO: 151)
Anti-sense strand: 5'-mU*i2FC*mAmUmUi2FAmGmAmAi2FGmAmAmAi2FAmGi2FG mUmGmGmGmA*mG*-mA-3' (SEQ ID NO: 152);

Modified duplex 60

Sense strand: 5'-mU*mC*mCmCmAmCi2FCmUi2FUi2FUi2FUmCmUmUmCi2FUmAm AmUmGmA-3' (SEQ ID NO: 153)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Modified duplex 61

Sense strand: 5'-mC*mC*mAmGmUmCi2FUmCi2FCi2FCi2FAmCmCmUmUmUmUmC mUmUmU-3' (SEQ ID NO: 154)
Anti-sense strand: 5'-mA*i2FA*mAmGmAi2FAmAmAmGmGmUmGmGi2FGmAi2FGm AmCmUmGmG*mG*mG-3' (SEQ ID NO: 155);

Modified duplex 62

Sense strand: 5'-mC*mA*mGmUmCmUi2FCmCi2FCi2FAi2FCmCmUmUmUmUmCmU mUmCmU-3' (SEQ ID NO: 156)
Anti-sense strand: 5'-mA*i2FG*mAmAmGi2FAmAmAmAmGmGmUmGi2FGmGi2FAm GmAmCmUmG*mG*mG-3' (SEQ ID NO: 157);

Modified duplex 63

Sense strand: 5'-mA*mG*mUmCmUmCi2FCmCi2FAi2FCi2FCmUmUmUmUmCmUmU mCmUmA-3' (SEQ ID NO: 158)
Anti-sense strand: 5'-mU*i2FA*mGmAmAi2FGmAmAmAmAmGmGmUi2FGmGi2FGm AmGmAmCmU*mG*mG-3' (SEQ ID NO: 159);

Modified duplex 64

Sense strand: 5'-mG*mU*mCmUmCmCi2FCmAi2FCi2FCi2FUmUmUmUmCmUmUmC mUmAmA-3' (SEQ ID NO: 160)
Anti-sense strand: 5'-mU*i2FU*mAmGmAi2FAmGmAmAmAmAmGmGi2FUmGi2FGm GmAmGmAmC*-mU*mG-3' (SEQ ID NO: 161);

Modified duplex 65

Sense strand: 5'-mU*mC*mUmCmCmCi2FAmCi2FCi2FUi2FUmUmUmCmUmUmCmU mAmAmU-3' (SEQ ID NO: 162)
Anti-sense strand: 5'-mA*i2FU*mUmAmGi2FAmAmGmAmAmAmAmGi2FGmUi2FGm GmGmAmGmA*mC*-mU-3' (SEQ ID NO: 163);

Modified duplex 66

Sense strand: 5’-mC*mU*mCmCmCmAi2FCmCi2FUi2FUi2FUmUmCmUmUmCmUmA mAmUmU-3’ (SEQ ID NO: 164)
Anti-sense strand: 5’-mA*i2FA*mUmUmAi2FGmAmAmGmAmAmAmAi2FGmGi2FUm GmGmGmAmG*-mA*mC-3’ (SEQ ID NO: 165);

Modified duplex 67

Sense strand: 5’-mC*mC*mCmAmCmCi2FUmUi2FUi2FUi2FCmUmUmCmUmAmAmU mGmAmU-3’ (SEQ ID NO: 166)
Anti-sense strand: 5’-mA*i2FU*mCmAmUi2FUmAmGmAmAmGmAmAi2FAmAi2FGm GmUmGmGmG*-mA*mG-3’ (SEQ ID NO: 167);

Modified duplex 68

Sense strand: 5’-mC*mC*mAmCmCmUi2FUmUi2FUi2FCi2FUmUmCmUmAmAmUmG mAmGmU-3’ (SEQ ID NO: 168)
Anti-sense strand: 5’-mA*i2FC*mUmCmAi2FUmUmAmGmAmAmGmAi2FAmAi2FAm GmGmUmGmG*mG*-mA-3’ (SEQ ID NO: 169);

Modified duplex 69

Sense strand: 5’-mC*mA*mCmCmUmUi2FUmUi2FCi2FUi2FUmCmUmAmAmUmGmA mGmUmU-3’ (SEQ ID NO: 170)
Anti-sense strand: 5’-mA*i2FA*mCmUmCi2FAmUmUmAmGmAmAmGi2FAmAi2FAm AmGmGmUmG*mG*mG-3’ (SEQ ID NO: 171);

Modified duplex 70

Sense strand: 5’-mA*mC*mCmUmUmUi2FUmCi2FUi2FUi2FCmUmAmAmUmGmAmG mUmCmU-3’ (SEQ ID NO: 172)
Anti-sense strand: 5’-mA*i2FG*mAmCmUi2FCmAmUmUmAmGmAmAi2FGmAi2FAm AmAmGmGmU*mG*mG-3’ (SEQ ID NO: 173);

Modified duplex 71

Sense strand: 5’-mC*mA*mCmCmUmUi2FUmUi2FCi2FUi2FUmCi2FUmAmAmUmGm AmGmUmU-3’ (SEQ ID NO: 174)
Anti-sense strand: 5’-mA*i2FA*mCi2FUmCi2FAmUmUmAmGmAmAmGi2FAmAi2FA mAmGmGmUmG*mG*mG-3’ (SEQ ID NO: 175);

Modified duplex 72

Sense strand: 5’-mC*mA*mCmCmUmUi2FUmUi2FCi2FUi2FUmCi2FUmAmAmUmGm AmGmUmU-3’ (SEQ ID NO: 174)
Anti-sense strand: 5’-mA*i2FA*mCmUmCi2FAmUi2FUmAmGmAmAmGi2FAmAi2FA mAmGmGmUmG*mG*mG-3’ (SEQ ID NO: 176);

Modified duplex 73

Sense strand: 5’-mC*mA*mCmCmUmUi2FUmUi2FCi2FUi2FUmCi2FUmAmAmUmGm AmGmUmU-3’ (SEQ ID NO: 174)
Anti-sense strand: 5’-mA*i2FA*mCmUmCi2FAmUmUmAi2FGmAmAmGi2FAmAi2FA mAmGmGmUmG*mG*mG-3’ (SEQ ID NO: 177);

Modified duplex 74

Sense strand: 5’-mC*mA*mCmCmUmUi2FUmUi2FCi2FUi2FUmCi2FUmAmAmUmGm AmGmUmU-3’ (SEQ ID NO: 174)

Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Modified duplex 75

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Modified duplex 76

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 77

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Modified duplex 78

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Modified duplex 79

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Modified duplex 80

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Modified duplex 81

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Modified duplex 82

Sense strand: 5'-mC*mA*mCmCmUmUi2FUmUi2FCi2FUi2FUmCmUmAi2FAmUmGm AmGmUmU-3' (SEQ ID NO: 181)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 83

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Modified duplex 84

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Modified duplex 85

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Modified duplex 86

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Modified duplex 87

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Modified duplex 88

Sense strand: 5'-mC*mA*mCmCmUmUi2FUmUi2FCi2FUi2FUmCmUmAmAmUi2FGm AmGmUmU-3' (SEQ ID NO: 182)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 89

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Modified duplex 90

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Modified duplex 91

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Modified duplex 92

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Modified duplex 93

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Modified duplex 94

Sense strand: 5'-mC*mA*mCmCmUmUi2FUmUi2FCi2FUi2FUmCmUmAmAmUmGmA i2FGmUmU-3' (SEQ ID NO: 183)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 95

Sense strand: 5'-mC*mA*mCmCmUmUi2FUmUi2FCi2FUi2FUmCmUmAmAmUmGmA mGmUi2FU-3' (SEQ ID NO: 184)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Modified duplex 96

Sense strand: 5'-mC*mA*mCmCmUmUi2FUmUi2FCi2FUi2FUmCmUmAmAmUmGmA mGmUi2FU-3' (SEQ ID NO: 184)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Modified duplex 97

Sense strand: 5'-mC*mA*mCmCmUmUi2FUmUi2FCi2FUi2FUmCmUmAmAmUmGmA mGmUi2FU-3' (SEQ ID NO: 184)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Modified duplex 98

Sense strand: 5'-mC*mA*mCmCmUmUi2FUmUi2FCi2FUi2FUmCmUmAmAmUmGmA mGmUi2FU-3' (SEQ ID NO: 184)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Modified duplex 99

Sense strand: 5'-mC*mA*mCmCmUmUi2FUmUi2FCi2FUi2FUmCmUmAmAmUmGmA mGmUi2FU-3' (SEQ ID NO: 184)

Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Modified duplex 100

Sense strand: 5'-mC*mA*mCmCmUmUi2FUmUi2FCi2FUi2FUmCmUmAmAmUmGmA mGmUi2FU-3' (SEQ ID NO: 184)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 101

Sense strand: 5'-mG*mA*mCmCmUmUi2FUmUi2FCi2FUi2FUmCi2FUmAmAmUmGm AmGmUmU-3' (SEQ ID NO: 185)
Anti-sense strand: 5'-mA*i2FA*mCmUmCi2FAmUmUmAmGmAmAmGi2FAmAi2FAm AmGmGmUm-C*i2FG*mG-3' (SEQ ID NO: 186);

Modified duplex 102

Sense strand: 5'-mC*mU*mCmCmUmUi2FUmUi2FCi2FUi2FUmCi2FUmAmAmUmGm AmGmUmU-3' (SEQ ID NO: 187)
Anti-sense strand: 5'-mA*i2FA*mCmUmCi2FAmUmUmAmGmAmAmGi2FAmAi2FAm AmGmGmAm-G*i2FG*mG-3' (SEQ ID NO: 188);

Modified duplex 103

Sense strand: 5'-mC*mA*mGmCmUmUi2FUmUi2FCi2FUi2FUmCi2FUmAmAmUmGm AmGmUmU-3' (SEQ ID NO: 189)
Anti-sense strand: 5'-mA*i2FA*mCmUmCi2FAmUmUmAmGmAmAmGi2FAmAi2FAm AmGmCmUm-G*i2FG*mG-3' (SEQ ID NO: 190);

Modified duplex 104

Sense strand: 5'-mC*mA*mCmGmUmUi2FUmUi2FCi2FUi2FUmCi2FUmAmAmUmGm AmGmUmU-3' (SEQ ID NO: 191)
Anti-sense strand: 5'-mA*i2FA*mCmUmCi2FAmUmUmAmGmAmAmGi2FAmAi2FAm AmCmGmUm-G*i2FG*mG-3' (SEQ ID NO: 192);

Modified duplex 105

Sense strand: 5'-mC*mA*mCmCmAmUi2FUmUi2FCi2FUi2FUmCi2FUmAmAmUmGm AmGmUmU-3' (SEQ ID NO: 193)
Anti-sense strand: 5'-mA*i2FA*mCmUmCi2FAmUmUmAmGmAmAmGi2FAmAi2FAm UmGmGmUm-G*i2FG*mG-3' (SEQ ID NO: 194);

Modified duplex 106

Sense strand: 5'-mC*mA*mCmCmUmAi2FUmUi2FCi2FUi2FUmCi2FUmAmAmUmGm AmGmUmU-3' (SEQ ID NO: 195)
Anti-sense strand: 5'-mA*i2FA*mCmUmCi2FAmUmUmAmGmAmAmGi2FAmAi2FUm AmGmGmUm-G*i2FG*mG-3' (SEQ ID NO: 196);

Modified duplex 107

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUmUmAmGmAmAmGi2FAmAi2FAm AmGmGi2FUm-G*i2FG*mG-3' (SEQ ID NO: 197);

Modified duplex 108

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUi2FUmAmGmAmAmGi2FAmAi2FA mAmGmGmUm-G*i2FG*mG-3' (SEQ ID NO: 198);

Modified duplex 109

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCi2FUmCi2FAmUmUmAmGmAmAmGi2FAmAi2FA mAmGmGmUm-G*i2FG*mG-3' (SEQ ID NO: 199);

Modified duplex 110

Sense strand: 5'-mC*mA*mCmCmUmUi2FUmUi2FCi2FUi2FUmCi2FUmAmAmUi2FG mAmGmUmU-3' (SEQ ID NO: 200)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 111

Sense strand: 5'-mC*mA*mCmCmUmUi2FUmUi2FCi2FUi2FUmCi2FUmAmAmUmGm AmGmUi2FU-3' (SEQ ID NO: 201)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 112

Sense strand: 5'-mC*mA*mCmCmUmUi2FUmUi2FCmUi2FUmCmUmAmAmUmGmA mGmUmU-3' (SEQ ID NO: 202)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Modified duplex 113

Sense strand: 5'-mC*mA*mCmCmUmUmUmUi2FCmUi2FUmCi2FUmAmAmUmGmA mGmUmU-3' (SEQ ID NO: 204)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Modified duplex 114

Sense strand: 5'-mC*mA*mCmCmUmUmUmUi2FCmUi2FUmCmUmAmAmUi2FGmA mGmUmU-3' (SEQ ID NO: 205)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Modified duplex 115

Sense strand: 5'-mC*mA*mCmCmUmUmUmUi2FCmUi2FUmCmUmAmAmUi2FGmA mGmUmU-3' (SEQ ID NO: 205)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 116

Sense strand: 5'-mC*mA*mCmCmUmUmUmUi2FCi2FUi2FUmCmUmAmAmUmGmA mGmUmU-3' (SEQ ID NO: 206)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Modified duplex 117

Sense strand: 5'-mG*mA*mCmCmUmUi2FUmUi2FCi2FUi2FUmCi2FUmAmAmUi2FG mAmGmUmU-3' (SEQ ID NO: 207)
Anti-sense strand: 5'-mA*i2FA*mCmUmCi2FAmUmUmAmGmAmAmGi2FAmAi2FAm AmGmGmUm-C*i2FG*mG-3' (SEQ ID NO: 186);

Modified duplex 118

Sense strand: 5'-mC*mU*mCmCmUmUi2FUmUi2FCi2FUi2FUmCi2FUmAmAmUi2FG mAmGmUmU-3' (SEQ ID NO: 208)
Anti-sense strand: 5'-mA*i2FA*mCmUmCi2FAmUmUmAmGmAmAmGi2FAmAi2FAm AmGmGmAm-G*i2FG*mG-3' (SEQ ID NO: 188);

Modified duplex 119

Sense strand: 5'-mC*mA*mGmCmUmUi2FUmUi2FCi2FUi2FUmCi2FUmAmAmUi2FG mAmGmUmU-3' (SEQ ID NO: 209)
Anti-sense strand: 5'-mA*i2FA*mCmUmCi2FAmUmUmAmGmAmAmGi2FAmAi2FAm AmGmCmUm-G*i2FG*mG-3' (SEQ ID NO: 190);

Modified duplex 120

Sense strand: 5'-mG*mU*mCmCmUmUi2FUmUi2FCi2FUi2FUmCi2FUmAmAmUi2FG mAmGmUmU-3' (SEQ ID NO: 210)
Anti-sense strand: 5'-mA*i2FA*mCmUmCi2FAmUmUmAmGmAmAmGi2FAmAi2FAm AmGmGmAm-C*i2FG*mG-3' (SEQ ID NO: 211);

Modified duplex 121

Sense strand: 5'-mG*mA*mGmCmUmUi2FUmUi2FCi2FUi2FUmCi2FUmAmAmUi2FG mAmGmUmU-3' (SEQ ID NO: 212)
Anti-sense strand: 5'-mA*i2FA*mCmUmCi2FAmUmUmAmGmAmAmGi2FAmAi2FAm AmGmCmUm-C*i2FG*mG-3' (SEQ ID NO: 213);

Modified duplex 122

Sense strand: 5'-mC*mU*mGmCmUmUi2FUmUi2FCi2FUi2FUmCi2FUmAmAmUi2FG mAmGmUmU-3' (SEQ ID NO: 214)
Anti-sense strand: 5'-mA*i2FA*mCmUmCi2FAmUmUmAmGmAmAmGi2FAmAi2FAm AmGmCmAm-G*i2FG*mG-3' (SEQ ID NO: 215);

Modified duplex 123

Sense strand: 5'-mG*mU*mGmCmUmUi2FUmUi2FCi2FUi2FUmCi2FUmAmAmUi2FG mAmGmUmU-3' (SEQ ID NO: 216)
Anti-sense strand: 5'-mA*i2FA*mCmUmCi2FAmUmUmAmGmAmAmGi2FAmAi2FAm AmGmCmAm-C*i2FG*mG-3' (SEQ ID NO: 217);

Modified duplex 124

Sense strand: 5'-mG*mU*mCmCmUmUi2FUmUi2FCi2FUi2FUmCi2FUmAmAmUmGm AmGmUmU-3' (SEQ ID NO: 218)

Anti-sense strand: 5'-mA*i2FA*mCmUmCi2FAmUmUmAmGmAmAmGi2FAmAi2FAm AmGmGmAm-C*i2FG*mG-3' (SEQ ID NO: 211);

Modified duplex 125

Sense strand: 5'-mG*mU*mGmCmUmUi2FUmUi2FCi2FUi2FUmCi2FUmAmAmUmGm AmGmUmU-3' (SEQ ID NO: 219)
Anti-sense strand: 5'-mA*i2FA*mCmUmCi2FAmUmUmAmGmAmAmGi2FAmAi2FAm AmGmCmAm-C*i2FG*mG-3' (SEQ ID NO: 217);

Modified duplex 129

Sense strand: 5'-mU*mC*mCmCmAmCi2FCmUi2FUi2FUi2FUmCi2FUmUmCmUi2FA mAmUmGmA-3' (SEQ ID NO: 220)
Anti-sense strand: 5'-mU*i2FC*mAmUmUi2FAmGmAmAmGmAmAmAi2FAmGi2FGm UmGmGmGmA*i2FG*-mA-3' (SEQ ID NO: 221);

Modified duplex 130

Sense strand: 5'-mA*mC*mCmCmAmCi2FCmUi2FUi2FUi2FUmCi2FUmUmCmUi2FA mAmUmGmA-3' (SEQ ID NO: 222)
Anti-sense strand: 5'-mU*i2FC*mAmUmUi2FAmGmAmAmGmAmAmAi2FAmGi2FGm UmGmGmGmU*i2FG*-mA-3' (SEQ ID NO: 223);

Modified duplex 133

Sense strand: 5'-mA*mG*mGmCmAmCi2FCmUi2FUi2FUi2FUmCi2FUmUmCmUi2FA mAmUmGmA-3' (SEQ ID NO: 224)
Anti-sense strand: 5'-mU*i2FC*mAmUmUi2FAmGmAmAmGmAmAmAi2FAmGi2FGm UmGmCmCmU*i2FG*-mA-3' (SEQ ID NO: 225);

Modified duplex 134

Sense strand: 5'-mA*mC*mCmGmAmCi2FCmAi2FGi2FCi2FUmUi2FGmUmUmUi2FG mUmGmAmA-3' (SEQ ID NO: 226)
Anti-sense strand: 5'-mU*i2FU*mCmAmCi2FAmAmAmCmAmAmGmCi2FUmGi2FGm UmCmGmGmU*i2-FU*mG-3' (SEQ ID NO: 227);

Modified duplex 135

Sense strand: 5'-mU*mC*mCmGmAmCi2FCmAi2FGi2FCi2FUmUi2FGmUmUmUi2FG mUmGmAmA-3' (SEQ ID NO: 228)
Anti-sense strand: 5'-mU*i2FU*mCmAmCi2FAmAmAmCmAmAmGmCi2FUmGi2FGm UmCmGmGmA*i2-FU*mG-3' (SEQ ID NO: 229);

Modified duplex 136

Sense strand: 5'-mU*mG*mGmGmAmCi2FCmAi2FGi2FCi2FUmUi2FGmUmUmUi2FG mUmGmAmA-3' (SEQ ID NO: 230)
Anti-sense strand: 5'-mU*i2FU*mCmAmCi2FAmAmAmCmAmAmGmCi2FUmGi2FGm UmCmCmCmA*i2-FU*mG-3' (SEQ ID NO: 231);

Modified duplex 137

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*mA*mCi2FUmCi2FAmUmUmAmGmAmAmGi2FAmAi2FAm AmGmGmUm-G*i2FG*mG-3' (SEQ ID NO: 232);

Modified duplex 138

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCi2FUmCi2FAmUmUmAmGmAmAmGi2FAmAmAm AmGmGmUm-G*i2FG*mG-3' (SEQ ID NO: 233);

Modified duplex 139

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*mA*mCmUmCi2FAmUi2FUmAmGmAmAmGi2FAmAi2FAm AmGmGmUm-G*i2FG*mG-3' (SEQ ID NO: 234);

Modified duplex 140

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUi2FUmAmGmAmAmGi2FAmAmAm AmGmGmUm-G*i2FG*mG-3' (SEQ ID NO: 235);

Modified duplex 141

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*mA*mCmUmCi2FAmUmUmAmGmAmAmGi2FAmAi2FAmA i2FGmGmUm-G*i2FG*mG-3' (SEQ ID NO: 236);

Modified duplex 142

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUmUmAmGmAmAmGi2FAmAmAmA i2FGmGmUm-G*i2FG*mG-3' (SEQ ID NO: 237);

Modified duplex 143

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCmAmUi2FUmAmGmAmAmGi2FAmAi2FAm AmGmGmUm-G*i2FG*mG-3' (SEQ ID NO: 238);

Modified duplex 144

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCi2FUmCmAmUmUmAmGmAmAmGi2FAmAi2FAm AmGmGmUm-G*i2FG*mG-3' (SEQ ID NO: 239);

Modified duplex 145

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*mA*mCmUmCi2FAmUmUi2FAmGmAmAmGi2FAmAi2FAm AmGmGmUm-G*i2FG*mG-3' (SEQ ID NO: 240);

Modified duplex 146

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCmAmUmUi2FAmGmAmAmGi2FAmAi2FAm AmGmGmUm-G*i2FG*mG-3' (SEQ ID NO: 241);

Modified duplex 147

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUmUi2FAmGmAmAmGi2FAmAmAm AmGmGmUm-G*i2FG*mG-3' (SEQ ID NO: 242);

Modified duplex 148

Sense strand: 5'-mC*mC*mUmUi2FUmUi2FCi2FUi2FUmCi2FUmAmAmUmGmAmGm UmU-3' (SEQ ID NO: 243)
Anti-sense strand: 5'-mA*i2FA*mCmUmCi2FAmUmUmAmGmAmAmGi2FAmAi2FAm AmGmG*mU*mG-3' (SEQ ID NO: 244);

Modified duplex 149

Sense strand: 5'-mC*mC*mUmUi2FUmUi2FCi2FUi2FUmCi2FUmAmAmUmGmAmGm UmU-3' (SEQ ID NO: 243)
Anti-sense strand: 5'-mA*i2FA*mCmUmCi2FAmUmUmAmGmAmAmGi2FAmAi2FAm AmGmG*i2FU*mG-3' (SEQ ID NO: 245);

Modified duplex 152

Sense strand: 5'-mC*mA*mCmCmUmUi2FUmUi2FCi2FUi2FUmCmUmAmAmUmGmA mGmUmU-3' (SEQ ID NO: 170)
Anti-sense strand: 5'-mA*i2FA*mCmUmCi2FAmUi2FUi2FAmGmAmAmGi2FAmAi2F AmAmGmGmUmG*mG*mG-3' (SEQ ID NO: 246);

Modified duplex 153

Sense strand: 5'-mC*mA*mCmCmUmUi2FUmUi2FCi2FUi2FUmCi2FUmAmAmUmGm AmGmUmU-3' (SEQ ID NO: 174)
Anti-sense strand: 5'-mA*i2FA*mCmUmCi2FAmUi2FUi2FAmGmAmAmGi2FAmAi2F AmAmGmGmUm-G*i2FG*mG-3' (SEQ ID NO: 247);

Modified duplex 154

Sense strand: 5'-mC*mA*mCmCmUmUi2FUmUi2FCi2FUi2FUmCi2FUmAmAmUi2FG mAmGmUmU-3' (SEQ ID NO: 200)
Anti-sense strand: 5'-mA*i2FA*mCmUmCi2FAmUi2FUi2FAmGmAmAmGi2FAmAi2F AmAmGmGmUm-G*i2FG*mG-3' (SEQ ID NO: 247);

Modified duplex 155

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCmUmCi2FAmUmUmAmGmAmAmGi2FAmA i2FAmAmGmGmUm-G*i2FG*mG-3' (SEQ ID NO: 249);

Modified duplex 156

Sense strand: 5'-mC*mU*mCmCmUmUi2FUmUi2FCi2FUi2FUmCi2FUmAmAmUmGm AmGmUmA-3' (SEQ ID NO: 250)

Anti-sense strand: 5'-(E)-VPmU*i2FA*mCmUmCi2FAmUmUmAmGmAmAmGi2FAmA i2FAmAmGmGmAm-G*i2FG*mG-3' (SEQ ID NO: 251);

Modified duplex 157

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCmUmCi2FAmUmUmAi2FGmAmAmGi2FAm Ai2FAmAmGmGmUm-G*i2FG*mG-3' (SEQ ID NO: 252);

Modified duplex 158

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCmUmCi2FAmUi2FUmAmGmAmAmGi2FAm Ai2FAmAmGmGmUm-G*i2FG*mG-3' (SEQ ID NO: 253);

Modified duplex 159

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCi2FUmCi2FAmUmUmAmGmAmAmGi2FAm Ai2FAmAmGmGmUm-G*i2FG*mG-3' (SEQ ID NO: 254);

Modified duplex 160

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*i2FCmUmCi2FAmUmUmAi2FGmAmAmGi2FA mAi2FAmAmGmGmUmG*i2FG*mG-3' (SEQ ID NO: 255);

Modified duplex 161

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(*E*)-VPmU*i2FA*mCmUi2FCi2FAmUmUmAmGmAmAmGi2FAm Ai2FAmAmGmGmUm-G*i2FG*mG-3' (SEQ ID NO: 256);

Modified duplex 162

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(*E*)-VPmU*i2FA*mCmUmCi2FAi2FUmUmAmGmAmAmGi2FAm Ai2FAmAmGmGmUm-G*i2FG*mG-3' (SEQ ID NO: 257);

Modified duplex 163

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCmUmCi2FAmUi2FUi2FAmGmAmAmGi2FA mAi2FAmAmGmGmUm-G*i2FG*mG-3' (SEQ ID NO: 258);

Modified duplex 164

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(*E*)-VPmU*i2FA*mCmUmCi2FAmUmUi2FAi2FGmAmAmGi2FA mAi2FAmAmGmGmUm-G*i2FG*mG-3' (SEQ ID NO: 259);

Modified duplex 165

Sense strand: 5'-mC*mA*mCmCmUmUi2FUmUi2FCi2FUi2FUmCmUmAmAmUi2FGm AmGmUmA-3' (SEQ ID NO: 260)
Anti-sense strand: 5'-(*E*)-VPmU*i2FA*mCmUmCi2FAmUmUi2FAi2FGmAmAmGi2FA mAi2FAmAmGmGmUm-G*i2FG*mG-3' (SEQ ID NO: 259);

Modified duplex 166

Sense strand: 5'-mC*mG*mCmCmUmUi2FUmUi2FCi2FUi2FUmCi2FUmAmAmUmGm AmGmUmA-3' (SEQ ID NO: 261)
Anti-sense strand: 5'-(E)-VPmU*i2FA*mCmUmCi2FAmUmUmAmGmAmAmGi2FAmA i2FAmAmGmGmCm-G*i2FG*mG-3' (SEQ ID NO: 262);

Modified duplex 167

Sense strand: 5'-mC*mA*mCmCmUmUi2FUmUi2FCi2FUi2FUmCi2FUmAmAmUmGm AmGTmA-3' (SEQ ID NO: 263)
Anti-sense strand: 5'-(*E*)-VPmU*i2FA*mCmUmCi2FAmUmUmAmGmAmAmGi2FAmA i2FAmAmGmGmUm-G*i2FG*mG-3' (SEQ ID NO: 249);

Modified duplex 168

Sense strand: 5'-mC*mU*mCmCmCmAmCmCmUmUi2FUmUi2FCi2FUi2FUmCi2FUm AmAmUmGmAmGmUmA-3' (SEQ ID NO: 264)
Anti-sense strand: 5'-(E)-VPmU*i2FA*mCmUmCi2FAmUmUmAmGmAmAmGi2FAmA i2FAmAmGmGmUmGmGmGmAmG*i2FA*mC-3' (SEQ ID NO: 265);

Modified duplex 169

Sense strand: 5'-mU*mC*mCmCmAmCmCmUmUi2FUmUi2FCi2FUi2FUmCi2FUmAm AmUmGmAmGmUmA-3' (SEQ ID NO: 266)
Anti-sense strand: 5'-(E)-VPmU*i2FA*mCmUmCi2FAmUmUmAmGmAmAmGi2FAmA i2FAmAmGmGmUmGmGmGmA*i2FG*mA-3' (SEQ ID NO: 267);

Modified duplex 170

Sense strand: 5'-mC*mC*mCmAmCmCmUmUi2FUmUi2FCi2FUi2FUmCi2FUmAmAm UmGmAmGmUmA-3' (SEQ ID NO: 268)
Anti-sense strand: 5'-(E)-VPmU*i2FA*mCmUmCi2FAmUmUmAmGmAmAmGi2FAmA i2FAmAmGmGmUmGmGmG*i2FA*mG-3' (SEQ ID NO: 269);

Modified duplex 171

Sense strand: 5'-mC*mC*mAmCmCmUmUi2FUmUi2FCi2FUi2FUmCi2FUmAmAmUm GmAmGmUmA-3' (SEQ ID NO: 270)
Anti-sense strand: 5'-(E)-VPmU*i2FA*mCmUmCi2FAmUmUmAmGmAmAmGi2FAmA i2FAmAmGmGmUmGmG*i2FG*mA-3' (SEQ ID NO: 271);

Modified duplex 172

Sense strand: 5'-mA*mC*mCmUmUi2FUmUi2FCi2FUi2FUmCi2FUmAmAmUmGmAm GmUmA-3' (SEQ ID NO: 272)
Anti-sense strand: 5'-(E)-VPmU*i2FA*mCmUmCi2FAmUmUmAmGmAmAmGi2FAmA i2FAmAmGmGmU*i2FG*mG-3' (SEQ ID NO: 273);

Modified duplex 173

Sense strand: 5'-mC*mC*mUmUi2FUmUi2FCi2FUi2FUmCi2FUmAmAmUmGmAmGm UmA-3'(SEQ ID NO: 274)
Anti-sense strand: 5'-(E)-VPmU*i2FA*mCmUmCi2FAmUmUmAmGmAmAmGi2FAmA i2FAmAmGmG*i2-FU*mG-3' (SEQ ID NO: 275);

Modified duplex 174

Sense strand: 5'-mU*i2FU*mUi2FCi2FUi2FUmCi2FUmAmAmUmGmAmGmUmA-3' (S EQ ID NO: 276)
Anti-sense strand: 5'-(E)-VPmU*i2FA*mCmUmCi2FAmUmUmAmGmAmAmGi2FAmA i2FAmAmGmG*i2-FU*mG-3' (SEQ ID NO: 275);

Modified duplex 175

Sense strand: 5'-mU*mU*i2FUmUi2FCi2FUi2FUmCi2FUmAmAmUmGmAmGmUmA-3 '(SEQ ID NO: 277)
Anti-sense strand: 5'-(E)-VPmU*i2FA*mCmUmCi2FAmUmUmAmGmAmAmGi2FAmA i2FAmAmGmG*i2-FU*mG-3' (SEQ ID NO: 275);

Modified duplex 176

Sense strand: 5'-mU*mU*i2FUmUi2FCi2FUi2FUmCi2FUmAmAmUmGmAmGmUmA-3 '(SEQ ID NO: 277)
Anti-sense strand: 5'-(E)-VPmU*i2FA*mCmUmCi2FAmUmUmAmGmAmAmGi2FAmA i2FAmAmGmGmUm-G*i2FG*mG-3' (SEQ ID NO: 249);

Modified duplex 177

Sense strand: 5'-mC*mU*mUi2FUmUi2FCi2FUi2FUmCi2FUmAmAmUmGmAmGmUm A-3' (SEQ ID NO: 278)
Anti-sense strand: 5'-(*E*)-VPmU*i2FA*mCmUmCi2FAmUmUmAmGmAmAmGi2FAmA i2FAmAmGmGmUm-G*i2FG*mG-3' (SEQ ID NO: 249);

Modified duplex 178

Sense strand: 5'-mC*mC*mUmUi2FUmUi2FCi2FUi2FUmCi2FUmAmAmUmGmAmGm UmA-3'(SEQ ID NO: 274)
Anti-sense strand: 5'-(E)-VPmU*i2FA*mCmUmCi2FAmUmUmAmGmAmAmGi2FAmA i2FAmAmGmGmUm-G*i2FG*mG-3' (SEQ ID NO: 249);

Modified duplex 179

Sense strand: 5'-mU*i2FU*mUi2FCi2FUi2FUmCi2FUmAmAmUmGmAmGmUmA-3' (S EQ ID NO: 276)
Anti-sense strand: 5'-(E)-VPmU*i2FA*mCmUmCi2FAmUmUmAmGmAmAmGi2FAmA i2FAmA*i2FG*mG-3' (SEQ ID NO: 279);

Modified duplex 180

Sense strand: 5'-mU*mU*i2FUmUi2FCi2FUi2FUmCi2FUmAmAmUmGmAmGmUmA-3 '(SEQ ID NO: 277)
Anti-sense strand: 5'-(*E*)-VPmU*i2FA*mCmUmCi2FAmUmUmAmGmAmAmGi2FAmA i2FAmA*i2FG*mG-3' (SEQ ID NO: 279);

Modified duplex 181

Sense strand: 5'-mC*mA*mCmCmUmUi2FUmUi2FCi2FUi2FUmCi2FUmAmAmUmGm AmGmUmA-3' (SEQ ID NO: 248)
Anti-sense strand: 5'-(E)-VPmU*i2FA*mCmUmCi2FAi2FUmUmAi2FGmAmAmGi2FA mAi2FAmAmGmGmUmG*i2FG*mG-3' (SEQ ID NO: 280);

Modified duplex 182

Sense strand: 5'-mC*mU*mCmCmUmUi2FUmUi2FCi2FUi2FUmCi2FUmAmAmUmGm AmGmUmA-3' (SEQ

ID NO: 250)
Anti-sense strand: 5'-(E)-VPmU*i2FA*mCmUmCi2FAi2FUmUmAmGmAmAmGi2FAm Ai2FAmAmGmGmAm-G*i2FG*mG-3' (SEQ ID NO: 281);

Modified duplex 183

Sense strand: 5'-mC*mG*mCmCmUmUi2FUmUi2FCi2FUi2FUmCi2FUmAmAmUmGm AmGmUmA-3' (SEQ ID NO: 261)
Anti-sense strand: 5'-(E)-VPmU*i2FA*mCmUmCi2FAi2FUmUmAmGmAmAmGi2FAm Ai2FAmAmGmGmCm-G*i2FG*mG-3' (SEQ ID NO: 282);

Modified duplex 184

Sense strand: 5'-mC*mU*mCmCmUmUi2FUmUi2FCi2FUi2FUmCi2FUmAmAmUmGm AmGmUmA-3' (SEQ ID NO: 250)
Anti-sense strand: 5'-(*E*)-VPmU*i2FA*mCmUmCi2FAi2FUmUmAi2FGmAmAmGi2FA mAi2FAmAmGmGmAm-G*i2FG*mG-3' (SEQ ID NO: 283);

Modified duplex 185

Sense strand: 5'-mC*mG*mCmCmUmUi2FUmUi2FCi2FUi2FUmCi2FUmAmAmUmGm AmGmUmA-3' (SEQ ID NO: 261)
Anti-sense strand: 5'-(E)-VPmU*i2FA*mCmUmCi2FAi2FUmUmAi2FGmAmAmGi2FA mAi2FAmAmGmGmCmG*i2FG*mG-3' (SEQ ID NO: 284);

Modified duplex 186

Sense strand: 5'-mU*mU*i2FUmUi2FCi2FUi2FUmCi2FUmAmAmUmGmAmGmUmA-3 '(SEQ ID NO: 277)
Anti-sense strand: 5'-(*E*)-VPmU*i2FA*mCmUmCi2FAmUmUmAi2FGmAmAmGi2FAm Ai2FAmAmGmG*i2-FU*mG-3' (SEQ ID NO: 285);

Modified duplex 187

Sense strand: 5'-mU*mU*i2FUmUi2FCi2FUi2FUmCi2FUmAmAmUmGmAmGmUmA-3 '(SEQ ID NO: 277)
Anti-sense strand: 5'-(E)-VPmU*i2FA*mCmUmCi2FAi2FUmUmAmGmAmAmGi2FAm Ai2FAmAmGmG*i2-FU*mG-3' (SEQ ID NO: 286);

Modified duplex 188

Sense strand: 5'-mU*mU*i2FUmUi2FCi2FUi2FUmCi2FUmAmAmUmGmAmGmUmA-3 '(SEQ ID NO: 277)
Anti-sense strand: 5'-(*E*)-VPmU*i2FA*mCmUmCi2FAi2FUmUmAi2FGmAmAmGi2FA mAi2FAmAmGmG*i2-FU*mG-3' (SEQ ID NO: 287);

Modified duplex 189

Sense strand: 5'-mA*mC*mCmUmUi2FUmUi2FCi2FUi2FUmCi2FUmAmAmUmGmAm GmUmA-3' (SEQ ID NO: 272)
Anti-sense strand: 5'-(*E*)-VPmU*i2FA*mCmUmCi2FAi2FUmUmAmGmAmAmGi2FAm Ai2FAmAmGmGmU*i2FG*mG-3' (SEQ ID NO: 288);

Modified duplex 190

Sense strand: 5'-mU*mC*mCmUmUi2FUmUi2FCi2FUi2FUmCi2FUmAmAmUmGmAm GmUmA-3' (SEQ ID NO: 289)
Anti-sense strand: 5'-(*E*)-VPmU*i2FA*mCmUmCi2FAi2FUmUmAmGmAmAmGi2FAm Ai2FAmAmGmGmA*i2FG*mG-3' (SEQ ID NO: 290);

Modified duplex 191

Sense strand: 5'-mA*mG*mCmUmUi2FUmUi2FCi2FUi2FUmCi2FUmAmAmUmGmAm GmUmA-3' (SEQ ID NO: 291)
Anti-sense strand: 5'-(*E*)-VPmU*i2FA*mCmUmCi2FAi2FUmUmAmGmAmAmGi2FAm Ai2FAmAmGmCmU*i2FG*mG-3' (SEQ ID NO: 292);

Modified duplex 192

Sense strand: 5'-mA*mC*mGmUmUi2FUmUi2FCi2FUi2FUmCi2FUmAmAmUmGmAm GmUmA-3' (SEQ ID NO: 293)
Anti-sense strand: 5'-(E)-VPmU*i2FA*mCmUmCi2FAi2FUmUmAmGmAmAmGi2FAm Ai2FAmAmCmGmU*i2FG*mG-3' (SEQ ID NO: 294);

Modified duplex 193

Sense strand: 5'-mG*mC*mCmUmUi2FUmUi2FCi2FUi2FUmCi2FUmAmAmUmGmAm GmUmA-3' (SEQ ID NO: 295)
Anti-sense strand: 5'-(E)-VPmU*i2FA*mCmUmCi2FAi2FUmUmAmGmAmAmGi2FAm Ai2FAmAmGmGm-C*i2FG*mG-3' (SEQ ID NO: 296);

Modified duplex 194

Sense strand: 5'-mC*mC*mAmCmCmUmUi2FUmUi2FCi2FUi2FUmCi2FUmAmAmUm GmAmGmUmA-3' (SEQ ID NO: 271)
Anti-sense strand: 5'-(*E*)-VPmU*i2FA*mCmUmCi2FAi2FUmUmAmGmAmAmGi2FAm Ai2FAmAmGmGmUmGmG*i2FG*mA-3' (SEQ ID NO: 297);

Modified duplex 195

Sense strand: 5'-mG*mC*mAmCmCmUmUi2FUmUi2FCi2FUi2FUmCi2FUmAmAmUm GmAmGmUmA-3' (SEQ ID NO: 298)
Anti-sense strand: 5'-(E)-VPmU*i2FA*mCmUmCi2FAi2FUmUmAmGmAmAmGi2FAm Ai2FAmAmGmGmUmGmC*i2FG*mA-3' (SEQ ID NO: 299);

Modified duplex 196

Sense strand: 5'-mC*mG*mAmCmCmUmUi2FUmUi2FCi2FUi2FUmCi2FUmAmAmUm GmAmGmUmA-3' (SEQ ID NO: 300)
Anti-sense strand: 5'-(E)-VPmU*i2FA*mCmUmCi2FAi2FUmUmAmGmAmAmGi2FAm Ai2FAmAmGmGmUmCmG*i2FG*mA-3' (SEQ ID NO: 301);

Modified duplex 197

Sense strand: 5'-mC*mC*mUmCmCmUmUi2FUmUi2FCi2FUi2FUmCi2FUmAmAmUm GmAmGmUmA-3' (SEQ ID NO: 302)
Anti-sense strand: 5'-(*E*)-VPmU*i2FA*mCmUmCi2FAi2FUmUmAmGmAmAmGi2FAm Ai2FAmAmGmGmAmGmG*i2FG*mA-3' (SEQ ID NO: 303);

Modified duplex 198

Sense strand: 5'-mC*mC*mGmCmCmUmUi2FUmUi2FCi2FUi2FUmCi2FUmAmAmUm GmAmGmUmA-3' (SEQ ID NO: 304)
Anti-sense strand: 5'-(E)-VPmU*i2FA*mCmUmCi2FAi2FUmUmAmGmAmAmGi2FAm Ai2FAmAmGmGmCmGmG*i2FG*mA-3' (SEQ ID NO: 305);

Modified duplex 199

Sense strand: 5'-mC*mA*mCmCmUmUi2FUmUi2FCi2FUi2FUmCi2FUmAmAmUi2FG mAmGmUmA-3' (SEQ ID NO: 306)

Anti-sense strand: 5'-(E)-VPmU*i2FA*mCmUmCi2FAmUi2FUi2FAmGmAmAmGi2FA mAi2FAmAmGmGmUm-G*i2FG*mG-3' (SEQ ID NO: 258);

Modified duplex 200

Sense strand: 5'-mC*mA*mCmCmUmUi2FUmUi2FCi2FUi2FUmCi2FUmAmAmUi2FG mAmGmUmA-3' (SEQ ID NO: 306)
Anti-sense strand: 5'-(E)-VPmU*i2FA*mCmUmCi2FAmUmUmAmGmAmAmGi2FAmA i2FAmAmGmGmUm-G*i2FG*mG-3' (SEQ ID NO: 249);

Modified duplex 201

Sense strand: 5'-mC*mA*mGmCmUmUi2FUmUi2FCi2FUi2FUmCi2FUmAmAmUi2FG mAmGmUmA-3' (SEQ ID NO: 307)
Anti-sense strand: 5'-(E)-VPmU*i2FA*mCmUmCi2FAmUmUmAmGmAmAmGi2FAmA i2FAmAmGmCmUm-G*i2FG*mG-3' (SEQ ID NO: 308).

In some embodiments of the present disclosure, the siRNA is at least one selected from modified duplex 46, modified duplex 61 to modified duplex 64 and modified duplex 66 to modified duplex 116, modified duplex 117 to modified duplex 125, modified duplex 129 to modified duplex 130, modified duplex 133 to modified duplex 136, and modified duplex 155 to modified duplex 201 (e.g., modified duplex 155 to modified duplex 157, modified duplex 162, modified duplex 166, modified duplex 171 to modified duplex 172, modified duplex 181 to modified duplex 198).

In some embodiments of the present disclosure, the siRNA is at least one selected from modified duplex 156, modified duplex 182, and modified duplex 184.

In some embodiments of the present disclosure, the siRNA comprises a sense strand and an antisense strand, wherein the antisense strand comprises a sequence set forth in any one selected from SEQ ID NOs: 145, 155, 157, 159, 161, 165, 167, 169, 171, 173, 175, 176, 177, 178, 179, 180, 186, 188, 190, 192, 194, 196, 197, 198, 199, 203, 211, 213, 215, 217, 221, 223, 225, 227, 229, 231, 249, 251, 252, 253, 254, 255, 256, 257, 258, 259, 262, 265, 267, 269, 271, 273, 275, 279, 280, 281, 282, 283, 284, 285, 286, 287, 288, 290, 292, 294, 296, 297, 299, 301, 303, 305, and 308 (e.g., a sequence set forth in any one selected from SEQ ID NOs: 145, 155, 157, 159, 161, 165, 167, 169, 171, 173, 175, 176, 177, 178, 179, 180, 186, 188, 190, 192, 194, 196, 197, 198, 199, 203, 211, 213, 215, 217, 221, 223, 225, 227, 229, 231, 249, 251, 252, 257, 262, 271, 273, 280, 281, 282, 283, 284, 285, 286, 287, 288, 290, 292, 294, 296, 297, 299, 301, 303, 305, and 308).

It is known to the skilled in the art that the siRNA described in the present disclosure can be obtained by conventional siRNA preparation methods in the art (e.g., solid-phase synthesis and liquid-phase synthesis), wherein both solid-phase synthesis and liquid-phase synthesis have available commercially customized services. It is also clearly known to the skilled in the art that modified nucleotide groups can be introduced into the siRNA described in the present disclosure by using nucleotide monomers with corresponding modifications. Methods for preparing nucleotide monomers with corresponding modifications are well known to the skilled in the art, and commercial monomers are also available on the market.

2. siRNA conjugates of the present disclosure

The siRNA conjugates of the present disclosure are obtained by conjugating the siRNA of the present disclosure with pharmaceutically acceptable conjugate molecules comprising pharmaceutically acceptable targeting ligands and linkers.

A "linker" means an organic moiety that links two parts of a compound, for example, covalently attaches two parts of a compound. A linker typically comprises a direct bond or an atom such as oxygen or sulfur, a unit such as NR, C(O), C(O)NH, SO, $SO_2$, $SO_2NH$ or a chain of atoms, such as, but not limited to, substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocycloalkyl, hetero-cycloalkenyl, heterocycloalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocycloalkyl, alkylheterocycloalkenyl, alkylheterocycloalkynyl, alkenylheterocycloalkyl, alkenylheterocycloalkenyl, alkenylheterocycloalkynyl, alkynylheterocycloalkyl, alkynylheterocycloalkenyl, alkynylheterocycloalkynyl, alkylaryl, alkenylaryl, alkynylaryl, alkylheteroaryl, alkenylheteroaryl, alkynylheteroaryl, wherein one or more methylene groups may be interrupted or terminated by O, S, S(O), $SO_2$, N(R), C(O), substituted or unsubstituted aryl, substituted or unsubstituted heteroaryl, substituted or unsubstituted heterocyclyl; wherein R is

hydrogen, acyl, aliphatic or substituted aliphatic. In some embodiments of the present disclosure, the linker is about between 1 and 24 atoms, between 2 and 24 atoms, between 3 and 24 atoms, between 4 and 24 atoms, between 5 and 24 atoms, between 6 and 24 atoms, between 6 and 18 atoms, between 7 and 18 atoms, between 8 and 18 atoms, between 7 and 17 atoms, between 8 and 17 atoms, between 6 and 16, between 7 and 16 atoms, or between 8 and 16 atoms.

In some embodiments of the present disclosure, the siRNA is covalently conjugated to the conjugate molecule. To reduce the potential impact of conjugation on siRNA activity, the conjugation site of the siRNA to the conjugate molecule may be at the 3' end or 5' end of the sense strand of the siRNA, or at the 5' end of the antisense strand. In some embodiments, the conjugation site of the siRNA to the conjugate molecule may also be in the internal sequence of the siRNA.

Pharmaceutically acceptable targeting ligands may be targeting ligands conventionally used in the siRNA administration field. In some embodiments of the present disclosure, the targeting ligand may include, but is not limited to, one or more of the following targeting ligands or derivatives thereof: lipophilic molecules, polymers, polypeptides, aptamers, antibodies, chimeric antigen receptors, quantum dots, carbohydrates, folate, or receptor ligands expressed by hepatocytes. In some embodiments of the present disclosure, the lipophilic molecule may include at least one of cholesterol, bile acids, vitamins (e.g., vitamin E), lipid molecules with different chain lengths (such as phospholipids, phospholipid ethers (PLE)). In some embodiments of the present disclosure, the polymer may include polyethylene glycol. In some embodiments of the present disclosure, the polypeptide may include a membrane-permeable peptide. In some embodiments of the present disclosure, the carbohydrate may include at least one of lactose, polylactose, mannose, galactose, N-acetylgalactosamine (GalNAc), CMM (Chemically Modified Mannose). In some embodiments of the present disclosure, the receptor ligand expressed by hepatocytes may include at least one of asialoglycoproteins, desialylated carbohydrate residues, lipoproteins (such as high-density lipoprotein, low-density lipoprotein, etc.), glucagon, neurotransmitters (such as adrenaline), growth factors, transferrin.

In some embodiments of the present disclosure, the targeting ligand is N-acetylgalactosamine or CMM. In some embodiments of the present disclosure, the targeting ligand is N-acetylgalactosamine.

In some embodiments of the present disclosure, the targeting ligand may be directly linked to the 3'-terminus of the sense strand of the siRNA. In some embodiments of the present disclosure, the targeting ligand may be linked to the 3'-terminus of the sense strand of the siRNA via a linker.

In some embodiments of the present disclosure, the linker-targeting ligand moiety has the following structure:

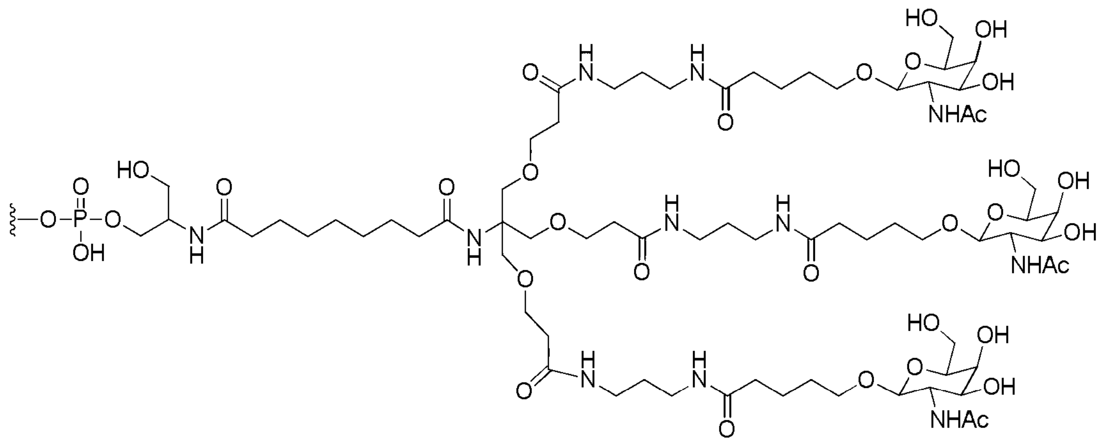

;

or

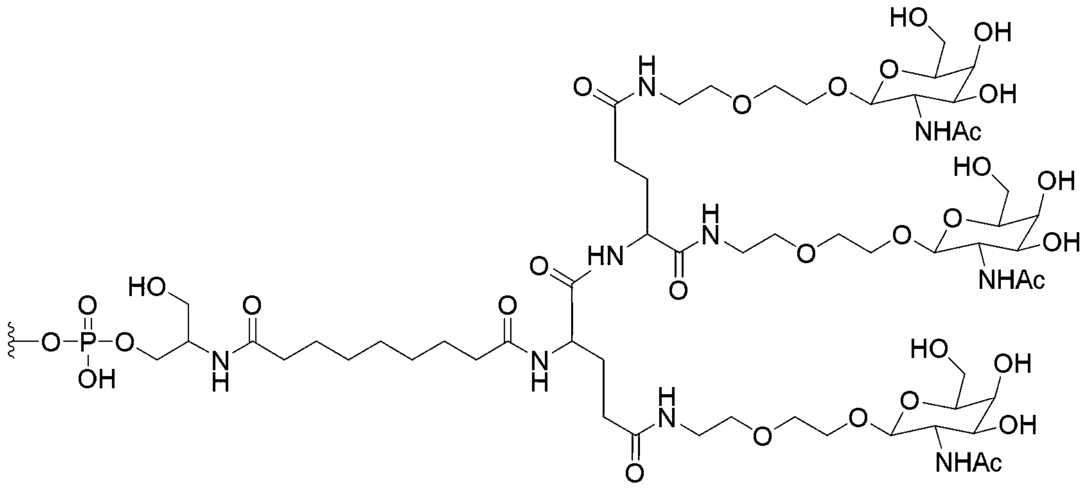

;

or

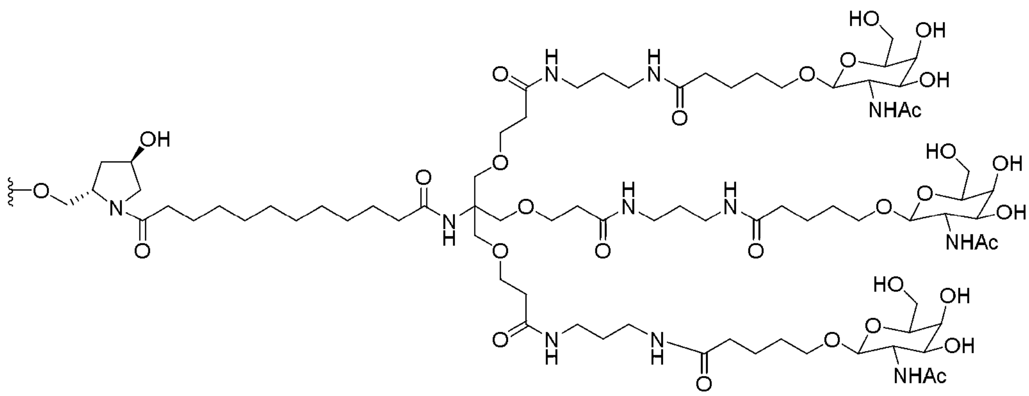

GalNAc(L96); or

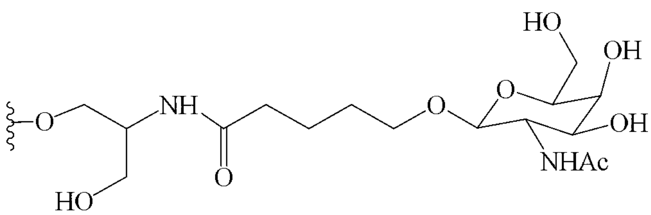

SerGalNAc.

In some embodiments of the present disclosure, the structure of the siRNA conjugate may be as follows:

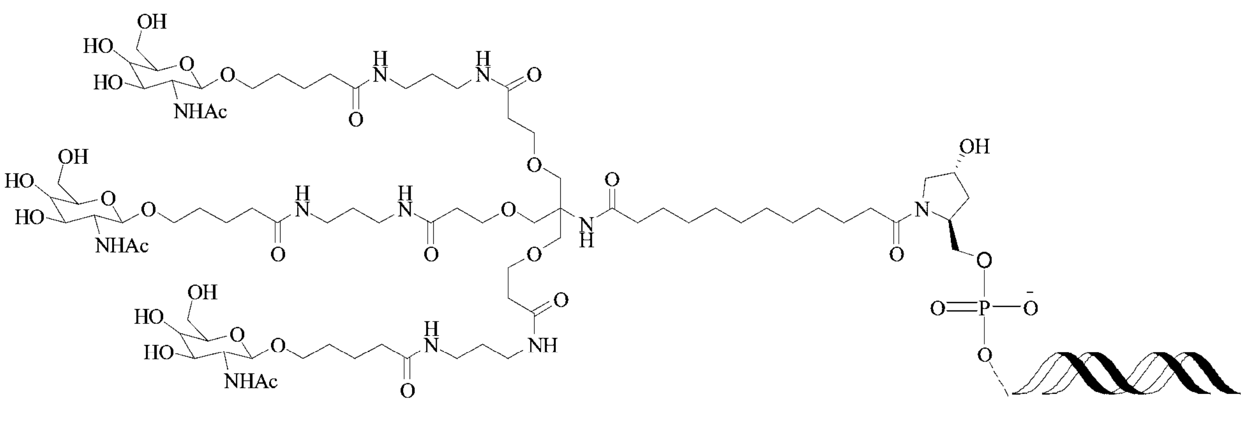

wherein the conjugate molecule described herein is covalently conjugated to the 3' end of the sense strand of the siRNA denoted by

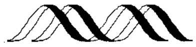

in the manner shown in the above formula.

In some embodiments of the present disclosure, the siRNA conjugate may be selected from at least one of conjugate 46 to conjugate 50, conjugate 60 to conjugate 125, conjugate 129 to conjugate 130, conjugate 133 to conjugate 136, conjugate 150 to conjugate 151, conjugate 155 to conjugate 204, wherein the structure of the conjugate is as follows:

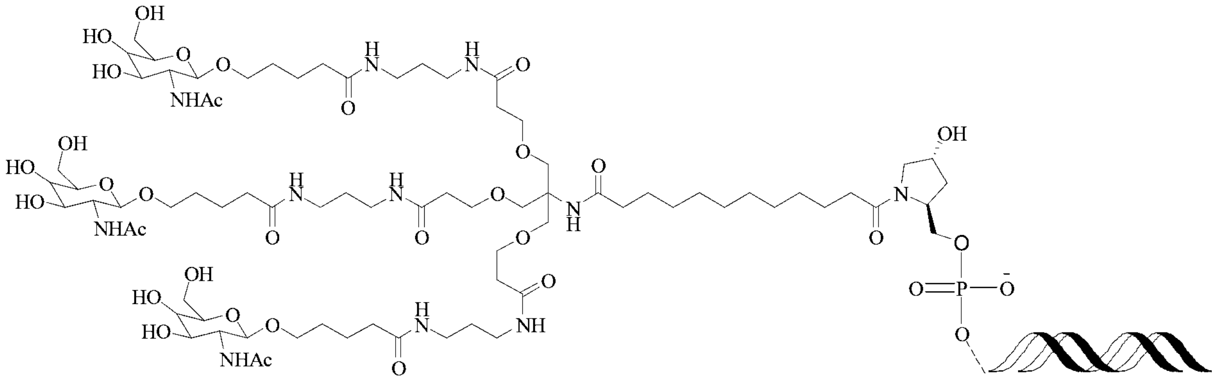

,

wherein

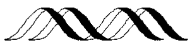

represents siRNA;

Conjugate 46

Sense strand: 5'-mU*mC*mCmCmAmCi2FCmUi2FUi2FUi2FUmCmUmUmCmUmAmA mUmGmAGalNAc(L96)-3' (SEQ ID NO: 309)
Anti-sense strand: 5'-mU*i2FC*mAmUmUi2FAmGmAmAmGmAmAmAi2FAmGi2FGm UmGmGmGmA*mG*-mA-3' (SEQ ID NO: 145);

Conjugate 47

Sense strand: 5'-mU*mC*mCmCmAmCi2FCmUi2FUi2FUi2FUmCi2FUmUmCmUmAm AmUmGmAGalNAc(L96)-3' (SEQ ID NO: 310)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Conjugate 48

Sense strand: 5'-mU*mC*mCmCmAmCi2FCmUi2FUi2FUi2FUmCmUmUi2FCmUmAm AmUmGmAGalNAc(L96)-3' (SEQ ID NO: 311)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Conjugate 49

Sense strand: 5'-mU*mC*mCmCmAmCi2FCmUi2FUi2FUi2FUmCmUmUmCmUi2FAm AmUmGmAGalNAc(L96)-3' (SEQ ID NO: 312)
Anti-sense strand: 5'-mU*i2FC*mAmUmUi2FAmGmAmAmGmAmAmAi2FAmGi2FGm UmGmGi2FGmA*mG*-

mA-3' (SEQ ID NO: 150);

Conjugate 50

Sense strand: 5'-mU*mC*i2FCmCmAmCi2FCmUi2FUi2FUi2FUmCmUmUmCmUmAm AmUmGmAGal-NAc(L96)-3' (SEQ ID NO: 313)
Anti-sense strand: 5'-mU*i2FC*mAmUmUi2FAmGmAmAi2FGmAmAmAi2FAmGi2FG mUmGmGmGmA*mG*-mA-3' (SEQ ID NO: 152);

Conjugate 60

Sense strand: 5'-mU*mC*mCmCmAmCi2FCmUi2FUi2FUi2FUmCmUmUmCi2FUmAm AmUmGmAGal-NAc(L96)-3' (SEQ ID NO: 314)
Anti-sense strand: 5'-mU*i2FC*mAi2FUmUi2FAmGmAmAmGmAmAmAi2FAmGi2FG mUmGmGmGmA*mG*-mA-3' (SEQ ID NO: 147);

Conjugate 61

Sense strand: 5'-mC*mC*mAmGmUmCi2FUmCi2FCi2FCi2FAmCmCmUmUmUmUmC mUmUmUGal-NAc(L96)-3' (SEQ ID NO: 315)
Anti-sense strand: 5'-mA*i2FA*mAmGmAi2FAmAmAmGmGmUmGmGi2FGmAi2FGm AmCmUmGmG*mG*mG-3' (SEQ ID NO: 155);

Conjugate 62

Sense strand: 5'-mC*mA*mGmUmCmUi2FCmCi2FCi2FAi2FCmCmUmUmUmUmCmU mUmCmUGal-NAc(L96)-3' (SEQ ID NO: 316)
Anti-sense strand: 5'-mA*i2FG*mAmAmGi2FAmAmAmAmGmGmUmGi2FGmGi2FAm GmAmCmUmG*mG*mG-3' (SEQ ID NO: 157);

Conjugate 63

Sense strand: 5'-mA*mG*mUmCmUmCi2FCmCi2FAi2FCi2FCmUmUmUmUmCmUmU mCmUmAGal-NAc(L96)-3' (SEQ ID NO: 317)
Anti-sense strand: 5'-mU*i2FA*mGmAmAi2FGmAmAmAmAmGmGmUi2FGmGi2FGm AmGmAmCmU*mG*mG-3' (SEQ ID NO: 159);

Conjugate 64

Sense strand: 5'-mG*mU*mCmUmCmCi2FCmAi2FCi2FCi2FUmUmUmUmCmUmUmC mUmAmAGal-NAc(L96)-3' (SEQ ID NO: 318)
Anti-sense strand: 5'-mU*i2FU*mAmGmAi2FAmGmAmAmAmAmGmGi2FUmGi2FGm GmAmGmAmC*-mU*mG-3' (SEQ ID NO: 161);

Conjugate 65

Sense strand: 5'-mU*mC*mUmCmCmCi2FAmCi2FCi2FUi2FUmUmUmCmUmUmCmU mAmAmUGal-NAc(L96)-3' (SEQ ID NO: 319)
Anti-sense strand: 5'-mA*i2FU*mUmAmGi2FAmAmGmAmAmAmAmGi2FGmUi2FGm GmGmAmGmA*mC*-mU-3' (SEQ ID NO: 163);

Conjugate 66

Sense strand: 5'-mC*mU*mCmCmCmAi2FCmCi2FUi2FUi2FUmUmCmUmUmCmUmA mAmUmUGal-NAc(L96)-3' (SEQ ID NO: 320)
Anti-sense strand: 5'-mA*i2FA*mUmUmAi2FGmAmAmGmAmAmAmAi2FGmGi2FUm GmGmGmAmG*-mA*mC-3' (SEQ ID NO: 165);

Conjugate 67

Sense strand: 5'-mC*mC*mCmAmCmCi2FUmUi2FUi2FUi2FCmUmUmCmUmAmAmU mGmAmUGal-NAc(L96)-3' (SEQ ID NO: 321)
Anti-sense strand: 5'-mA*i2FU*mCmAmUi2FUmAmGmAmAmGmAmAi2FAmAi2FGm GmUmGmGmG*-mA*mG-3' (SEQ ID NO: 167);

Conjugate 68

Sense strand: 5'-mC*mC*mAmCmCmUi2FUmUi2FUi2FCi2FUmUmCmUmAmAmUmG mAmGmUGal-NAc(L96)-3' (SEQ ID NO: 322)
Anti-sense strand: 5'-mA*i2FC*mUmCmAi2FUmUmAmGmAmAmGmAi2FAmAi2FAm GmGmUmGmG*mG*-mA-3' (SEQ ID NO: 169);

Conjugate 69

Sense strand: 5'-mC*mA*mCmCmUmUi2FUmUi2FCi2FUi2FUmCmUmAmAmUmGmA mGmUmUGal-NAc(L96)-3' (SEQ ID NO: 323)
Anti-sense strand: 5'-mA*i2FA*mCmUmCi2FAmUmUmAmGmAmAmGi2FAmAi2FAm AmGmGmUmG*mG*mG-3' (SEQ ID NO: 171);

Conjugate 70

Sense strand: 5'-mA*mC*mCmUmUmUi2FUmCi2FUi2FUi2FCmUmAmAmUmGmAmG mUmCmUGal-NAc(L96)-3' (SEQ ID NO: 324)
Anti-sense strand: 5'-mA*i2FG*mAmCmUi2FCmAmUmUmAmGmAmAi2FGmAi2FAm AmAmGmGmU*mG*mG-3' (SEQ ID NO: 173);

Conjugate 71

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Conjugate 72

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Conjugate 73

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Conjugate 74

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Conjugate 75

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Conjugate 76

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 77

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Conjugate 78

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Conjugate 79

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Conjugate 80

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Conjugate 81

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Conjugate 82

Sense strand: 5'-mC*mA*mCmCmUmUi2FUmUi2FCi2FUi2FUmCmUmAi2FAmUmGm AmGmUmUGal-NAc(L96)-3' (SEQ ID NO: 326)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 83

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)

Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Conjugate 84

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Conjugate 85

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Conjugate 86

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Conjugate 87

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)
Anti-sense strand: 5'-mA*i2FA*mCmUmCi2FAmUmUmAmGmAmAmGi2FAmAi2FAm AmGmGi2-FUmG*mG*mG-3' (SEQ ID NO: 179);

Conjugate 88

Sense strand: 5'-mC*mA*mCmCmUmUi2FUmUi2FCi2FUi2FUmCmUmAmAmUi2FGm AmGmUmUGal-NAc(L96)-3' (SEQ ID NO: 327)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 89

Sense strand: 5'-mC*mA*mCmCmUmUi2FUmUi2FCi2FUi2FUmCmUmAmAmUmGmA i2FGmUmUGal-NAc(L96)-3' (SEQ ID NO: 328)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Conjugate 90

Sense strand: 5'-mC*mA*mCmCmUmUi2FUmUi2FCi2FUi2FUmCmUmAmAmUmGmA i2FGmUmUGal-NAc(L96)-3' (SEQ ID NO: 328)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Conjugate 91

Sense strand: 5'-mC*mA*mCmCmUmUi2FUmUi2FCi2FUi2FUmCmUmAmAmUmGmA i2FGmUmUGal-NAc(L96)-3' (SEQ ID NO: 328)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Conjugate 92

Sense strand: 5'-mC*mA*mCmCmUmUi2FUmUi2FCi2FUi2FUmCmUmAmAmUmGmA i2FGmUmUGalNAc(L96)-3' (SEQ ID NO: 328)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Conjugate 93

Sense strand: 5'-mC*mA*mCmCmUmUi2FUmUi2FCi2FUi2FUmCmUmAmAmUmGmA i2FGmUmUGalNAc(L96)-3' (SEQ ID NO: 328)
Anti-sense strand: 5'-mA*i2FA*mCmUmCi2FAmUmUmAmGmAmAmGi2FAmAi2FAm AmGmGi2FUmG*mG*mG-3' (SEQ ID NO: 179);

Conjugate 94

Sense strand: 5'-mC*mA*mCmCmUmUi2FUmUi2FCi2FUi2FUmCmUmAmAmUmGmA i2FGmUmUGalNAc(L96)-3' (SEQ ID NO: 328)
Anti-sense strand: 5'-mA*i2FA*mCmUmCi2FAmUmUmAmGmAmAmGi2FAmAi2FAm AmGmGmUmG*i2FG*mG-3' (SEQ ID NO: 180);

Conjugate 95

Sense strand: 5'-mC*mA*mCmCmUmUi2FUmUi2FCi2FUi2FUmCmUmAmAmUmGmA mGmUi2FUGalNAc(L96)-3' (SEQ ID NO: 329)
Anti-sense strand: 5'-mA*i2FA*mCi2FUmCi2FAmUmUmAmGmAmAmGi2FAmAi2FA mAmGmGmUmG*mG*mG-3' (SEQ ID NO: 175);

Conjugate 96

Sense strand: 5'-mC*mA*mCmCmUmUi2FUmUi2FCi2FUi2FUmCmUmAmAmUmGmA mGmUi2FUGalNAc(L96)-3' (SEQ ID NO: 329)
Anti-sense strand: 5'-mA*i2FA*mCmUmCi2FAmUi2FUmAmGmAmAmGi2FAmAi2FA mAmGmGmUmG*mG*mG-3' (SEQ ID NO: 176);

Conjugate 97

Sense strand: 5'-mC*mA*mCmCmUmUi2FUmUi2FCi2FUi2FUmCmUmAmAmUmGmA mGmUi2FUGalNAc(L96)-3' (SEQ ID NO: 329)
Anti-sense strand: 5'-mA*i2FA*mCmUmCi2FAmUmUmAi2FGmAmAmGi2FAmAi2FA mAmGmGmUmG*mG*mG-3' (SEQ ID NO: 177);

Conjugate 98

Sense strand: 5'-mC*mA*mCmCmUmUi2FUmUi2FCi2FUi2FUmCmUmAmAmUmGmA mGmUi2FUGalNAc(L96)-3' (SEQ ID NO: 329)
Anti-sense strand: 5'-mA*i2FA*mCmUmCi2FAmUmUmAmGmAmAmGi2FAmAi2FAm Ai2FGmGmUmG*mG*mG-3' (SEQ ID NO: 178);

Conjugate 99

Sense strand: 5'-mC*mA*mCmCmUmUi2FUmUi2FCi2FUi2FUmCmUmAmAmUmGmA mGmUi2FUGalNAc(L96)-3' (SEQ ID NO: 329)
Anti-sense strand: 5'-mA*i2FA*mCmUmCi2FAmUmUmAmGmAmAmGi2FAmAi2FAm AmGmGi2FUmG*mG*mG-3' (SEQ ID NO: 179);

Conjugate 100

Sense strand: 5'-mC*mA*mCmCmUmUi2FUmUi2FCi2FUi2FUmCmUmAmAmUmGmA mGmUi2FUGal-NAc(L96)-3' (SEQ ID NO: 329)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 101

Sense strand: 5'-mG*mA*mCmCmUmUi2FUmUi2FCi2FUi2FUmCi2FUmAmAmUmGm AmGmUmUGal-NAc(L96)-3' (SEQ ID NO: 330)
Anti-sense strand: 5'-mA*i2FA*mCmUmCi2FAmUmUmAmGmAmAmGi2FAmAi2FAm AmGmGmUm-C*i2FG*mG-3' (SEQ ID NO: 186);

Conjugate 102

Sense strand: 5'-mC*mU*mCmCmUmUi2FUmUi2FCi2FUi2FUmCi2FUmAmAmUmGm AmGmUmUGal-NAc(L96)-3' (SEQ ID NO: 331)
Anti-sense strand: 5'-mA*i2FA*mCmUmCi2FAmUmUmAmGmAmAmGi2FAmAi2FAm AmGmGmAm-G*i2FG*mG-3' (SEQ ID NO: 188);

Conjugate 103

Sense strand: 5'-mC*mA*mGmCmUmUi2FUmUi2FCi2FUi2FUmCi2FUmAmAmUmGm AmGmUmUGal-NAc(L96)-3' (SEQ ID NO: 332)
Anti-sense strand: 5'-mA*i2FA*mCmUmCi2FAmUmUmAmGmAmAmGi2FAmAi2FAm AmGmCmUm-G*i2FG*mG-3' (SEQ ID NO: 190);

Conjugate 104

Sense strand: 5'-mC*mA*mCmGmUmUi2FUmUi2FCi2FUi2FUmCi2FUmAmAmUmGm AmGmUmUGal-NAc(L96)-3' (SEQ ID NO: 333)
Anti-sense strand: 5'-mA*i2FA*mCmUmCi2FAmUmUmAmGmAmAmGi2FAmAi2FAm AmCmGmUm-G*i2FG*mG-3' (SEQ ID NO: 192);

Conjugate 105

Sense strand: 5'-mC*mA*mCmCmAmUi2FUmUi2FCi2FUi2FUmCi2FUmAmAmUmGm AmGmUmUGal-NAc(L96)-3' (SEQ ID NO: 334)
Anti-sense strand: 5'-mA*i2FA*mCmUmCi2FAmUmUmAmGmAmAmGi2FAmAi2FAm UmGmGmUm-G*i2FG*mG-3' (SEQ ID NO: 194);

Conjugate 106

Sense strand: 5'-mC*mA*mCmCmUmAi2FUmUi2FCi2FUi2FUmCi2FUmAmAmUmGm AmGmUmUGal-NAc(L96)-3' (SEQ ID NO: 335)
Anti-sense strand: 5'-mA*i2FA*mCmUmCi2FAmUmUmAmGmAmAmGi2FAmAi2FUm AmGmGmUm-G*i2FG*mG-3' (SEQ ID NO: 196);

Conjugate 107

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCmUmCi2FAmUmUmAmGmAmAmGi2FAmAi2FAm AmGmGi2FUm-G*i2FG*mG-3' (SEQ ID NO: 197);

Conjugate 108

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)

Anti-sense strand: 5'-mA*i2FA*mCmUmCi2FAmUi2FUmAmGmAmAmGi2FAmAi2FA mAmGmGmUm-G*i2FG*mG-3' (SEQ ID NO: 198);

Conjugate 109

Sense strand: 5'-mC*mA*mCmCmUmUi2FUmUi2FCi2FUi2FUmCi2FUmAmAmUmGm AmGmUmUGal-NAc(L96)-3' (SEQ ID NO: 325)
Anti-sense strand: 5'-mA*i2FA*mCi2FUmCi2FAmUmUmAmGmAmAmGi2FAmAi2FA mAmGmGmUm-G*i2FG*mG-3' (SEQ ID NO: 199);

Conjugate 110

Sense strand: 5'-mC*mA*mCmCmUmUi2FUmUi2FCi2FUi2FUmCi2FUmAmAmUi2FG mAmGmUmUGal-NAc(L96)-3' (SEQ ID NO: 336)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 111

Sense strand: 5'-mC*mA*mCmCmUmUi2FUmUi2FCi2FUi2FUmCi2FUmAmAmUmGm AmGmUi2FUGal-NAc(L96)-3' (SEQ ID NO: 337)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 112

Sense strand: 5'-mC*mA*mCmCmUmUi2FUmUi2FCmUi2FUmCmUmAmAmUmGmA mGmUmUGal-NAc(L96)-3' (SEQ ID NO: 338)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Conjugate 113

Sense strand: 5'-mC*mA*mCmCmUmUmUmUi2FCmUi2FUmCi2FUmAmAmUmGmA mGmUmUGal-NAc(L96)-3' (SEQ ID NO: 339)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Conjugate 114

Sense strand: 5'-mC*mA*mCmCmUmUmUmUi2FCmUi2FUmCmUmAmAmUi2FGmA mGmUmUGal-NAc(L96)-3' (SEQ ID NO: 340)
Anti-sense strand: 5'-mA*i2FA*mCmUmCi2FAmUmUmAmGmAi2FAmGi2FAmAi2FA mAmGmGmUm-G*i2FG*mG-3' (SEQ ID NO: 203);

Conjugate 115

Sense strand: 5'-mC*mA*mCmCmUmUmUmUi2FCmUi2FUmCmUmAmAmUi2FGmA mGmUmUGal-NAc(L96)-3' (SEQ ID NO: 340)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 116

Sense strand: 5'-mC*mA*mCmCmUmUmUmUi2FCi2FUi2FUmCmUmAmAmUmGmA mGmUmUGal-NAc(L96)-3' (SEQ ID NO: 341)
Anti-sense strand: 5'-mA*i2FA*mCmUmCi2FAmUmUmAmGmAmAmGi2FAmAi2FAm AmGmGmUm-G*i2FG*mG-3' (SEQ ID NO: 180);

Conjugate 117

Sense strand: 5'-mG*mA*mCmCmUmUi2FUmUi2FCi2FUi2FUmCi2FUmAmAmUi2FG mAmGmUmUGalNAc(L96)-3' (SEQ ID NO: 342)
Anti-sense strand: 5'-mA*i2FA*mCmUmCi2FAmUmUmAmGmAmAmGi2FAmAi2FAm AmGmGmUmC*i2FG*mG-3' (SEQ ID NO: 186);

Conjugate 118

Sense strand: 5'-mC*mU*mCmCmUmUi2FUmUi2FCi2FUi2FUmCi2FUmAmAmUi2FG mAmGmUmUGalNAc(L96)-3' (SEQ ID NO: 343)
Anti-sense strand: 5'-mA*i2FA*mCmUmCi2FAmUmUmAmGmAmAmGi2FAmAi2FAm AmGmGmAmG*i2FG*mG-3' (SEQ ID NO: 188);

Conjugate 119

Sense strand: 5'-mC*mA*mGmCmUmUi2FUmUi2FCi2FUi2FUmCi2FUmAmAmUi2FG mAmGmUmUGalNAc(L96)-3' (SEQ ID NO: 344)
Anti-sense strand: 5'-mA*i2FA*mCmUmCi2FAmUmUmAmGmAmAmGi2FAmAi2FAm AmGmCmUmG*i2FG*mG-3' (SEQ ID NO: 190);

Conjugate 120

Sense strand: 5'-mG*mU*mCmCmUmUi2FUmUi2FCi2FUi2FUmCi2FUmAmAmUi2FG mAmGmUmUGalNAc(L96)-3' (SEQ ID NO: 345)
Anti-sense strand: 5'-mA*i2FA*mCmUmCi2FAmUmUmAmGmAmAmGi2FAmAi2FAm AmGmGmAmC*i2FG*mG-3' (SEQ ID NO: 211);

Conjugate 121

Sense strand: 5'-mG*mA*mGmCmUmUi2FUmUi2FCi2FUi2FUmCi2FUmAmAmUi2FG mAmGmUmUGalNAc(L96)-3' (SEQ ID NO: 346)
Anti-sense strand: 5'-mA*i2FA*mCmUmCi2FAmUmUmAmGmAmAmGi2FAmAi2FAm AmGmCmUmC*i2FG*mG-3' (SEQ ID NO: 213);

Conjugate 122

Sense strand: 5'-mC*mU*mGmCmUmUi2FUmUi2FCi2FUi2FUmCi2FUmAmAmUi2FG mAmGmUmUGalNAc(L96)-3' (SEQ ID NO: 347)
Anti-sense strand: 5'-mA*i2FA*mCmUmCi2FAmUmUmAmGmAmAmGi2FAmAi2FAm AmGmCmAmG*i2FG*mG-3' (SEQ ID NO: 215);

Conjugate 123

Sense strand: 5'-mG*mU*mGmCmUmUi2FUmUi2FCi2FUi2FUmCi2FUmAmAmUi2FG mAmGmUmUGalNAc(L96)-3' (SEQ ID NO: 348)
Anti-sense strand: 5'-mA*i2FA*mCmUmCi2FAmUmUmAmGmAmAmGi2FAmAi2FAm AmGmCmAmC*i2FG*mG-3' (SEQ ID NO: 217);

Conjugate 124

Sense strand: 5'-mG*mU*mCmCmUmUi2FUmUi2FCi2FUi2FUmCi2FUmAmAmUmGm AmGmUmUGalNAc(L96)-3' (SEQ ID NO: 349)
Anti-sense strand: 5'-mA*i2FA*mCmUmCi2FAmUmUmAmGmAmAmGi2FAmAi2FAm AmGmGmAmC*i2FG*mG-3' (SEQ ID NO: 211);

Conjugate 125

Sense strand: 5'-mG*mU*mGmCmUmUi2FUmUi2FCi2FUi2FUmCi2FUmAmAmUmGm AmGmUmUGalNAc(L96)-3' (SEQ ID NO: 350)
Anti-sense strand: 5'-mA*i2FA*mCmUmCi2FAmUmUmAmGmAmAmGi2FAmAi2FAm AmGmCmAmC*i2FG*mG-3' (SEQ ID NO: 217);

Conjugate 129

Sense strand: 5'-mU*mC*mCmCmAmCi2FCmUi2FUi2FUi2FUmCi2FUmUmCmUi2FA mAmUmGmAGalNAc(L96)-3' (SEQ ID NO: 351)
Anti-sense strand: 5'-mU*i2FC*mAmUmUi2FAmGmAmAmGmAmAmAi2FAmGi2FGm UmGmGmGmA*i2FG*mA-3' (SEQ ID NO: 221);

Conjugate 130

Sense strand: 5'-mA*mC*mCmCmAmCi2FCmUi2FUi2FUi2FUmCi2FUmUmCmUi2FA mAmUmGmAGalNAc(L96)-3' (SEQ ID NO: 352)
Anti-sense strand: 5'-mU*i2FC*mAmUmUi2FAmGmAmAmGmAmAmAi2FAmGi2FGm UmGmGmGmU*i2FG*mA-3' (SEQ ID NO: 223);

Conjugate 133

Sense strand: 5'-mA*mG*mGmCmAmCi2FCmUi2FUi2FUi2FUmCi2FUmUmCmUi2FA mAmUmGmAGalNAc(L96)-3' (SEQ ID NO: 353)
Anti-sense strand: 5'-mU*i2FC*mAmUmUi2FAmGmAmAmGmAmAmAi2FAmGi2FGm UmGmCmCmU*i2FG*mA-3' (SEQ ID NO: 225);

Conjugate 134

Sense strand: 5'-mA*mC*mCmGmAmCi2FCmAi2FGi2FCi2FUmUi2FGmUmUmUi2FG mUmGmAmAGalNAc(L96)-3' (SEQ ID NO: 354)
Anti-sense strand: 5'-mU*i2FU*mCmAmCi2FAmAmAmCmAmAmGmCi2FUmGi2FGm UmCmGmGmU*i2FU*mG-3' (SEQ ID NO: 227);

Conjugate 135

Sense strand: 5'-mU*mC*mCmGmAmCi2FCmAi2FGi2FCi2FUmUi2FGmUmUmUi2FG mUmGmAmAGalNAc(L96)-3' (SEQ ID NO: 355)
Anti-sense strand: 5'-mU*i2FU*mCmAmCi2FAmAmAmCmAmAmGmCi2FUmGi2FGm UmCmGmGmA*i2FU*mG-3' (SEQ ID NO: 229);

Conjugate 136

Sense strand: 5'-mU*mG*mGmGmAmCi2FCmAi2FGi2FCi2FUmUi2FGmUmUmUi2FG mUmGmAmAGalNAc(L96)-3' (SEQ ID NO: 356)
Anti-sense strand: 5'-mU*i2FU*mCmAmCi2FAmAmAmCmAmAmGmCi2FUmGi2FGm UmCmCmCmA*i2FU*mG-3' (SEQ ID NO: 231);

Conjugate 150

Sense strand: 5'-mC*mA*mCmCmUmUi2FUmUi2FCi2FUi2FUmCi2FUmAmAmUi2FG mAmGmUmAGalNAc(L96)-3' (SEQ ID NO: 357)
Anti-sense strand: 5'-(E)-VPmU*i2FA*mCmUmCi2FAmUmUmAmGmAmAmGi2FAmA i2FAmAmGmGmUmG*i2FG*mG-3' (SEQ ID NO: 249);

Conjugate 151

Sense strand: 5'-mC*mA*mGmCmUmUi2FUmUi2FCi2FUi2FUmCi2FUmAmAmUi2FG mAmGmUmAGalNAc(L96)-3' (SEQ ID NO: 358)

Anti-sense strand: 5'-(E)-VPmU*i2FA*mCmUmCi2FAmUmUmAmGmAmAmGi2FAmA i2FAmAmGmCmUm-G*i2FG*mG-3' (SEQ ID NO: 308);

Conjugate 155

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(E)-VPmU*i2FA*mCmUmCi2FAmUmUmAmGmAmAmGi2FAmA i2FAmAmGmGmUm-G*i2FG*mG-3' (SEQ ID NO: 249);

Conjugate 156

Sense strand: 5'-mC*mU*mCmCmUmUi2FUmUi2FCi2FUi2FUmCi2FUmAmAmUmGm AmGmUmAGal-NAc(L96)-3' (SEQ ID NO: 360)
Anti-sense strand: 5'-(E)-VPmU*i2FA*mCmUmCi2FAmUmUmAmGmAmAmGi2FAmA i2FAmAmGmGmAm-G*i2FG*mG-3' (SEQ ID NO: 251);

Conjugate 157

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(*E*)-VPmU*i2FA*mCmUmCi2FAmUmUmAi2FGmAmAmGi2FAm Ai2FAmAmGmGmUm-G*i2FG*mG-3' (SEQ ID NO: 252);

Conjugate 158

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(E)-VPmU*i2FA*mCmUmCi2FAmUi2FUmAmGmAmAmGi2FAm Ai2FAmAmGmGmUm-G*i2FG*mG-3' (SEQ ID NO: 253);

Conjugate 159

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(E)-VPmU*i2FA*mCi2FUmCi2FAmUmUmAmGmAmAmGi2FAm Ai2FAmAmGmGmUm-G*i2FG*mG-3' (SEQ ID NO: 254);

Conjugate 160

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(E)-VPmU*i2FA*i2FCmUmCi2FAmUmUmAi2FGmAmAmGi2FA mAi2FAmAmGmGmUmG*i2FG*mG-3' (SEQ ID NO: 255);

Conjugate 161

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(*E*)-VPmU*i2FA*mCmUi2FCi2FAmUmUmAmGmAmAmGi2FAm Ai2FAmAmGmGmUm-G*i2FG*mG-3' (SEQ ID NO: 256);

Conjugate 162

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(E)-VPmU*i2FA*mCmUmCi2FAi2FUmUmAmGmAmAmGi2FAm Ai2FAmAmGmGmUm-G*i2FG*mG-3' (SEQ ID NO: 257);

Conjugate 163

Sense strand: 5'-mC*mA*mCmCmUmUi2FUmUi2FCi2FUi2FUmCi2FUmAmAmUmGm AmGmUmAGalNAc(L96)-3' (SEQ ID NO: 359)
Anti-sense strand: 5'-(E)-VPmU*i2FA*mCmUmCi2FAmUi2FUi2FAmGmAmAmGi2FA mAi2FAmAmGmGmUmG*i2FG*mG-3' (SEQ ID NO: 258);

Conjugate 164

Sense strand: 5'-mC*mA*mCmCmUmUi2FUmUi2FCi2FUi2FUmCi2FUmAmAmUmGm AmGmUmAGalNAc(L96)-3' (SEQ ID NO: 359)
Anti-sense strand: 5'-(*E*)-VPmU*i2FA*mCmUmCi2FAmUmUi2FAi2FGmAmAmGi2FA mAi2FAmAmGmGmUmG*i2FG*mG-3' (SEQ ID NO: 259);

Conjugate 165

Sense strand: 5'-mC*mA*mCmCmUmUi2FUmUi2FCi2FUi2FUmCmUmAmAmUi2FGm AmGmUmAGalNAc(L96)-3' (SEQ ID NO: 361)
Anti-sense strand: 5'-(E)-VPmU*i2FA*mCmUmCi2FAmUmUi2FAi2FGmAmAmGi2FA mAi2FAmAmGmGmUmG*i2FG*mG-3' (SEQ ID NO: 259);

Conjugate 166

Sense strand: 5'-mC*mG*mCmCmUmUi2FUmUi2FCi2FUi2FUmCi2FUmAmAmUmGm AmGmUmAGalNAc(L96)-3' (SEQ ID NO: 362)
Anti-sense strand: 5'-(E)-VPmU*i2FA*mCmUmCi2FAmUmUmAmGmAmAmGi2FAmA i2FAmAmGmGmCmG*i2FG*mG-3' (SEQ ID NO: 262);

Conjugate 167

Sense strand: 5'-mC*mA*mCmCmUmUi2FUmUi2FCi2FUi2FUmCi2FUmAmAmUmGm AmGTmAGalNAc(L96)-3' (SEQ ID NO: 363)
Anti-sense strand: 5'-(E)-VPmU*i2FA*mCmUmCi2FAmUmUmAmGmAmAmGi2FAmA i2FAmAmGmGmUmG*i2FG*mG-3' (SEQ ID NO: 249);

Conjugate 168

Sense strand: 5'-mC*mU*mCmCmCmAmCmCmUmUi2FUmUi2FCi2FUi2FUmCi2FUm AmAmUmGmAmGmUmAGalNAc(L96)-3' (SEQ ID NO: 364)
Anti-sense strand: 5'-(E)-VPmU*i2FA*mCmUmCi2FAmUmUmAmGmAmAmGi2FAmA i2FAmAmGmGmUmGmGmGmAmG*i2FA*mC-3' (SEQ ID NO: 265);

Conjugate 169

Sense strand: 5'-mU*mC*mCmCmAmCmCmUmUi2FUmUi2FCi2FUi2FUmCi2FUmAm AmUmGmAmGmUmAGalNAc(L96)-3' (SEQ ID NO: 365)
Anti-sense strand: 5'-(E)-VPmU*i2FA*mCmUmCi2FAmUmUmAmGmAmAmGi2FAmA i2FAmAmGmGmUmGmGmGmA*i2FG*mA-3' (SEQ ID NO: 267);

Conjugate 170

Sense strand: 5'-mC*mC*mCmAmCmCmUmUi2FUmUi2FCi2FUi2FUmCi2FUmAmAm UmGmAmGmUmAGalNAc(L96)-3' (SEQ ID NO: 366)
Anti-sense strand: 5'-(E)-VPmU*i2FA*mCmUmCi2FAmUmUmAmGmAmAmGi2FAmA i2FAmAmGmGmUmGmGmG*i2FA*mG-3' (SEQ ID NO: 269);

Conjugate 171

Sense strand: 5'-mC*mC*mAmCmCmUmUi2FUmUi2FCi2FUi2FUmCi2FUmAmAmUm GmAmGmUmAGalNAc(L96)-3' (SEQ ID NO: 367)
Anti-sense strand: 5'-(E)-VPmU*i2FA*mCmUmCi2FAmUmUmAmGmAmAmGi2FAmA i2FAmAmGmGmUmGmG*i2FG*mA-3' (SEQ ID NO: 271);

Conjugate 172

Sense strand: 5'-mA*mC*mCmUmUi2FUmUi2FCi2FUi2FUmCi2FUmAmAmUmGmAm GmUmAGalNAc(L96)-3' (SEQ ID NO: 368)
Anti-sense strand: 5'-(E)-VPmU*i2FA*mCmUmCi2FAmUmUmAmGmAmAmGi2FAmA i2FAmAmGmGmU*i2FG*mG-3' (SEQ ID NO: 273);

Conjugate 173

Sense strand: 5'-mC*mC*mUmUi2FUmUi2FCi2FUi2FUmCi2FUmAmAmUmGmAmGm UmAGalNAc(L96)-3' (SEQ ID NO: 369)
Anti-sense strand: 5'-(E)-VPmU*i2FA*mCmUmCi2FAmUmUmAmGmAmAmGi2FAmA i2FAmAmGmG*i2FU*mG-3' (SEQ ID NO: 275);

Conjugate 174

Sense strand: 5'-mU*i2FU*mUi2FCi2FUi2FUmCi2FUmAmAmUmGmAmGmUmAGalN Ac(L96)-3'(SEQ ID NO: 370)
Anti-sense strand: 5'-(E)-VPmU*i2FA*mCmUmCi2FAmUmUmAmGmAmAmGi2FAmA i2FAmAmGmG*i2FU*mG-3' (SEQ ID NO: 275);

Conjugate 175

Sense strand: 5'-mU*mU*i2FUmUi2FCi2FUi2FUmCi2FUmAmAmUmGmAmGmUmAG alNAc(L96)-3' (SEQ ID NO: 371)
Anti-sense strand: 5'-(E)-VPmU*i2FA*mCmUmCi2FAmUmUmAmGmAmAmGi2FAmA i2FAmAmGmG*i2FU*mG-3' (SEQ ID NO: 275);

Conjugate 176

Sense strand: 5'-mU*mU*i2FUmUi2FCi2FUi2FUmCi2FUmAmAmUmGmAmGmUmAG alNAc(L96)-3' (SEQ ID NO: 371)
Anti-sense strand: 5'-(E)-VPmU*i2FA*mCmUmCi2FAmUmUmAmGmAmAmGi2FAmA i2FAmAmGmGmUmG*i2FG*mG-3' (SEQ ID NO: 249);

Conjugate 177

Sense strand: 5'-mC*mU*mUi2FUmUi2FCi2FUi2FUmCi2FUmAmAmUmGmAmGmUm AGalNAc(L96)-3' (SEQ ID NO: 372)
Anti-sense strand: 5'-(E)-VPmU*i2FA*mCmUmCi2FAmUmUmAmGmAmAmGi2FAmA i2FAmAmGmGmUmG*i2FG*mG-3' (SEQ ID NO: 249);

Conjugate 178

Sense strand: 5'-mC*mC*mUmUi2FUmUi2FCi2FUi2FUmCi2FUmAmAmUmGmAmGm UmAGalNAc(L96)-3' (SEQ ID NO: 369)
Anti-sense strand: 5'-(E)-VPmU*i2FA*mCmUmCi2FAmUmUmAmGmAmAmGi2FAmA i2FAmAmGmGmUmG*i2FG*mG-3' (SEQ ID NO: 249);

Conjugate 179

Sense strand: 5'-mU*i2FU*mUi2FCi2FUi2FUmCi2FUmAmAmUmGmAmGmUmAGalN Ac(L96)-3' (SEQ ID NO: 370)

Anti-sense strand: 5'-(E)-VPmU*i2FA*mCmUmCi2FAmUmUmAmGmAmAmGi2FAmA i2FAmA*i2FG*mG-3' (SEQ ID NO: 279);

Conjugate 180

Sense strand: 5'-mU*mU*i2FUmUi2FCi2FUi2FUmCi2FUmAmAmUmGmAmGmUmAG alNAc(L96)-3' (SEQ ID NO: 371)
Anti-sense strand: 5'-(E)-VPmU*i2FA*mCmUmCi2FAmUmUmAmGmAmAmGi2FAmA i2FAmA*i2FG*mG-3' (SEQ ID NO: 279);

Conjugate 181

Sense strand: 5'-mC*mA*mCmCmUmUi2FUmUi2FCi2FUi2FUmCi2FUmAmAmUmGm AmGmUmAGal-NAc(L96)-3' (SEQ ID NO: 359)
Anti-sense strand: 5'-(*E*)-VPmU*i2FA*mCmUmCi2FAi2FUmUmAi2FGmAmAmGi2FA mAi2FAmAmGmGmUmG*i2FG*mG-3' (SEQ ID NO: 280);

Conjugate 182

Sense strand: 5'-mC*mU*mCmCmUmUi2FUmUi2FCi2FUi2FUmCi2FUmAmAmUmGm AmGmUmAGal-NAc(L96)-3' (SEQ ID NO: 360)
Anti-sense strand: 5'-(E)-VPmU*i2FA*mCmUmCi2FAi2FUmUmAmGmAmAmGi2FAm Ai2FAmAmGmGmAm-G*i2FG*mG-3' (SEQ ID NO: 281);

Conjugate 183

Sense strand: 5'-mC*mG*mCmCmUmUi2FUmUi2FCi2FUi2FUmCi2FUmAmAmUmGm AmGmUmAGal-NAc(L96)-3' (SEQ ID NO: 362)
Anti-sense strand: 5'-(E)-VPmU*i2FA*mCmUmCi2FAi2FUmUmAmGmAmAmGi2FAm Ai2FAmAmGmGmCm-G*i2FG*mG-3' (SEQ ID NO: 282);

Conjugate 184

Sense strand: 5'-mC*mU*mCmCmUmUi2FUmUi2FCi2FUi2FUmCi2FUmAmAmUmGm AmGmUmAGal-NAc(L96)-3' (SEQ ID NO: 360)
Anti-sense strand: 5'-(E)-VPmU*i2FA*mCmUmCi2FAi2FUmUmAi2FGmAmAmGi2FA mAi2FAmAmGmGmAm-G*i2FG*mG-3' (SEQ ID NO: 283);

Conjugate 185

Sense strand: 5'-mC*mG*mCmCmUmUi2FUmUi2FCi2FUi2FUmCi2FUmAmAmUmGm AmGmUmAGal-NAc(L96)-3' (SEQ ID NO: 362)
Anti-sense strand: 5'-(*E*)-VPmU*i2FA*mCmUmCi2FAi2FUmUmAi2FGmAmAmGi2FA mAi2FAmAmGmGmCmG*i2FG*mG-3' (SEQ ID NO: 284);

Conjugate 186

Sense strand: 5'-mU*mU*i2FUmUi2FCi2FUi2FUmCi2FUmAmAmUmGmAmGmUmAG alNAc(L96)-3' (SEQ ID NO: 371)
Anti-sense strand: 5'-(E)-VPmU*i2FA*mCmUmCi2FAmUmUmAi2FGmAmAmGi2FAm Ai2FAmAmGmG*i2-FU*mG-3' (SEQ ID NO: 285);

Conjugate 187

Sense strand: 5'-mU*mU*i2FUmUi2FCi2FUi2FUmCi2FUmAmAmUmGmAmGmUmAG alNAc(L96)-3' (SEQ ID NO: 371)
Anti-sense strand: 5'-(E)-VPmU*i2FA*mCmUmCi2FAi2FUmUmAmGmAmAmGi2FAm Ai2FAmAmGmG*i2-FU*mG-3' (SEQ ID NO: 286);

Conjugate 188

Sense strand: 5'-mU*mU*i2FUmUi2FCi2FUi2FUmCi2FUmAmAmUmGmAmGmUmAG alNAc(L96)-3' (SEQ ID NO: 372)
Anti-sense strand: 5'-(E)-VPmU*i2FA*mCmUmCi2FAi2FUmUmAi2FGmAmAmGi2FA mAi2FAmAmGmG*i2-FU*mG-3' (SEQ ID NO: 287);

Conjugate 189

Sense strand: 5'-mA*mC*mCmUmUi2FUmUi2FCi2FUi2FUmCi2FUmAmAmUmGmAm GmUmAGal-NAc(L96)-3' (SEQ ID NO: 368)
Anti-sense strand: 5'-(E)-VPmU*i2FA*mCmUmCi2FAi2FUmUmAmGmAmAmGi2FAm Ai2FAmAmGmGmU*i2FG*mG-3' (SEQ ID NO: 288);

Conjugate 190

Sense strand: 5'-mU*mC*mCmUmUi2FUmUi2FCi2FUi2FUmCi2FUmAmAmUmGmAm GmUmAGal-NAc(L96)-3' (SEQ ID NO: 373)
Anti-sense strand: 5'-(E)-VPmU*i2FA*mCmUmCi2FAi2FUmUmAmGmAmAmGi2FAm Ai2FAmAmGmGmA*i2FG*mG-3' (SEQ ID NO: 290);

Conjugate 191

Sense strand: 5'-mA*mG*mCmUmUi2FUmUi2FCi2FUi2FUmCi2FUmAmAmUmGmAm GmUmAGal-NAc(L96)-3' (SEQ ID NO: 374)
Anti-sense strand: 5'-(*E*)-VPmU*i2FA*mCmUmCi2FAi2FUmUmAmGmAmAmGi2FAm Ai2FAmAmGmCmU*i2FG*mG-3' (SEQ ID NO: 292);

Conjugate 192

Sense strand: 5'-mA*mC*mGmUmUi2FUmUi2FCi2FUi2FUmCi2FUmAmAmUmGmAm GmUmAGal-NAc(L96)-3' (SEQ ID NO: 375)
Anti-sense strand: 5'-(E)-VPmU*i2FA*mCmUmCi2FAi2FUmUmAmGmAmAmGi2FAm Ai2FAmAmCmGmU*i2FG*mG-3' (SEQ ID NO: 294);

Conjugate 193

Sense strand: 5'-mG*mC*mCmUmUi2FUmUi2FCi2FUi2FUmCi2FUmAmAmUmGmAm GmUmAGal-NAc(L96)-3' (SEQ ID NO: 376)
Anti-sense strand: 5'-(E)-VPmU*i2FA*mCmUmCi2FAi2FUmUmAmGmAmAmGi2FAm Ai2FAmAmGmGm-C*i2FG*mG-3' (SEQ ID NO: 296);

Conjugate 194

Sense strand: 5'-mC*mC*mAmCmCmUmUi2FUmUi2FCi2FUi2FUmCi2FUmAmAmUm GmAmGmUmAGal-NAc(L96)-3' (SEQ ID NO: 367)
Anti-sense strand: 5'-(E)-VPmU*i2FA*mCmUmCi2FAi2FUmUmAmGmAmAmGi2FAm Ai2FAmAmGmGmUmGmG*i2FG*mA-3' (SEQ ID NO: 297);

Conjugate 195

Sense strand: 5'-mG*mC*mAmCmCmUmUi2FUmUi2FCi2FUi2FUmCi2FUmAmAmUm GmAmGmUmAGal-NAc(L96)-3' (SEQ ID NO: 377)
Anti-sense strand: 5'-(E)-VPmU*i2FA*mCmUmCi2FAi2FUmUmAmGmAmAmGi2FAm Ai2FAmAmGmGmUmGmC*i2FG*mA-3' (SEQ ID NO: 299);

Conjugate 196

Sense strand: 5'-mC*mG*mAmCmCmUmUi2FUmUi2FCi2FUi2FUmCi2FUmAmAmUm GmAmGmUmAGalNAc(L96)-3' (SEQ ID NO: 378)
Anti-sense strand: 5'-(E)-VPmU*i2FA*mCmUmCi2FAi2FUmUmAmGmAmAmGi2FAm Ai2FAmAmGmGmUmCmG*i2FG*mA-3' (SEQ ID NO: 301);

Conjugate 197

Sense strand: 5'-mC*mC*mUmCmCmUmUi2FUmUi2FCi2FUi2FUmCi2FUmAmAmUm GmAmGmUmAGalNAc(L96)-3' (SEQ ID NO: 379)
Anti-sense strand: 5'-(*E*)-VPmU*i2FA*mCmUmCi2FAi2FUmUmAmGmAmAmGi2FAm Ai2FAmAmGmGmAmGmG*i2FG*mA-3' (SEQ ID NO: 303);

Conjugate 198

Sense strand: 5'-mC*mC*mGmCmCmUmUi2FUmUi2FCi2FUi2FUmCi2FUmAmAmUm GmAmGmUmAGalNAc(L96)-3' (SEQ ID NO: 380)
Anti-sense strand: 5'-(E)-VPmU*i2FA*mCmUmCi2FAi2FUmUmAmGmAmAmGi2FAm Ai2FAmAmGmGmCmGmG*i2FG*mA-3' (SEQ ID NO: 305);

Conjugate 199

Sense strand: 5'-mC*mA*mCmCmUmUi2FUmUi2FCi2FUi2FUmCi2FUmAmAmUi2FG mAmGmUmAGalNAc(L96)-3' (SEQ ID NO: 357)
Anti-sense strand: 5'-(E)-VPmU*i2FA*mCmUmCi2FAmUi2FUi2FAmGmAmAmGi2FA mAi2FAmAmGmGmUmG*i2FG*mG-3' (SEQ ID NO: 258);

Conjugate 200

Sense strand: 5'-mG*mA*mCmCmUmUi2FUmUi2FCi2FUi2FUmCi2FUmAmAmUmGm AmGmUmAGalNAc(L96)-3' (SEQ ID NO: 381)
Anti-sense strand: 5'-(E)-VPmU*i2FA*mCmUmCi2FAi2FUmUmAmGmAmAmGi2FAm Ai2FAmAmGmGmUmC*i2FG*mG-3' (SEQ ID NO: 382);

Conjugate 201

Sense strand: 5'-mC*mA*mGmCmUmUi2FUmUi2FCi2FUi2FUmCi2FUmAmAmUmGm AmGmUmAGalNAc(L96)-3' (SEQ ID NO: 383)
Anti-sense strand: 5'-(E)-VPmU*i2FA*mCmUmCi2FAi2FUmUmAmGmAmAmGi2FAm Ai2FAmAmGmCmUmG*i2FG*mG-3' (SEQ ID NO: 384);

Conjugate 202

Sense strand: 5'-mC*mA*mCmGmUmUi2FUmUi2FCi2FUi2FUmCi2FUmAmAmUmGm AmGmUmAGalNAc(L96)-3' (SEQ ID NO: 385)

Anti-sense strand: 5'-(E)-VPmU*i2FA*mCmUmCi2FAi2FUmUmAmGmAmAmGi2FAm Ai2FAmAmCmGmUmG*i2FG*mG-3' (SEQ ID NO: 386);

Conjugate 203

Sense strand: 5'-mC*mA*mCmCmAmUi2FUmUi2FCi2FUi2FUmCi2FUmAmAmUmGm AmGmUmAGalNAc(L96)-3' (SEQ ID NO: 387)

Anti-sense strand: 5'-(*E*)-VPmU*i2FA*mCmUmCi2FAi2FUmUmAmGmAmAmGi2FAm Ai2FAmUmGmGmUmG*i2FG*mG-3' (SEQ ID NO: 388);

Conjugate 204

Sense strand: 5'-mC*mA*mCmCmUmAi2FUmUi2FCi2FUi2FUmCi2FUmAmAmUmGm AmGmUmAGalNAc(L96)-3' (SEQ ID NO: 389)

Anti-sense strand: 5'-(E)-VPmU*i2FA*mCmUmCi2FAi2FUmUmAmGmAmAmGi2FAm Ai2FUmAmGmGmUmG*i2FG*mG-3' (SEQ ID NO: 390).

In some embodiments of the present disclosure, the siRNA conjugate may be selected from at least one of conjugates 101-103, conjugate 110, conjugate 117 to conjugate 125, conjugate 129 to conjugate 130, conjugate 133 to conjugate 136, conjugate 150 to conjugate 151, conjugate 155 to conjugate 199 (e.g., conjugate 155 to conjugate 157, conjugate 162, conjugate 166, conjugate 171 to conjugate 172, conjugate 181 to conjugate 198).

In some embodiments of the present disclosure, the siRNA comprises a sense strand and an antisense strand, wherein the antisense strand comprises a sequence set forth in any one selected from SEQ ID NOs: 186, 188, 190, 180, 211, 213, 215, 217, 221, 223, 225, 227, 229, 231, 249, 308, 251, 252, 253, 254, 255, 256, 257, 258, 259, 262, 265, 267, 269, 271, 273, 275, 279, 280, 281, 282, 283, 284, 285, 286, 287, 288, 290, 292, 294, 296, 297, 299, 301, 303, 305, 382, 384, 386, 388 and 390 (e.g., a sequence set forth in any one of SEQ ID NOs: 186, 188, 190, 180, 211, 213, 215, 217, 221, 223, 225, 227, 229, 231, 249, 308, 251, 252, 257, 262, 271, 273, 280, 281, 282, 283, 284, 285, 286, 287, 288, 290, 292, 294, 296, 297, 299, 301, 303 and 305).

In some embodiments of the present disclosure, the siRNA conjugate may be selected from at least one of conjugate 150, conjugate 151, conjugate 155, conjugate 156, conjugate 157, conjugate 162, conjugate 166, conjugate 171, conjugate 172, conjugate 175, conjugate 181, conjugate 182, conjugate 183, conjugate 184, conjugate 185. In some embodiments of the present disclosure, the siRNA conjugate may be selected from conjugate 155, conjugate 156, conjugate 157, conjugate 162, conjugate 171, conjugate 172, conjugate 175, conjugate 182, conjugate 183 and conjugate 184 (e.g., conjugate 155, conjugate 156, conjugate 157, conjugate 162, conjugate 171, conjugate 172, conjugate 175, conjugate 182, conjugate 183). In some embodiments of the present disclosure, the siRNA conjugate may be selected from conjugate 157, conjugate 158, conjugate 159, conjugate 160, conjugate 161, conjugate 162, conjugate 163, conjugate 164, and conjugate 165.

In some embodiments of the present disclosure, the siRNA conjugate comprises a sense strand and an antisense strand, wherein the antisense strand comprises a sequence set forth in any one selected from SEQ ID NOs: 249, 251, 252, 257, 271, 273, 275, 281, 282 and 283 (e.g., SEQ ID NOs: 249, 251, 252, 257, 271, 273, 275, 281 and 282). In some embodiments of the present disclosure, the antisense strand comprises a sequence selected from SEQ ID NOs: 252, 253, 254, 255, 256, 257, 258, and 259.

In some embodiments of the present disclosure, the siRNA conjugate may be selected from conjugate 155, conjugate 156, conjugate 162, conjugate 182 and conjugate 184. In further embodiments, the siRNA conjugate may be selected from conjugate 156, conjugate 182 and conjugate 184.

In some embodiments of the present disclosure, the siRNA conjugate comprises a sense strand and an antisense strand, wherein the antisense strand comprises the following sequence in which all nucleotides are modified nucleotides:
UACUCAUUAGAAGAAAAGG$X_1$GGG (SEQ ID NO: 396)

wherein $X_1$ is A or U,
the nucleotides at positions 2, 6, 14, 16, and 22 in the direction from the 5' end to the 3' end of the antisense strand are 2'-fluoro nucleotides; the nucleotide at position 7 or the nucleotides at positions 7 and 10 in the direction from the 5' end to the 3' end of the antisense strand may further be 2'-fluoro nucleotides; the nucleotides at other positions of the antisense strand may be 2'-methoxy nucleotides;
the nucleotide at position 1 in the direction from the 5' end to the 3' end of the antisense strand further has an (E)-VP modification;
phosphorothioate groups exist between the nucleotides at positions 1 and 2, and between the nucleotides at positions 2 and 3 at the 5' end of the antisense strand, as well as between the nucleotides at positions 1 and 2, and between the nucleotides at positions 2 and 3 at the 3' end of the antisense strand .

In some embodiments of the present disclosure, the siRNA conjugate comprises a sense strand and an antisense strand, wherein the antisense strand comprises the following sequence in the 5' to 3' direction:

(*E*)-VPmU*i2FA*mCmUmCi2FA$X_2$mUmA$X_3$mAmAmGi2FAmAi2FAmAmGmGm$X_4$mG*i2FG*mG (SEQ ID NO: 397)

wherein $X_2$ is mU or i2FU, $X_3$ is mG or i2FG, and $X_4$ is A or U.

In further embodiments, the sense strand comprises the following sequence in the 5' to 3' direction: mC*m$X_5$*mCmCmUmUi2FUmUi2FCi2FUi2FUmCi2FUmAmAmUmGmAmGmUm-AGalNAc(L96) (SEQ ID NO: 398), wherein $X_5$ is A or U.

In some embodiments of the present disclosure, the siRNA conjugate comprises a sense strand and an antisense strand, wherein the antisense strand comprises a sequence set forth in any one selected from SEQ ID NOs: 249, 251, 257, 281 and 283. In further embodiments, the sense strand comprises a sequence set forth in SEQ ID NO: 359, or SEQ ID NO: 360; in still further embodiments, the sense strand comprises the sequence set forth in SEQ ID NO: 360.

In some embodiments of the present disclosure, the siRNA conjugate may be conjugate 156 or conjugate 182.

In some embodiments of the present disclosure, the siRNA conjugate comprises a sense strand and an antisense strand, wherein the antisense strand comprises the sequence set forth in SEQ ID NO: 399:

5'-(*E*)-VPmU*i2FA*mCmUmCi2FA$X_6$mUmAmGmAmAmGi2FAmAi2FAmAmGmGmAmG*i2FG*mG-3' (SEQ ID NO: 399),

wherein $X_6$ is i2FU or mU.

In some embodiments of the present disclosure, the siRNA comprises a sense strand and an antisense strand, wherein the antisense strand comprises the sequence set forth in SEQ ID NO: 251 or 281. In further embodiments, the sense strand comprises the sequence set forth in SEQ ID NO: 360.

In some embodiments of the present disclosure, the siRNA conjugate comprises a sense strand and an antisense strand, wherein the antisense strand comprises the following sequence in the 5' to 3' direction:

(*E*)-VPmU*i2FA*$X_7X_8X_9$i2FA$X_{10}X_{11}X_{12}X_{13}$mAmAmGi2FAmAi2FAmAmGmGmUmG*i2FG*mG (SEQ ID NO: 400),

wherein $X_7$ is mC or i2FC, $X_8$, $X_{10}$, and $X_{11}$ are mU or i2FU, $X_9$ is mC or i2FC, $X_{12}$ is mA or i2FA, and $X_{13}$ is mG or i2FG.

In further embodiments, the sense strand comprises the following sequence in the 5' to 3' direction: mC*mA*mCmCmUmUi2FUmUi2FCi2FUi2FUmC$X_{14}$mAmAmU$X_{15}$mAmGmUmAGal-NAc(L96) (SEQ ID NO: 401), wherein $X_{14}$ is i2FU or mU, $X_{15}$ is mG or i2FG.

In some embodiments of the present disclosure, the siRNA comprises a sense strand and an antisense strand, wherein the antisense strand comprises a sequence set forth in any one of SEQ ID NOs: 252, 253, 254, 255, 256, 257, 258 and 259. In further embodiments, the sense strand comprises a sequence set forth in SEQ ID NO: 359 or 361.

The present disclosure also provides an siRNA or a conjugate thereof, the sense strand and antisense strand of which have at least 90%, 91%, 92%, 93%, 94%, 95%, 96%, 97%, 98%, or 99% sequence identity to the full-length nucleotide sequences of the sense strand and antisense strand described above, respectively, and the siRNA or the conjugate thereof is capable of effectively inhibiting the expression of the AGT gene.

In some embodiments of the present disclosure, the duplex, in which the nucleotide at position 19, 20 or 21 in the direction from the 5' end to the 3' end of the antisense strand is mismatched with the target sequence, is preferably a duplex in which the nucleotide at position 20 in the direction from the 5' end to the 3' end of the antisense strand is mismatched with the target sequence, and the nucleotides of the antisense strand and the target sequence are identical at the position where the antisense strand is mismatched with the target sequence (e.g., duplex 22, duplex 26). When a preferred modification pattern is selected (for example, the nucleotides at positions 9, 11, 12, 13, and 15 in the direction from the 3' end to the 5' end of the sense strand are 2'-fluoro nucleotides, the nucleotides at other positions of the sense strand are 2'-methoxy nucleotides, the nucleotides at positions 2, 6, 7, 14, 16, and 22 in the direction from the 5' end to the 3' end of the antisense strand are 2'-fluoro nucleotides, and the nucleotides at other positions of the antisense strand are 2'-methoxy nucleotides), the gene silencing efficacy of the resulting modified duplexes or the conjugates further obtained is improved compared to modified duplexes or further obtained conjugates obtained from duplexes without mismatches at the above-mentioned positions (e.g., duplex 21, duplex 24) under the same modification patterns.

In some embodiments of the present disclosure, when a preferred modification pattern is selected (for example, the nucleotides at positions 9, 11, 12, 13, and 15 in the direction from the 3' end to the 5' end of the sense strand are 2'-fluoro nucleotides, the nucleotides at other positions of the sense strand are2'-methoxy nucleotides, the nucleotides at positions

2, 6, 7, 14, 16, and 22 in the direction from the 5' end to the 3' end of the antisense strand are 2'-fluoro nucleotides, and the nucleotides at other positions of the antisense strand are 2'-methoxy nucleotides), the gene silencing efficacy of the resulting modified duplexes or the conjugates further obtained from duplex 26 in which the nucleotide at position 20 in the direction from the 5' end to the 3' end of the antisense strand is mismatched with the target sequence is significantly improved, compared to those obtained from duplexes without mismatches at the above-mentioned positions under the same modification patterns.

In some embodiments of the present disclosure, the gene silencing efficacy of conjugate 182 adopting a specific mismatch pattern (the nucleotide at position 20 in the direction from the 5' end to the 3' end of the antisense strand is identical to the nucleotide in the target sequence) is improved, compared to conjugate 162 with the same modification pattern in which the nucleotide at position 20 in the direction from the 5' end to the 3' end of the antisense strand is not mismatched with the target sequence.

3. The siRNA pharmaceutical composition of the present disclosure

The present disclosure also provides a pharmaceutical composition, comprising the siRNA or siRNA conjugate described in any embodiment of the present disclosure as an active ingredient and a pharmaceutically acceptable carrier.

In some embodiments of the present disclosure, the pharmaceutical composition comprises one siRNA or siRNA conjugate described in any embodiment of the present disclosure. In other embodiments of the present disclosure, the pharmaceutical composition comprises at least two (including but not limited to two, three, four, five, six, seven, eight, nine, ten or more) siRNAs or siRNA conjugates described in any embodiment of the present disclosure as active ingredients. In some embodiments of the present disclosure, the at least two siRNAs or siRNA conjugates described in any embodiment of the present disclosure each target different target sequences in the AGT gene, thereby a synergistic effect brought by their simultaneous action on different target sequences may be expected. Here, the "different target sequences" means that there is no overlap between target sequences or the number of contiguous overlapping nucleotides between target sequences is less than 5 (e.g., the number of contiguous overlapping nucleotides is 4, 3, 2, 1, or 0). In this case, the at least two siRNAs or siRNA conjugates described in any embodiment of the present disclosure may be present in any different ratios. In some embodiments of the present disclosure, the at least two siRNAs or siRNA conjugates described in the first aspect may be present in a molar ratio of 1:100 to 100:1 relative to each other. In some embodiments of the present disclosure, the at least two siRNAs or siRNA conjugates described in the first aspect may be present in a molar ratio of 1:70 to 70:1 relative to each other. In some embodiments of the present disclosure, the at least two siRNAs described in the first aspect or siRNA conjugates described in the second aspect may be present in a molar ratio of 1:50 to 50:1 relative to each other. In some embodiments of the present disclosure, the at least two siRNAs described in the first aspect or siRNA conjugates described in the second aspect may be present in a molar ratio of 1:10 to 10:1 relative to each other. In some embodiments of the present disclosure, the at least two siRNAs described in the first aspect or siRNA conjugates described in the second aspect may be present in a molar ratio of 1:5 to 5:1 relative to each other. In some embodiments of the present disclosure, the at least two siRNAs described in the first aspect or siRNA conjugates described in the second aspect may be present in a molar ratio of 1:2 to 2:1 relative to each other. In some embodiments of the present disclosure, the at least two siRNAs described in the first aspect or siRNA conjugates described in the second aspect are present in a molar ratio of 1:1.

In some embodiments of the present disclosure, the pharmaceutically acceptable carrier may include, but is not limited to, one or more of magnetic nanoparticles (such as $Fe_3O_4$, $Fe_2O_3$), gold nanoparticles, quantum dots, silica nanoparticles, carbon nanotubes, mesoporous silicon, calcium phosphate nanoparticles, polyethyleneimine, polyamidoamine dendrimers, poly-L-lysine, chitosan, 1,2-dioleoyl-3-trimethylammonium propane, poly-(D-lactic-co-glycolic acid) or poly-(L-lactic-co-glycolic acid), poly(2-aminoethyl ethylene phosphate) and poly(N,N-dimethylaminoethyl methacrylate), as well as the derivatives thereof.

In the pharmaceutical composition of the present disclosure, there is no special requirement for the content of the pharmaceutically acceptable carrier. In some embodiments of the present disclosure, the ratio of the total weight of siRNA contained in the pharmaceutical composition to the total weight of the pharmaceutically acceptable carrier may be 1:(1-500). In some embodiments of the present disclosure, the ratio of the total weight of siRNA contained in the pharmaceutical composition to the total weight of the pharmaceutically acceptable carrier may be 1:(1-300). In some embodiments of the present disclosure, the ratio of the total weight of siRNA contained in the pharmaceutical composition to the total weight of the pharmaceutically acceptable carrier may be 1:(1-100). In some embodiments of the present disclosure, the ratio of the total weight of siRNA or siRNA conjugate contained in the pharmaceutical composition to the total weight of the pharmaceutically acceptable carrier may be 1:(1-50).

In some embodiments of the present disclosure, the pharmaceutical composition may also comprise a cationic component to assist in the *in vivo* delivery of the drug. In some embodi ments of the present disclosure, the cationic component may include, but is not limited to, at least one of positively charged polypeptides or proteins, cationic lipids, positively charge d polymers, cationic nanoemulsions (e.g., formed by mixing DOTAP with squalene, sorbit an trioleate,

polysorbate 80 in a citric acid buffer at pH 6.5), etc. In some embodiments of t he present disclosure, the positively charged polypeptides or proteins may include at least o ne of oligoarginine, oligolysine, protamine, etc. In some embodiments of the present disclo sure, the cationic lipids may be selected from at least one of dimethyldioctadecylammoniu m bromide (DDAB), 1,2-dimyristoyl-3-trimethylammonium propane, 1,2-dioleoyl-3-trimet hylammonium propane (DOTAP), 1,2-dioleoyl-3-trimethylammonium propane (methyl sul fate), 1,2-dipalmitoyl-3-trimethylammonium propane, 1,2-distearoyl-3-trimethylammoniu m propane, N-(1-(2,3-dioleoyloxy)propyl)-N,N,N-trimethylammonium chloride (DOTMA) , dimyristyloxypropyl dimethylhydroxyethyl ammonium bromide (DMRIE), dioleoyloxypr opyl dimethylhydroxyethylammonium bromide (DORIE), dimethyldi(dodecyl) ammonium bromide, N-(a-trimethylammonioacetyl)-di(dodecyl)-D-glutamine hydrochloride, N-(a-tri methylammonioacetyl)-O,O'-bis-(1H,1H,2H,2H-perfluorodecyl)-L-glutamine hydrochlori de, O,O'-di(dodecanoyl)-N-(a-trimethylammonioacetyl)diethanolamine hydrochloride, met hylallyldi(dodecyl)ammonium bromide, N-{p-(w-trimethylammoniobutoxy)-benzoyl}-di(d odecyl)-L-glutamine hydrochloride, 9-(w-trimethylammoniobutyl)-3,6-bis(dodecanoyl)car bazole bromide, dimethyldi(octadecyl)ammonium hydrochloride, N-w-trimethylammoniod ecanoyl-di(hexadecyl)-D-glutamine bromide,N-{p-(w-trimethylammoniohexyloxy)-benzo yl}-di(tetradecyl)-L-glutamine bromide, p-(w-trimethylammoniodecyloxy)-p'-octyloxyazo benzene bromide (MC-1-0810), p-{w-(b-hydroxyethyl)dimethyl-ammonium-decyloxy}-p'-octyloxyazobenzene bromide salt (MC-3-0810), O,O',O"-tris(dodecanoyl)-N-(w-trimethyl-ammoniodecanoyl)-tris(hydroxymethyl)aminomethane bromide (TC-1-12), 1,2-dilauryl-gl ycero-3-ethylphosphocholine, 1,2-dimyristoyl-glycero-3-ethylphosphocholine, 1,2-dipalmi toyl-glycero-3-ethylphosphocholine, 1,2-distearoyl-glycero-3-ethylphosphocholine, 1,2-dio leoyl-glycero-3-ethylphosphocholine, 1-palmitoyl-2-oleoyl-glycero-3-ethylphosphocholine , N,N-dihydroxyethyl-N-methyl-N-2-(cholesteryloxycarbonylamino)ethylammonium brom ide (BHEM-Chol), (2,3-dioleoyloxypropyl)trimethylammonium chloride (DOTAP) and N-(1-(2,3-dioleoyloxy)propyl)-N,N,N-trimethylammonium chloride. In some embodiments o f the present disclosure, the positively charged polymers may be selected from at least one of poly-L-lysine, poly-L-arginine, polyethyleneimine, poly(β-amino ester), chitosan, dextra n (e.g., DEAE-dextran, diethylaminoethyl dextran), hyaluronic acid, chitosan quaternary a mmonium salt.

For the same purpose, the pharmaceutical composition may also comprise a non-cationic component. The non-cationic component may include, but is not limited to, at least one of neutral membrane fusogenic lipids, anionic lipids, amphiphilic polymers. In some embodiments of the present disclosure, the membrane fusogenic lipids may include at least one of dioleoyl phosphatidylethanolamine, dioleoyl phosphatidylcholine, trans-phosphatidylethanolamine, 1,2-bis(10,12-tricosadiacyl)-phosphoethanolamine, 1,2-dielaidoyl phosphoethanolamine, 1,2-di(hexadecyl)phosphoethanolamine, 1,2-dihexanoyl phosphoethanolamine, 1,2-dilauroyl phosphoethanolamine, 1,2-dilinoleoyl phosphoethanolamine, 1,2-dimyristoyl phosphoethanolamine, 1,2-dioleoyl phosphoethanolamine, 1,2-dipalmitoleoyl phosphoethanolamine, 1,2-dipalmitoyl phosphoethanolamine, 1,2-diphytanoyl phosphoethanolamine, 1,2-distearoyl phosphoethanolamine, 1-palmitoyl-2-oleoyl phosphoethanolamine, 1-palmitoyl-2-(10,12-tricosadiacyl) phosphoethanolamine, 1,2-dioleoyl phosphoethanolamine-N-hexanamide, 1,2-dipalmitoyl phosphoethanolamine-N-hexanamide, N,N-dimethyl-1,2-dioleoyl phosphoethanolamine, N,N-dimethyl-1,2-dipalmitoyl phosphoethanolamine, N-dodecanoyl-1,2-dipalmitoyl phosphoethanolamine, N-dodecanoyl-1,2-dioleoyl phosphoethanolamine, 1,2-dioleoyl phosphoethanolamine-N-dodecylamine, 1,2-dipalmitoyl phosphoethanolamine-N-dodecylamine, 1,2-dioleoyl phosphoethanolamine-N-glutaryl, 1,2-dipalmitoyl phosphoethanolamine-N-glutaryl, 1,2-dioleoyl phosphoethanolamine-N-lactosyl, 1,2-dioleoyl phosphoethanolamine-N-[4-(p-maleimidomethyl)cyclohexane-carboxylate], dipalmitoyl phosphoethanolamine-N-[4-(p-maleimidomethyl) cyclohexane-carboxylate], 1,2-dipalmitoyl phosphoethanolamine-N-[4-(p-maleimidophenyl)butyramide], 1,2-dioleoyl phosphoethanolamine-N-[4-(p-maleimidophenyl)butyrate], N-methyl-1,2-dioleoyl phosphoethanolamine, N-methyl-dipalmitoyl phosphoethanolamine, 1,2-dioleoyl phosphoethanolamine-N-[3-(2-pyridyldithio)propionate], 1,2-dipalmitoyl phosphoethanolamine-N-[3-(2-pyridyldithio)propionate], N-(succinyl)-1,2-dioleoyl phosphoethanolamine, N-(succinyl)-1,2-dipalmitoyl phosphoethanolamine. The transport and delivery efficiency of the pharmaceutical composition in mammalian bodies can be further improved by adding the above-mentioned membrane fusogenic lipids in the pharmaceutical composition of the present disclosure. In some embodiments of the present disclosure, the amphiphilic polymers may include at least one of polyethylene glycol-polylactic acid diblock copolymers, polyethylene glycol-polylactic acid triblock copolymers, polyethylene glycol-poly(lactic-co-glycolic acid) diblock copolymers or polyethylene glycol-poly (lactic-co-glycolic acid) triblock copolymers, polycaprolactone-polyphosphoester diblock copolymers, polycaprolactone-polyphosphoester triblock copolymers, polyethylene glycol-polycaprolactone diblock copolymers, polyethylene glycol-polycaprolactone triblock copolymers.

In some embodiments of the present disclosure, the pharmaceutical composition may also comprise other pharmaceutically acceptable excipients. In some embodiments of the present disclosure, the other pharmaceutically acceptable excipients may include at least one of pH buffers, protective agents, and osmotic pressure regulators. In some embodiments of the present disclosure, the pH buffer may be one or two of a tris(hydroxymethyl)aminomethane hydrochloride buffer of pH 7.5-8.5 and a phosphate buffered saline of pH 5.5-8.5. In some embodiments of the present disclosure, the pH buffer may be a phosphate buffered saline of pH 5.5-8.5. In some embodiments of the present disclosure, the protective agent may be at least one of inositol, sorbitol, sucrose, trehalose, mannose, maltose, lactose, and glucose. In some embodiments of the present disclosure, based on the total weight of the pharmaceutical composition, the weight proportion of the protective agent may be 0.01%-30%. In some embodiments of the present disclosure, based on the total weight of the pharmaceutical composition, the weight proportion of the protective agent may be 0.1%-25%. In some embodiments of the present disclosure, based on the total weight of the pharmaceutical composition, the weight proportion of the protective agent may be 1%-20%. In some embodiments of the present disclosure, based on the total weight of the pharmaceutical composition, the weight proportion of the protective agent may be 5%-15%. In some embodiments of the present disclosure, based on the total weight of the pharmaceutical composition, the weight proportion of the protective agent may be 10%-12%. In some embodiments of the present disclosure, the osmotic pressure regulator may be one or two of sodium chloride and potassium chloride. In some embodiments of the present disclosure, the content of the osmotic pressure regulator is such that the osmotic pressure of the pharmaceutical composition is 200-700 milliosmoles/kilogram (mOsmol/kg). In some embodiments of the present disclosure, the content of the osmotic pressure regulator is such that the osmotic pressure of the pharmaceutical composition is 300-600 milliosmoles/kilogram. In some embodiments of the present disclosure, the content of the osmotic pressure regulator is such the osmotic pressure of the pharmaceutical composition is 400-500 millios-moles/kilogram. According to the required osmotic pressure, the skilled in the art can easily determine the content of the osmotic pressure regulator.

In some embodiments of the present disclosure, the pharmaceutical composition may be a liquid preparation, such as an injection. In some embodiments of the present disclosure, the pharmaceutical composition may also be a lyophilized powder for injection, which is mixed with a liquid excipient to prepare a liquid preparation when being administered. In some embodiments of the present disclosure, the liquid preparation may include, but is not limited to, those for administration via subcutaneous, intramuscular or intravenous injection. In some embodiments of the present disclosure, the liquid preparation may include, but is not limited to, being administered to the lungs by aerosol, or being administered to other organ tissues (such as the liver) through the lungs by aerosol. In some embodiments of the present disclosure, the pharmaceutical composition is used for administration via intravenous injection.

4. Kits of the present disclosure

The kit of the present disclosure comprises at least one of the siRNA, the siRNA conjugate or the pharmaceutical composition according to any embodiment of the present disclosure.

Wherein, in cases where multiple siRNAs are present, each siRNA may exist individually or in the form of a mixture of two or more.

In some embodiments of the present disclosure, in addition to the siRNA, siRNA conjugate or pharmaceutical composition, the kit may also comprise components necessary or beneficial for achieving one or more specific applications of the present disclosure. Such components include, but are not limited to: (1) one or more components for achieving the desired cell transfection; (2) one or more components for achieving the diagnosis, treatment or prevention of a specific disease, such as one or more additional therapeutic compounds or compositions, one or more diagnostic agents; (3) one or more buffers; (4) positive or negative control samples; (5) at least one of excipients, stabilizers or preservatives. In some embodiments of the present disclosure, the kit may also comprise instructions for use.

In some embodiments of the present disclosure, the components for achieving the desired cell transfection include viruses (such as adenovirus, lentivirus, adeno-associated virus, retrovirus, herpes simplex virus) or virus-like particles (VLPs), etc.

In some embodiments of the present disclosure, the siRNA, siRNA conjugate or pharmaceutical composition in the kit may be provided in any form, such as liquid form, dried form or lyophilized form. In some embodiments of the present disclosure, the siRNA, siRNA conjugate or pharmaceutical composition in the kit is substantially pure and/or sterile.

5. Use of the siRNAs, siRNA conjugates and pharmaceutical compositions of the present disclosure

By administering to a subject in need thereof at least one of the siRNAs, siRNA conjugates and pharmaceutical compositions of the present disclosure, the expression level of the AGT gene can be effectively reduced in the subject, thereby being expected to be used in the prevention and/or treatment diseases associated with angiotensinogen dysregulation in the subject, or used to prepare a drug for preventing and/or treating diseases associated with angiotensinogen dysregulation.

In the method of preventing and/or treating diseases associated with angiotensinogen dysregulation of the present disclosure, an effective dosage of the siRNA, siRNA conjugate or pharmaceutical composition described in the present disclosure is administered to a subject suffering from diseases associated with angiotensinogen dysregulation or a subject at risk of suffering from diseases associated with angiotensinogen dysregulation. In one embodiment, the subject is a human.

In one embodiment, the diseases associated with angiotensinogen dysregulation are selected from hypertension or related conditions (e.g., borderline hypertension, essential hypertension, secondary hypertension, hypertensive crisis, hypertensive urgency, isolated systolic and diastolic hypertension, pregnancy-related hypertension, diabetic

hypertension, resistant hypertension, refractory hypertension, paroxysmal hypertension, renovascular hypertension, Goldblatt's hypertension, ocular hypertension, pulmonary hypertension, portal hypertension, systemic venous hypertension, systolic hypertension, labile hypertension, hypertensive heart disease, and hypertensive nephropathy).

In another embodiment, the diseases associated with angiotensinogen dysregulation are selected from hypertension, hypertensive heart disease, and hypertensive nephropathy.

Based on the average of properly measured sitting blood pressure readings during two or more clinic visits, a subject with normal blood pressure is a subject with a systolic pressure of about 90-119 mmHg (about 12-15.9 kPa ($kN/m^2$)) and a diastolic pressure of about 60-79 mmHg (about 8.0-10.5 kPa ($kN/m^2$)); a subject with prehypertension is a subject with a systolic pressure of about 120-139 mmHg (about 16.1-18.5 kPa ($kN/m^2$)) and a diastolic pressure of about 60-79 mmHg (about 8.0-10.5 kPa ($kN/m^2$)); a subject with hypertension (e.g., stage I hypertension) is a subject with a systolic pressure of about 140-159 mmHg (about 18.7-21.2 kPa ($kN/m^2$)) and a diastolic pressure of about 90-99 mmHg (about 12.0-13.2 kPa ($kN/m^2$)); a subject with hypertension (e.g., stage II hypertension) is a subject with a systolic pressure of about ≥160 mmHg (about ≥21.3 kPa ($kN/m^2$)) and a diastolic pressure of about ≥100 mmHg (about ≥13.3 kPa ($kN/m^2$)). A subject with a blood pressure of greater than 130/80 mmHg together with type 1 or type 2 diabetes or nephropathy is considered to be suffering from hypertension.

In one embodiment, the disease associated with angiotensinogen dysregulation is essential hypertension. "Essential hypertension" is the result of environmental or genetic factors (e.g., the result without apparent underlying medical cause).

In one embodiment, the disease associated with angiotensinogen dysregulation is secondary hypertension. "Secondary hypertension" has an identifiable underlying disorder that can be of multiple etiologies, including renal, vascular, and endocrine causes, for example, renal parenchymal diseases (e.g., polycystic kidney disease, glomerular or interstitial diseases), renal vascular diseases (e.g., renal artery stenosis, fibromuscular dysplasia), endocrine disorders (e.g., excessive adrenocortical hormones or mineralocorticoids, pheochromocytoma, hyperthyroidism or hypothyroidism, excessive growth hormone, hyperparathyroidism), aortic coarctation, or use of oral contraceptive.

In one embodiment, the disease associated with angiotensinogen dysregulation is hypertensive crisis, such as malignant hypertension and accelerated hypertension. "Accelerated hypertension" is a severely elevated blood pressure (i.e., a systolic pressure equal to or higher than 180 mmHg, or a diastolic pressure equal to or higher than 110 mmHg) with direct damage to one or more end organs, and blood pressure must be immediately lowered to prevent further organ damage. "Malignant hypertension" is a severely elevated blood pressure (a systolic pressure equal to or higher than 180 mmHg or a diastolic pressure equal to or higher than 110 mmHg) with direct damage to one or more end organs and papilledema, and blood pressure must be immediately lowered to prevent further organ damage. End-organ damage of nervous system caused by uncontrolled blood pressure may include hypertensive encephalopathy, cerebrovascular accident/cerebral infarction; subarachnoid hemorrhage, and/or intracranial hemorrhage. Cardiovascular end-organ damage may include myocardial ischemia/infarction, acute left ventricular dysfunction, acute pulmonary edema and/or aortic dissection. Other organ systems may also be affected by uncontrolled hypertension, which may lead to acute renal failure/renal insufficiency, retinopathy, eclampsia, or microangiopathic hemolytic anemia.

In one embodiment, the disease associated with angiotensinogen dysregulation is hypertensive urgency. "Hypertensive urgency" is severely elevated blood pressure (i.e., a systolic pressure equal to or higher than 180 mmHg or a diastolic pressure equal to or higher than 110 mmHg) without direct damage to one or more organs. Blood pressure can be safely lowered within a few hours.

In one embodiment, the disease associated with angiotensinogen dysregulation is resistant hypertension. "Resistant hypertension" is blood pressure that remains above the target level (e.g., 140/90 mmHg) despite the simultaneous use of three different kinds of antihypertensive drugs (one of which is a thiazide diuretic). Subjects whose blood pressure is controlled with four or more drugs are also considered to be suffering from resistant hypertension.

The term "administration/administering" as used herein refers to placing siRNA into a subject's body by a method or route that at least partially locates the siRNA at a desired site to produce a desired effect. Routes of administration suitable for the methods of the present disclosure include local administration and systemic administration. In general, local administration results in more siRNAs being delivered to a specific site compared to the entire body of the subject; systemic administration results in the siRNA being delivered to substantially the entire body of the subject. Given that AGT is primarily expressed in the liver, an administration mode that is able to specifically deliver the siRNA to the liver is preferred.

The administration may be performed to the subject by any suitable route known in the art. In some embodiments of the present disclosure, the route includes, but is not limited to, oral or parenteral routes, including at least one of intravenous administration, intramuscular administration, subcutaneous administration, transdermal administration, airway administration (aerosol), pulmonary administration, nasal administration, rectal administration, and topical administration (including buccal administration and sublingual administration). In some embodiments of the present disclosure, the administration frequency may be once or more per day, per week, per month, or per year. The dosage of the siRNA herein may be determined according to various parameters, particularly the age, weight, and gender of the subject.

Toxicity and efficacy may be determined by standard pharmaceutical procedures in cell culture or experimental animals, such as determining $LD_{50}$ (a dosage that is lethal to 50% of the population) and $ED_{50}$ (a dosage that causes 50% of the maximum response intensity in quantitative reactions, and a dosage that causes the occurrence of a positive response in 50% of the test subjects in qualitative reactions). The dosage ratio between toxicity and efficacy is a therapeutic index, which can be expressed as the ratio of $LD_{50}/ED_{50}$. The siRNAs, siRNA conjugates, or pharmaceutical compositions that exhibit a high therapeutic index are preferred. The dosage ranges for human use can be derived based on data obtained from cell culture assays and animal studies, which is within the capability of the skilled in the art.

When the siRNAs, siRNA conjugates or pharmaceutical compositions according to the present disclosure being administered, for example, for male or female C57BL/6J or C3H/HeNCrlVr mice aged 6-12 weeks and weighing 18-25 g, in the amount of siRNA, the dosage can be 0.001-50 mg/kg body weight, preferably 0.01-10 mg/kg body weight, more preferably 0.05-5 mg/kg body weight, most preferably 0.1-3 mg/kg body weight.

In some embodiments of the present disclosure, the siRNAs, siRNA conjugates and/or pharmaceutical compositions of the present disclosure are used as the sole active ingredient for preventing and/or treating the diseases associated with angiotensinogen dysregulation. In some embodiments of the present disclosure, the siRNAs, siRNA conjugates and/or pharmaceutical compositions of the present disclosure are used together with other active ingredients for preventing and/or treating the diseases associated with angiotensinogen dysregulation.

It should be understood by the skilled in the art that the above description of the present disclosure is exemplary, and the embodiments disclosed herein can be easily modified into other specific forms without changing the technical spirit or essential characteristics of the present disclosure. Therefore, it should be understood that the embodiments described above are exemplary in all aspects and not restrictive.

Unless otherwise specifically stated, singular terms encompass plural terms, and plural terms encompass singular terms. Unless otherwise specifically stated, the word "a" or "an" can encompass "at least one" or "at least one of". Unless otherwise stated, the use of "or" means "and/or".

For the purpose of description and disclosure, all patents, patent applications and other identified publications are expressly incorporated herein by reference. These publications are provided solely because their disclosure antedates the filing date of the present application. All statements regarding the dates of these documents or the representations of the contents of these documents are based on the information available to the applicant and do not constitute any admission as to the correctness of the dates of these documents or the contents of these documents. Furthermore, in any country, any reference to these publications herein does not constitute an acknowledgment that the publications have become part of the common knowledge in the art.

## Examples

Hereinafter, exemplary examples will be provided to help understand the present disclosure. However, the following examples are provided only to make the present disclosure easier to understand and are not intended to limit the present disclosure. Unless otherwise specified, the materials and reagents used in the examples are all commercially available products. Operations such as nucleic acid electrophoresis and real-time PCR used are all performed according to conventional protocols. For example, they can be performed as described in Molecular Cloning (Cold Spring Harbor Laboratory Press (1989)).

Example 1: siRNA synthesis

All the siRNAs used in this example are commercially available or can be synthesized. The specific synthesis method is as follows.

For the sense strand and antisense strand of the siRNA duplexes of the present disclosure, standard CPG was used as the solid-phase support, for the sense strand and antisense strand of the modified duplexes, universal CPG was used as the solid-phase support, and for siRNAs with GalNAc linked to the 3' end of the sense strand, GalNAc was linked to a long-chain amino CPG support and then chain extension was performed according to the standard phosphoramidite method.

Sequence synthesis was performed using a high-throughput synthesizer. The phosphoramidite monomers were used at a concentration of 0.05 M, and the activator used was 0.3 M BTT.

The synthesized sequence was cleaved and deprotected using the solvent of ammonia water and methylamine ethanol (in a volume ratio of 1:1), and then triethylamine trihydrofluoride was used to remove the 2'-position protecting group. For sequences comprising modifications at all of 2'-positions, ammonolysis with ammonia water was required. The cleaved and deprotected sequences were precipitated using a mixture of acetone-ethanol (in a volume ratio of 80:20) and dissolved in RNase-free water. Each sequence was analyzed by LC-MS to determine sequence accuracy, quantified by a spectrophotometer, and purity was determined by HPLC.

After HPLC purification, lyophilization and quality testing, salt exchange was performed by ethanol precipitation

with sodium acetate, desalting was performed using a 3 KD ultrafiltration tube. After desalting, the sense strands and antisense strands were quantified and determined by a spectrophotometer. The sense strands and antisense strands were mixed at a molar ratio of 1:1 and annealed to form the siRNA duplexes and conjugates of the present disclosure (i.e., duplexes 1 to 27, modified duplex 46 to modified duplex 50, modified duplex 60 to modified duplex 125, modified duplex 129 to modified duplex 130, modified duplex 133 to modified duplex 149, modified duplex 152 to modified duplex 201, conjugate 46 to conjugate 50, conjugate 60 to conjugate 125, conjugate 129 to conjugate 130, conjugate 133 to conjugate 136, conjugate 150 to conjugate 151, and conjugate 155 to conjugate 204).

Example 2: *In vitro* activity testing

Cell culture: Hep3B cells were cultured in DMEM complete medium (Gibco, supplemented with 10% FBS and 1% penicillin-streptomycin) in an environment of 37°C and 5% $CO_2$ until nearly confluent, and then cells digested with trypsin were used for plating. 1.0×$10^5$ Hep3B cells and 1.0 mL DMEM complete medium were added to each well of a 24-well plate, and cultured in an environment of 37°C and 5% $CO_2$ for 16-24 hours before transfection.

Cell transfection: 3 μL lipofectamineRNAiMax (Invitrogen) was added to 47 μL opti-MEM (Gibco) in each well, then 50 μL of the opt-MEM dilution of each siRNA or conjugate prepared in Example 1 was added to mix, the resulted mixture was added to a centrifuge tube and incubated at room temperature for 5 min, and finally the obtained siRNA transfection complex was added to the above-mentioned cells, and RNA was extracted after the cells were further cultured for 24 h. Single-dose experiments were performed at 1 nM and 0.1 nM siRNA duplex or conjugate concentrations. $IC_{50}$ test experiments were performed at 10 nM, 1.0 nM, 0.1 nM, 0.01 nM, 0.001 nM, 0.0001 nM and 0.00001 nM siRNA duplex or conjugate concentrations.

RNA extraction: it was performed using the total RNA isolation kit (VAZYME, Cat: RC112-01) according to the instructions of this RNA isolation kit. Finally, 50 μL of RNase-free water was added, allowed to stand for 5 min, then centrifuged at 12000 rpm for 1 min, and the RNA was eluted.

cDNA synthesis: cDNA was synthesized using the cDNA synthesis kit with gDNA wiper (VAZYME, Cat: RC333-01). For each sample, 400 ng of total RNA was added, and cDNA synthesis was performed using a gradient thermal cycler (Bio-rad, T100) according to the steps in the instrument and kit instructions.

Real-time fluorescence quantitative PCR: A 20 μL amplification system was used. The synthesized cDNA and mixed master solution (comprising primers, 1×SYBR Green premix and ultrapure water) were added to a 96-well plate (Thermofisher, Cat: A36924), so that the final real-time fluorescence quantitative PCR system comprised 0.4 μM of each of upstream and downstream primers of the target gene (hAGT, the sequence thereof is set forth in SEQ ID NO: 1) or the internal reference gene (GAPDH) and 10 μL of 1×SYBR Green premix (Thermofisher, Cat: A25742). Real-time fluorescence PCR was performed in the ABI QuantStudio 1 Plus real-time fluorescence PCR system using the ΔΔCt determination method. Each duplex or conjugate was subjected to three independent transfection tests, and each transfection was assayed in triplicate.

Results

The results of *in vitro* activity tests of some duplexes are shown in Table 2, and the results of *in vitro* activity tests of some modified duplexes and conjugates are shown in Tables 3 to 9. Among others, for the *in vitro* activity tests of duplexes, modified duplexes and conjugates, PC A, PC B and PC C, which are known to have AGT gene inhibitory effects, were used as positive controls, respectively. PC A is the duplex AD-85481 in WO2019222166A1, PC B is the modified duplex AD-85481, PC C is the conjugate of the modified duplex AD-85481 and GalNAc (L96). The test results were expressed as mean ± SD.

PC A is:

Sense strand: 5'-GUCAUCCACAAUGAGAGUACA-3' (SEQ ID NO: 391)
Antisense strand: 5'-UGUACUCUCAUUGUGGAUGACGA-3' (SEQ ID NO: 392);

PC B is:

Sense strand: 5'-mG*mU*mCmAmUmCi2FCmAi2FCi2FAi2FAmUmGmAmGmAmGmU mAmCmA-3' (SEQ ID NO: 393);
Antisense strand: 5'-mU*i2FG*mUmAmCTmCmUmCmAmUmUmGi2FUmGi2FGmAm UmGmAmC*mG*mA-3' (SEQ ID NO: 394);

PC C is:

Sense strand: 5'-mG*mU*mCmAmUmCi2FCmAi2FCi2FAi2FAmUmGmAmGmAmGmU mAmCmAGalNAc(L96)-3' (SEQ ID NO: 395);
Antisense strand: 5'-mU*i2FG*mUmAmCTmCmUmCmAmUmUmGi2FUmGi2FGmAm UmGmAmC*mG*mA-3' (SEQ ID NO: 394).

Table 2. Single-dose test results of unmodified duplex

| Duplex No. | InM siRNA | | 0.1nM siRNA | |
|---|---|---|---|---|
| | Mean | SD | Mean | SD |
| Duplex 1 | 0.089 | 0.0135 | 0.197 | 0.0006 |
| Duplex 2 | 0.141 | 0.0120 | 0.209 | 0.0182 |
| Duplex 3 | 0.145 | 0.0192 | 0.200 | 0.0189 |
| Duplex 4 | 0.147 | 0.0333 | 0.258 | 0.0209 |
| Duplex 5 | 0.085 | 0.0071 | 0.305 | 0.0403 |
| Duplex 6 | 0.135 | 0.0286 | 0.258 | 0.0288 |
| Duplex 7 | 0.056 | 0.0103 | 0.251 | 0.0353 |
| Duplex 8 | 0.085 | 0.0107 | 0.253 | 0.0420 |
| Duplex 9 | 0.123 | 0.0129 | 0.225 | 0.0064 |
| Duplex 10 | 0.100 | 0.0604 | 0.186 | 0.0222 |
| Duplex 13 | 0.103 | 0.0266 | 0.263 | 0.0234 |
| PC A | 0.132 | 0.0285 | 0.218 | 0.0274 |

Table 3. Single-dose test results of modified duplexes

| Duplex No. | 1nM siRNA | | 0.1nM siRNA | |
|---|---|---|---|---|
| | Mean | SD | Mean | SD |
| Modified Duplex 61 | 0.474 | 0.0735 | 0.768 | 0.0679 |
| Modified Duplex 62 | 0.323 | 0.0501 | 0.607 | 0.0020 |
| Modified Duplex 63 | 0.473 | 0.0316 | 0.793 | 0.0432 |
| Modified Duplex 66 | 0.294 | 0.0217 | 0.533 | 0.0585 |
| Modified Duplex 67 | 0.292 | 0.0320 | 0.670 | 0.0792 |
| Modified Duplex 68 | 0.428 | 0.0305 | 0.868 | 0.0701 |
| Modified Duplex 69 | 0.204 | 0.0434 | 0.448 | 0.0239 |
| Modified Duplex 70 | 0.302 | 0.0275 | 0.763 | 0.0381 |
| Modified Duplex 64 | 0.347 | 0.0669 | 0.565 | 0.0316 |
| Modified Duplex 46 | 0.365 | 0.0473 | 0.710 | 0.0430 |
| PC B | 0.232 | 0.0353 | 0.381 | 0.0273 |

Table 4. Single-dose test results of modified duplexes

| Duplex No. | InM siRNA | | 0.1nM siRNA | |
|---|---|---|---|---|
| | Mean | SD | Mean | SD |
| Modified Duplex 71 | 0.428 | 0.069 | 0.640 | 0.050 |
| Modified Duplex 72 | 0.439 | 0.059 | 0.641 | 0.079 |
| Modified Duplex 73 | 0.528 | 0.100 | 0.953 | 0.075 |

(continued)

| Duplex No. | InM siRNA | | 0.1nM siRNA | |
|---|---|---|---|---|
| | Mean | SD | Mean | SD |
| Modified Duplex 74 | 0.511 | 0.140 | 0.701 | 0.119 |
| Modified Duplex 75 | 0.402 | 0.079 | 0.708 | 0.062 |
| Modified Duplex 76 | 0.359 | 0.085 | 0.581 | 0.078 |
| Modified Duplex 77 | 0.444 | 0.052 | 0.719 | 0.032 |
| Modified Duplex 78 | 0.401 | 0.034 | 0.635 | 0.092 |
| Modified Duplex 79 | 0.480 | 0.110 | 0.556 | 0.022 |
| Modified Duplex 80 | 0.499 | 0.033 | 0.519 | 0.017 |
| Modified Duplex 81 | 0.482 | 0.132 | 0.745 | 0.166 |
| Modified Duplex 82 | 0.499 | 0.053 | 0.508 | 0.064 |
| Modified Duplex 83 | 0.466 | 0.040 | 0.611 | 0.058 |
| Modified Duplex 84 | 0.463 | 0.097 | 0.547 | 0.018 |
| Modified Duplex 85 | 0.512 | 0.050 | 0.720 | 0.070 |
| Modified Duplex 86 | 0.462 | 0.071 | 0.522 | 0.034 |
| Modified Duplex 87 | 0.585 | 0..061 | 0.595 | 0.076 |
| Modified Duplex 88 | 0.376 | 0.030 | 0.506 | 0.057 |
| Modified Duplex 69 | 0.483 | 0.092 | 0.679 | 0.081 |
| PC B | 0.366 | 0.063 | 0.530 | 0.083 |

Table 5. Single-dose test results of modified duplexes

| Duplex No. | 1nM siRNA | | 0.1nM siRNA | |
|---|---|---|---|---|
| | Mean | SD | Mean | SD |
| Modified Duplex 89 | 0.295 | 0.0004 | 0.458 | 0.0014 |
| Modified Duplex 90 | 0.283 | 0.0005 | 0.581 | 0.0008 |
| Modified Duplex 91 | 0.289 | 0.0006 | 0.470 | 0.0002 |
| Modified Duplex 92 | 0.298 | 0.0006 | 0.508 | 0.0012 |
| Modified Duplex 93 | 0.248 | 0.0003 | 0.489 | 0.0005 |
| Modified Duplex 94 | 0.228 | 0.0007 | 0.445 | 0.0006 |
| Modified Duplex 95 | 0.239 | 0.0003 | 0.571 | 0.0007 |
| Modified Duplex 96 | 0.262 | 0.0006 | 0.524 | 0.0007 |
| Modified Duplex 97 | 0.288 | 0.0005 | 0.545 | 0.0008 |
| Modified Duplex 98 | 0.252 | 0.0005 | 0.564 | 0.0008 |
| Modified Duplex 99 | 0.203 | 0.0002 | 0.655 | 0.0002 |
| Modified Duplex 100 | 0.171 | 0.0004 | 0.586 | 0.0005 |
| PC B | 0.147 | 0.0005 | 0.306 | 0.0003 |

Table 6. Single-dose test results of modified duplexes

| Duplex No. | 1nM siRNA | | 0.1nM siRNA | |
|---|---|---|---|---|
| | Mean | SD | Mean | SD |
| Modified Duplex 76 | 0.243 | 0.025 | 0.398 | 0.018 |

(continued)

| Duplex No. | 1nM siRNA | | 0.1nM siRNA | |
|---|---|---|---|---|
| | Mean | SD | Mean | SD |
| **Modified Duplex 88** | 0.286 | 0.051 | 0.368 | 0.040 |
| **Modified Duplex 101** | 0.175 | 0.011 | 0.339 | 0.015 |
| **Modified Duplex 102** | 0.170 | 0.019 | 0.315 | 0.051 |
| **Modified Duplex 103** | 0.180 | 0.015 | 0.291 | 0.043 |
| **Modified Duplex 104** | 0.207 | 0.026 | 0.465 | 0.045 |
| **Modified Duplex 105** | 0.216 | 0.056 | 0.374 | 0.040 |
| **Modified Duplex 106** | 0.303 | 0.064 | 0.433 | 0.004 |
| **Modified Duplex 107** | 0.260 | 0.023 | 0.418 | 0.020 |
| **Modified Duplex 108** | 0.219 | 0.039 | 0.293 | 0.014 |
| **Modified Duplex 109** | 0.226 | 0.073 | 0.343 | 0.013 |
| **Modified Duplex 110** | 0.236 | 0.018 | 0.296 | 0.016 |
| **Modified Duplex 111** | 0.344 | 0.021 | 0.299 | 0.052 |
| **Modified Duplex 112** | 0.300 | 0.054 | 0.661 | 0.043 |
| **Modified Duplex 113** | 0.247 | 0.063 | 0.449 | 0.019 |
| **Modified Duplex 114** | 0.221 | 0.100 | 0.495 | 0.029 |
| **Modified Duplex 115** | 0.353 | 0.018 | 0.565 | 0.006 |
| **Modified Duplex 116** | 0.298 | 0.042 | 0.286 | 0.044 |
| **PC B** | 0.224 | 0.018 | 0.306 | 0.036 |

It is found in the *in vitro* tests that modified duplexes with mismatches, preferably modified duplexes in which the nucleotides at position 19, 20 or 21 in the direction from 5' end to 3' end of the antisense strand were mismatched with the target sequence (e.g., modified duplexes 101-103), had significantly improved *in vitro* activities compared to duplexes in which the above-mentioned positions were not mismatched with the target sequence (e.g., modified duplex 76).

Table 7. Single-dose test results of conjugates

| Duplex No. | 1nM siRNA | | 0.1nM siRNA | |
|---|---|---|---|---|
| | Mean | SD | Mean | SD |
| **Conjugate 110** | 0.256 | 0.022 | 0.399 | 0.064 |
| **Conjugate 117** | 0.399 | 0.064 | 0.631 | 0.002 |
| **Conjugate 118** | 0.252 | 0.061 | 0.613 | 0.170 |
| **Conjugate 119** | 0.173 | 0.066 | 0.397 | 0.012 |
| **Conjugate 120** | 0.270 | 0.027 | 0.717 | 0.129 |
| **Conjugate 121** | 0.773 | 0.096 | 1.138 | 0.140 |
| **Conjugate 122** | 0.496 | 0.112 | 0.867 | 0.127 |
| **Conjugate 123** | 0.704 | 0.124 | 0.851 | 0.187 |
| **Conjugate 101** | 0.232 | 0.015 | 0.416 | 0.115 |
| **Conjugate 102** | 0.197 | 0.013 | 0.402 | 0.061 |
| **Conjugate 103** | 0.222 | 0.010 | 0.482 | 0.068 |
| **Conjugate 124** | 0.252 | 0.104 | 0.472 | 0.113 |
| **Conjugate 125** | 0.686 | 0.120 | 0.799 | 0.082 |

(continued)

| Duplex No. | 1nM siRNA | | 0.1nM siRNA | |
|---|---|---|---|---|
| | Mean | SD | Mean | SD |
| Conjugate 129 | 0.303 | 0.088 | 0.594 | 0.090 |
| Conjugate 130 | 0.230 | 0.025 | 0.403 | 0.037 |
| Conjugate 133 | 0.284 | 0.063 | 0.793 | 0.053 |
| Conjugate 134 | 0.278 | 0.045 | 0.623 | 0.095 |
| Conjugate 135 | 0.332 | 0.065 | 0.613 | 0.042 |
| Conjugate 136 | 0.508 | 0.032 | 0.929 | 0.062 |
| PC C | 0.192 | 0.032 | 0.446 | 0.137 |

Table 8. Single-dose test results of conjugates

| Duplex No. | 1nM siRNA | | 0.1nM siRNA | |
|---|---|---|---|---|
| | Mean | SD | Mean | SD |
| Conjugate 155 | 0.221 | 0.0230 | 0.376 | 0.0303 |
| Conjugate 156 | 0.158 | 0.0111 | 0.285 | 0.0119 |
| Conjugate 157 | 0.132 | 0.0340 | 0.218 | 0.0379 |
| Conjugate 162 | 0.153 | 0.0170 | 0.240 | 0.0198 |
| Conjugate 166 | 0.154 | 0.0114 | 0.312 | 0.0257 |
| Conjugate 171 | 0.184 | 0.0161 | 0.273 | 0.0099 |
| Conjugate 172 | 0.138 | 0.0503 | 0.237 | 0.0286 |
| Conjugate 175 | 0.115 | 0.0304 | 0.149 | 0.0295 |
| Conjugate 181 | 0.173 | 0.0283 | 0.221 | 0.0072 |
| Conjugate 182 | 0.131 | 0.0021 | 0.252 | 0.0489 |
| Conjugate 183 | 0.162 | 0.0388 | 0.216 | 0.0236 |
| Conjugate 184 | 0.147 | 0.0153 | 0.220 | 0.0123 |
| Conjugate 185 | 0.160 | 0.0090 | 0.226 | 0.0236 |
| Conjugate 186 | 0.313 | 0.0162 | 0.417 | 0.0292 |
| Conjugate 187 | 0.230 | 0.1013 | 0.239 | 0.0652 |
| Conjugate 188 | 0.209 | 0.0780 | 0.219 | 0.0870 |
| Conjugate 189 | 0.249 | 0.0201 | 0.246 | 0.0296 |
| Conjugate 190 | 0.309 | 0.0406 | 0.342 | 0.0526 |
| Conjugate 191 | 0.292 | 0.1159 | 0.264 | 0.0137 |
| Conjugate 192 | 0.333 | 0.0879 | 0.380 | 0.0775 |
| Conjugate 193 | 0.368 | 0.0218 | 0.433 | 0.0156 |
| Conjugate 194 | 0.323 | 0.0486 | 0.417 | 0.0674 |
| Conjugate 195 | 0.298 | 0.0552 | 0.323 | 0.0655 |
| Conjugate 196 | 0.283 | 0.0647 | 0.411 | 0.2027 |
| Conjugate 197 | 0.352 | 0.0288 | 0.384 | 0.0143 |
| Conjugate 198 | 0.264 | 0.0327 | 0.345 | 0.0170 |
| PC C | 0.246 | 0.0380 | 0.494 | 0.0506 |

Table 9. *In vitro* $IC_{50}$ test results of conjugate 182

| Duplex No. | administration dosages | Mean | SD |
|---|---|---|---|
| Conjugate 182 | 10 nM | 0.060 | 0.012 |
| | 1.0 nM | 0.139 | 0.075 |
| | 0.1 nM | 0.183 | 0.087 |
| | 0.01 nM | 0.725 | 0.046 |
| | 0.001 nM | 0.937 | 0.081 |
| | 0.0001 nM | 1.004 | 0.029 |
| | 0.00001 nM | 0.982 | 0.080 |

The results in Table 9 shown that the nonlinear fitting degree of the $IC_{50}$ curve for conjugate 182 was good, with an $IC_{50}$ value of 0.01975 nM and an R squared value of 0.9734.

Example 3: Free uptake by primary cynomolgus monkey hepatocyte (PCH)

Thawing of primary cynomolgus monkey hepatocytes: 1 mL of primary cynomolgus monkey hepatocytes was added to 19 mL of culture medium and gently inverted to mix, centrifuged at 50×g for 5 minutes. After discarding the supernatant, the cells were resuspended in 10 mL of the culture medium.

Administration: The diluted siRNA conjugate was added to a 96-well collagen-coated plate at a volume of 10 μL per well (administration group), and PCHs were seeded into the 96-well plate in $5.4\times10^4$ cells per well, and a nuclease-free water control group without siRNA conjugate was set up at the same time. Thereafter, the plate was placed in a 37°C, 5% $CO_2$ incubator for incubation.

RNA extraction and reverse transcription: After 48 hours of free uptake, the culture medium was removed and the cells were collected for RNA extraction. Total RNA was extracted using the RNeasy® 96 Kit (QIAGEN-74182) according to the kit instructions. Then, cDNA synthesis was performed using the cDNA synthesis kit with gDNA wiper (VAZYME, Cat: RC333-01) according to the instructions. For each sample, 400 ng of total RNA was added, and cDNA synthesis was performed using a gradient thermal cycler (Bio-rad, T100) according to the instructions.

qPCR detection of target gene mRNA expression levels: The synthesized cDNA and mixed master solution (comprising primers, 1×SYBR Green premix and ultrapure water) were added to a 96-well plate (Thermofisher, Cat: A36924), so that the final real-time fluorescence quantitative PCR system comprised the target gene (hAGT) or internal reference gene (GADPH), 0.4 μM of each of upstream and downstream primers, 1×SYBR Green premix (Thermofisher, Cat: A25742).

Data analysis: The mRNA expression level of the target gene AGT in the sample was calculated based on the CT value of each sample, and the calculation was performed by the ΔΔCT relative quantitative method. The relative expression amount of the target gene was expressed as 2-ΔΔCT.

The calculation formulas are as follows:

ΔCT = average CT value of target gene - average CT value of internal reference gene ΔΔCT = ΔCT (administration group) - ΔCT (nuclease-free water control group)

$$\text{Relative expression amount of target gene mRNA} = 2\text{-}\Delta\Delta CT$$

Inhibition rate % = (1-relative expression amount of administration group/average expression amount of nuclease-free water control group) × 100

The *in vitro* activity test results of the conjugates are shown in Table 10, and the test results are expressed as mean ± SD.

Table 10. Test results of free uptake of siRNA conjugates by primary cynomolgus monkey hepatocytes

| Duplex No. | 10nM siRNA | | 1nM siRNA | |
|---|---|---|---|---|
| | Mean | SD | Mean | SD |
| **PC C** | 8.12% | 0.460 | 23.32% | 0.784 |
| **Conjugate 155** | 5.73% | 0.240 | 15.80% | 0.588 |
| **Conjugate 156** | 4.46% | 0.343 | 13.14% | 0.610 |
| **Conjugate 166** | 20.26% | 0.765 | 60.49% | 1.489 |
| **Conjugate 150** | 5.01% | 0.517 | 15.25% | 1.092 |
| **Conjugate 157** | 6.26% | 0.937 | 19.37% | 1.257 |
| **Conjugate 162** | 7.39% | 0.974 | 20.73% | 1.437 |
| **Conjugate 182** | 6.35% | 0.606 | 13.90% | 0.726 |
| **Conjugate 183** | 7.17% | 0.407 | 16.69% | 1.523 |
| **Conjugate 175** | 28.98% | 1.380 | 47.07% | 1.721 |
| **Conjugate 172** | 5.57% | 0.515 | 12.26% | 2.789 |
| **Conjugate 171** | 10.38% | 0.824 | 20.33% | 2.216 |
| **Conjugate 185** | 6.35% | 0.310 | 15.83% | 2.364 |
| **Conjugate 151** | \ | \ | 21.58% | 3.430 |
| **Conjugate 181** | \ | \ | 12.51% | 1.730 |
| **Conjugate 184** | \ | \ | 12.19% | 1.070 |

Example 4: Free uptake by primary human hepatocyte (PHH)

Thawing of primary human hepatocytes: 1 mL of primary human hepatocytes was added to 19 mL of culture medium and gently inverted to mix, centrifuged at 50×g for 5 min. After discarding the supernatant, the cells were resuspended in 10 mL of the culture medium.

Administration: The diluted siRNA conjugate was added to a 96-well collagen-coated plate at a volume of 10 μL per well (administration group), and PHHs were seeded into the 96-well plate in $5.4\times10^4$ cells per well, and a nuclease-free water control group without siRNA conjugate was set up at the same time. Thereafter, the plate was placed in a 37°C, 5% $CO_2$ incubator for incubation.

RNA extraction and reverse transcription: After 48 hours of free uptake, the culture medium was removed and the cells were collected for RNA extraction. Total RNA was extracted using the RNeasy® 96 Kit (QIAGEN-74182) according to the kit instructions. Then, cDNA synthesis was performed using the cDNA synthesis kit with gDNA wiper (VAZYME, Cat: RC333-01) according to the instructions. For each sample, 400 ng of total RNA was added, and cDNA synthesis was performed using a gradient thermal cycler (Bio-rad, T100) according to the instructions.

qPCR detection of target gene mRNA expression levels: The synthesized cDNA and mixed master solution (comprising primers, 1×SYBR Green premix and ultrapure water) were added to a 96-well plate (Thermofisher, Cat: A36924), so that the final real-time fluorescence quantitative PCR system comprised the target gene (AGT) or internal reference gene (GADPH), 0.4 μM of each of upstream and downstream primers, 1×SYBR Green premix (Thermofisher, Cat: A25742).

Data analysis: The mRNA expression level of the target gene AGT in the sample was calculated based on the CT value of each sample, and the calculation was performed by the ΔΔCT relative quantitative method. The relative expression amount of the target gene was expressed as 2-ΔΔCT.

The calculation formulas are as follows:

$$\Delta CT = \text{average CT value of target gene - average CT value of internal reference gene}$$

$$\Delta\Delta CT = \Delta CT\ (\text{administration group}) - \Delta CT\ (\text{nuclease-free water control group})$$

$$\text{Relative expression amount of target gene mRNA} = 2\text{-}\Delta\Delta CT$$

Inhibition rate % = (1-relative expression amount of administration group/average expression amount of nuclease-free water control group) × 100

The *in vitro* activity test results of the conjugates are shown in Table 11, and the test results are expressed as mean ± SD.

Table 11. Test results of free uptake of siRNA conjugates by primary human hepatocytes

| Duplex No. | 10nM siRNA | | 1nM siRNA | |
|---|---|---|---|---|
| | Mean | SD | Mean | SD |
| PCC | 11.10% | 0.390 | 38.6% | 2.814 |
| Conjugate 155 | 10.59% | 0.304 | 39.4% | 2.503 |
| Conjugate 156 | 9.23% | 0.339 | 30.2% | 4.727 |
| Conjugate 166 | 49.97% | 0.625 | 94.5% | 7.973 |
| Conjugate 150 | 10.51% | 0.665 | 35.6% | 1.339 |
| Conjugate 157 | 12.48% | 0.752 | 56.2% | 7.993 |
| Conjugate 162 | 11.75% | 0.870 | 39.8% | 0.474 |
| Conjugate 182 | 9.55% | 0.415 | 26.9% | 4.507 |
| Conjugate 183 | 15.51% | 0.959 | 36.8% | 2.562 |
| Conjugate 175 | 54.86% | 0.884 | 72.3% | 8.997 |
| Conjugate 172 | 11.70% | 1.886 | 31.4% | 6.080 |
| Conjugate 171 | 18.10% | 1.423 | 40.7% | 8.303 |
| Conjugate 185 | 15.31% | 1.560 | 44.5% | 5.282 |
| Conjugate 151 | \ | \ | 47.4% | 6.772 |
| Conjugate 181 | \ | \ | 27.2% | 3.715 |
| Conjugate 184 | \ | \ | 25.2% | 3.944 |

It is found in the *in vitro* tests that the *in vitro* activities of multiple conjugates, including conjugate 156 and conjugate 182, were significantly superior to that of the positive control.

Example 5: *In vivo* activity test

(1) *In vivo* silencing of AGT in transgenic mice expressing human AGT:

Transgenic mice expressing human AGT were used. A single subcutaneous administration of different doses of siRNAs to mice was carried out (3 mice per group), and a PBS control group (3 mice per group) was set up at the same time. Blood was sampled 1 day before administration and every 7 days after administration.

After serum was extracted from the blood, the expression level of hAGT protein was detected using the ELISA method. The KD was calculated using the expression amount of hAGT protein 1 day before administration as the baseline value. The test results of the *in vivo* activities of some conjugates in mice are shown in Table 12, and the test results are expressed as mean ± SD.

Table 12. Results of in vivo test in mice

| Conjugate No. | Time | Mean KD of Serum AGT | SD |
|---|---|---|---|
| **Conjugate 155 (1mg/kg, n=3)** | Day -1 | 1.00 | 0.13 |
| | Day 7 | 0.18 | 0.02 |
| | Day 14 | 0.21 | 0.04 |
| **Conjugate 157 (1mg/kg, n=3)** | Day -1 | 1.00 | 0.05 |
| | Day 7 | 0.17 | 0.04 |
| | Day 14 | 0.24 | 0.05 |
| **Conjugate 162 (1mg/kg, n=3)** | Day -1 | 1.00 | 0.03 |
| | Day 7 | 0.17 | 0.01 |
| | Day 14 | 0.22 | 0.01 |
| **Conjugate 171 (1mg/kg, n=3)** | Day -1 | 1.00 | 0.13 |
| | Day 7 | 0.18 | 0.00 |
| | Day 14 | 0.21 | 0.02 |
| **Conjugate 172 (1mg/kg, n=3)** | Day -1 | 1.00 | 0.07 |
| | Day 7 | 0.19 | 0.01 |
| | Day 14 | 0.24 | 0.03 |
| **Conjugate 175 (1mg/kg, n=3)** | Day -1 | 1.00 | 0.02 |
| | Day 7 | 0.21 | 0.02 |
| | Day 14 | 0.27 | 0.01 |
| **Conjugate 182 (1mg/kg, n=3)** | Day -1 | 1.00 | 0.09 |
| | Day 7 | 0.13 | 0.01 |
| | Day 14 | 0.15 | 0.03 |
| **Conjugate 183 (1mg/kg, n=3)** | Day -1 | 1.00 | 0.08 |
| | Day 7 | 0.16 | 0.01 |
| | Day 14 | 0.19 | 0.00 |
| **PC C (1mg/kg, n=3)** | Day -1 | 1.00 | 0.03 |
| | Day 7 | 0.45 | 0.10 |
| | Day 14 | 0.38 | 0.06 |
| **PBS (n=3)** | Day -1 | 1.00 | 0.16 |
| | Day 7 | 0.90 | 0.14 |
| | Day 14 | 0.84 | 0.12 |

(2) Studies on *in vivo* activity and long-term efficacy in cynomolgus monkeys:

Cynomolgus monkeys were used for *in vivo* activity and long-term efficacy testing. A single subcutaneous administration of different doses of siRNA to cynomolgus monkeys was carried out (3 monkeys per group, male). Blood samples were collected on days 1, 6, and 15 before administration and after administration. The expression of hAGT protein of the collected blood samples was detected using the ELISA method. The mean value of the expression amounts of hAGT protein on days 1, 6, and 15 before administration was used as the baseline value. The test results of the *in vivo* activities of some conjugates in cynomolgus monkeys are shown in Table 13, and the test results are expressed as mean ± SD.

Table 13. Results of *in vivo* test in cynomolgus monkeys

| Conjugate No. | Time | Mean KD of Serum AGT | SD |
|---|---|---|---|
| Conjugate 156 (3mg/kg, n=3) | baseline value | 1.00 | 0.04 |
| | Day 4 | 0.90 | 0.13 |
| | Day 8 | 0.43 | 0.07 |
| | Day 14 | 0.19 | 0.03 |
| | Day 22 | 0.14 | 0.03 |
| | Day 28 | 0.12 | 0.03 |
| | Day 36 | 0.13 | 0.03 |
| | Day 42 | 0.18 | 0.04 |
| Conjugate 182 (3mg/kg, n=3) | baseline value | 1.00 | 0.08 |
| | Day 4 | 0.72 | 0.09 |
| | Day 8 | 0.28 | 0.03 |
| | Day 14 | 0.11 | 0.01 |
| | Day 22 | 0.08 | 0.01 |
| | Day 28 | 0.08 | 0.01 |
| | Day 36 | 0.10 | 0.02 |
| | Day 42 | 0.12 | 0.01 |
| Conjugate 182 (6mg/kg, n=3) | baseline value | 1.00 | 0.05 |
| | Day 4 | 0.70 | 0.10 |
| | Day 8 | 0.24 | 0.03 |
| | Day 14 | 0.09 | 0.01 |
| | Day 22 | 0.07 | 0.02 |
| | Day 28 | 0.06 | 0.01 |
| | Day 36 | 0.07 | 0.02 |
| | Day 42 | 0.10 | 0.04 |
| PC C (3mg/kg, n=3) | baseline value | 1.00 | 0.21 |
| | Day 4 | 0.93 | 0.09 |
| | Day 8 | 0.35 | 0.06 |
| | Day 14 | 0.18 | 0.03 |
| | Day 22 | 0.17 | 0.06 |
| | Day 28 | 0.16 | 0.04 |
| | Day 36 | 0.17 | 0.04 |
| | Day 42 | 0.27 | 0.09 |

The results shown that in the *in vivo* tests of monkeys, conjugate 156 and conjugate 182 both shown good KD activities, and the KD activities thereof were significantly superior to that of PC C, and they still exhibited favorable AGT inhibitory effects 42 days after administration.

Example 6: Serum stability in SD rat and human

20 μL of the siRNA to be tested was added to 180 μL serum of SD rat or human and incubated at 37°C for 0 h, 24 h, and 48 h, respectively. After incubation, it was vortexed and mixed thoroughly, and 200 μL of lysis buffer (Phenomenex, Cat: #AL0-8579) was added thereto. The extraction was performed using an SPE column (Phenomenex, Cat: #8B-S103-

EBJ). 5 μL of the extracted product was separated using an Agilent oligonucleotide Waters (100 mm×2.1 mm, 2.7 μm) chromatography column. The residual amounts of the sense strand and antisense strand and the production amount of metabolites were detected by high-resolution mass spectrometry, and 0 h was used as a blank control.

The results shown that PC C and conjugate 155, conjugate 156, conjugate 162, conjugate 182 and conjugate 184 were all stable in the serum of SD rat or human after incubation at 37°C for 48 h.

Example 7: Cytotoxicity test

Cell culture: Hep3B cells were cultured in DMEM complete medium (Gibco, supplemented with 10% FBS and 1% penicillin-streptomycin) in an environment of 37°C and 5% $CO_2$ until nearly confluent, then the cells were digested with trypsin and plated, 7000 Hep3B cells and 0.1 mL DMEM complete medium were added to each well of a 96-well plate, and cultured in an environment of 37°C and 5% $CO_2$ for 16-24 hours before transfection.

Cell transfection: 0.3 μL lipofectamine RNAiMax (Invitrogen) was added to 4.7 μL opti-MEM in each well, and then 5 μL of the opt-MEM dilution of siRNA conjugate was added to mix, the resulted mixture was added to a centrifuge tube and incubated at room temperature for 5 min, and finally the obtained siRNA transfection complex was added to the above-mentioned cells, and cytotoxicity was tested after the cells were further cultured for 24 h, 48 h, or 96 h. This experiment was performed at 100 nM, 10 nM, and 1 nM siRNA conjugate concentrations.

Cytotoxicity test: Cytotoxicity test was performed using CCK-8 kit (Abmle Cat: #M4839-5007ests). After transfection of Hep3B cells for 24 h, 48 h or 96 h according to the above steps, the culture medium was removed, 500 μL of the corresponding complete culture medium (comprising 10% CCK-8) was added to each well, and the plates were incubated at 37°C in the dark for 30 min-60 min. The OD values of the samples were measured using a microplate reader (Tecan Cat: #spark 20M) using a wavelength of 450 nm and a reference wavelength of 620 nm.

The results demonstrated that PC C and conjugate 155, conjugate 156, conjugate 162, conjugate 182 and conjugate 184 all shown no obvious cytotoxicity and had no effect on cell proliferation.

Example 8: siRNA off-target detection

Cell culture: Hep3B cells were cultured in DMEM complete medium (Gibco, supplemented with 10% FBS and 1% penicillin-streptomycin) in an environment of 37°C and 5% $CO_2$ until nearly confluent, then the cells were digested with trypsin and plated, $1.0\times10^5$ Hep3B cells and 1.0 mL DMEM complete medium were added to each well of a 24-well plate, and cultured in an environment of 37°C and 5% $CO_2$ for 16-24 hours before transfection.

Cell transfection: 3 μL lipofectamine RNAiMax (Invitrogen) was added to 47 μL opti-MEM in each well, and then 50 μL of the opt-MEM dilution of siRNA conjugate was added to mix, the resulted mixture was added to a centrifuge tube and incubated at room temperature for 5 min, and finally the obtained siRNA transfection complex was added to the above-mentioned cells, and RNA was extracted after the cells were further cultured for 24 hours. Single-dose experiments were performed at 10 nM and 1 nM siRNA conjugate concentrations.

RNA extraction: Cells were washed with PBS, and then 1 mL Trizol reagent was added thereto, mixed thoroughly on a vortex mixer, and left to stand at room temperature for 5 min. After centrifugation at 12,000×g for 10 min under 4°C, the upper aqueous phase was transferred to a new 1.5 mL EP tube (about 400-500 μL) and left to stand at room temperature for 5 min to ensure sufficient lysis of the tissue cells. For every 1 mL Trizol, 200 μL chloroform was added; the tube cap was tightly closed, and the resulted mixture was vortexed and mixed thoroughly on a vortex mixer, then left to stand at room temperature for 3-5 min to allow natural phase separation. After centrifugation at 12,000×g for 10 min under 4°C, the mixture was separated into three layers: the lower red layer that was the phenol-chloroform organic phase, the middle protein layer and the colorless upper aqueous phase layer, wherein RNA was mainly concentrated in the aqueous phase. The upper aqueous phase layer (about 400-500 μL) was transferred, into which an equal volume of chloroform/isoamyl alcohol (24: 1) was added. After mixing, the mixture was left to stand on ice for 5 min, centrifuged at 12,000×g for 10 min under 4°C. Chloroform/isoamyl alcohol extraction was repeated once until the middle layer was relatively clean. An equal volume of isopropanol was added to the transferred supernatant, and the resulted mixture was placed at -20°C for 1 hour. After centrifugation at 13600 rpm for 20 min under 4°C, the supernatant was discarded. The RNA precipitate was rinsed twice with 1.0 mL of 75% ethanol, allowed to stand for 3 min each time, with inversion for washing during this period, and centrifuged at 13600 rpm for 3 min under 4°C. Residual ethanol was removed, the precipitate was air-dried in a laminar flow hood, and the RNA precipitate was dissolved with 30-50 μL of nuclease-free water.

mRNA sequencing: 1 μg of the extracted total RNA was taken, RNase was removed, the sample was treated with an rRNA kit and a library was constructed. All samples were sequenced using a next-generation sequencer (Nova-Seq6000, Illumina). Sequencing results were compared and analyzed with those of blank samples.

The degree of inhibition of target gene AGT expression was compared with the degree of inhibition of all other mRNAs expressed in Hep3B cells. The results shown that PC C and conjugate 155, conjugate 156, conjugate 162, conjugate 182 and conjugate 184 all had no obvious off-target risk.

Example 9: siRNA immunogenicity test

Cell thawing and plating: One tubule of cryopreserved PBMC cells was taken out from the liquid nitrogen tank and immediately placed in a 37°C water bath to thaw. The cells were transferred into preheated complete medium RF (RPMI 1640+10% FBS, prepared in advance) and mixed well. A small amount of cell suspension was taken out for counting and viability detection using a cell counter and a hemocytometer. Then, RF was supplemented to 14 mL, and the cells in the centrifuge tube were centrifuged at 400×g for 10 min at room temperature. The supernatant was discarded, 2 mL of RF was added thereto, and the cells were gently blown to mix. $8\times10^5$ cells were plated in each well of a 96-well plate with a volume of 0.2 mL per well.

Cell transfection: 0.6 μL per well of transfection reagent (lipofectamine RNAiMax, Invitrogen); 24.4 μL per well of opti-MEM (Invitrogen); the administration dosages of siRNA conjugates were 1 μM, 100 nM, and 10 nM, respectively. 50 μL per well of the above mixed solution was transferred to the above-mentioned 96-well plate for cell culture, which was cultured at 37°C for 24 hours.

Cytokine detection: After 24 hours, the cell culture supernatant was transferred to a new centrifuge tube, centrifuged at 1000×g for 20 min under 2-8°C, and the supernatant was collected. The cytokines were quantified using the hIFN-α and hIL-6 Elisa kit (Elab-science), and the optical density (OD value) of each well was measured at a wavelength of 450 nm using a microplate reader.

The results shown that the effects of PC C and conjugate 155, conjugate 156, conjugate 162, conjugate 182, and conjugate 184 on the expressions of hIFN-α and hIL-6 in PBMC cells had no significant difference.

The applicant has found that the conjugates of the present application (e.g., conjugate 182) exhibit comprehensively excellent druggabilities and are superior to those of PC C in terms of *in vitro activity* in Hep3B cells, *in vitro* activity of free uptake by primary cynomolgus monkey hepatocytes, *in vitro* activity of free uptake by primary human hepatocytes, *in vivo* activities in AGT transgenic mice and cynomolgus monkeys, and *in vitro* stability, etc.

## Claims

1. An siRNA for inhibiting the expression of angiotensinogen, comprising a sense strand and an antisense strand, wherein the antisense strand comprises at least 19 contiguous nucleotides that differ by no more than 3 nucleotides from any one of the antisense strand sequences shown in Table 1.

2. The siRNA of claim 1, wherein the antisense strand comprises a terminal overhang of 1-4 nucleotides in length at the 3' end.

3. The siRNA of claim 1 or 2, wherein the number of nucleotides in the sense strand is 16-25.

4. The siRNA of any one of claims 1-3, wherein the antisense strand has at most four mismatches with a target sequence set forth in SEQ ID NO: 1, preferably the antisense strand has at most two mismatches with the target sequence.

5. The siRNA of any one of claims 1-4, wherein the nucleotides at positions 16 to 23 in the direction from the 5' end to the 3' end of the antisense strand have at most one mismatch with the target sequence, preferably the nucleotide at position 20 in the direction from the 5' end to the 3' end of the antisense strand has a mismatch with the target sequence.

6. The siRNA of any one of claims 1-5, wherein the sense strand has at most one mismatch with the antisense strand, preferably the sense strand has no mismatch with the antisense strand.

7. The siRNA of any one of claims 1-6, the antisense strand of the siRNA differs by no more than 1 nucleotide from any one of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, and 74.

8. The siRNA of any one of claims 1-6, wherein the antisense strand comprises a sequence set forth in any one selected from of SEQ ID NOs: 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 109, 111, 113, 115, 117, 119, 121, 123, 125, 127, 129, 131, 133, 135, 137, 139, 141, and 143.

9. The siRNA of any one of claims 1-8, wherein the siRNA is an unmodified duplex selected from any one of duplex 1 to duplex 27.

**10.** The siRNA of any one of claims 1-9, wherein the siRNA comprises at least one modified nucleotide or nucleotide analog.

**11.** The siRNA of claim 10, wherein all nucleotides in the sense strand and/or antisense strand are modified nucleotides or nucleotide analogs, the modified nucleotides are at least one selected from2'-methoxy nucleotides, 2'-fluoro nucleotides, 2'-deoxy nucleotides, 2'-methoxyethyl nucleotides, 2'-amino nucleotides, 2'-alkyl nucleotides, 3'-methoxy nucleotides, (*E*)-vinylphosphonate modified nucleotides, (Z)-vinylphosphonate modified nucleotides, phosphorothioate modified nucleotides, Sp-configured phosphorothioate modified nucleotides, Rp-configured phosphorothioate modified nucleotides or mesyl phosphoramidate modified nucleotides, and the nucleotide analogs comprise locked nucleic acids, unlocked nucleic acids or glycerol nucleic acids.

**12.** The siRNA of any one of claims 1-11, wherein a portion of adjacent nucleotides in the sense strand and/or antisense strand are linked via phosphorothioate groups, preferably, at least one of linkages between the nucleotide at position 1 and the nucleotide at position 2 at the 5' end of the sense strand, between the nucleotide at position 2 and the nucleotide at position 3 at the 5' end of the sense strand, between the nucleotide at position 1 and the nucleotide at position 2 at the 3' end of the antisense strand, between the nucleotide at position 2 and the nucleotide at position 3 at the 3' end of the antisense strand, between the nucleotide at position 1 and the nucleotide at position 2 at the 5' end of the antisense strand, and between the nucleotide at position 2 and the nucleotide at position 3 at the 5' end of the antisense strand is a phosphorothioate linkage, an Rp-configured phosphorothioate linkage, or an Sp-configured phosphorothioate linkage.

**13.** The siRNA of any one of claims 1-12, wherein four, five, six or seven nucleotides in the antisense strand are 2'-fluoro nucleotides selected from the nucleotides at positions 2, 3, 4, 5, 6, 7, 8, 9, 10, 14, 16, 18, 20, 21, and optionally 22 in the direction from the 5' end to the 3' end of the antisense strand, and the nucleotides at positions 2, 6, 14 and 16 are all 2'-fluoro nucleotides.

**14.** The siRNA of any one of claims 1-12, wherein the antisense strand has the following modified nucleotides:

(1) the nucleotides at positions 2, 6, 7, 14, 16, and 22 in the direction from the 5' end to the 3' end of the antisense strand are 2'-fluoro nucleotides; the nucleotides at other positions of the antisense strand may be 2'-methoxy nucleotides; the nucleotide at position 1 in the direction from the 5' end to the 3' end of the antisense strand further has an (*E*)-VP modification;
(2) the nucleotides at positions 2, 6, 14, and 16 in the direction from the 5' end to the 3' end of the antisense strand are 2'-fluoro nucleotides; one or two of the nucleotides at positions 4, 8, 9, 10, 18, and 20 in the direction from the 5' end to the 3' end of the antisense strand may further be 2'-fluoro nucleotides; the nucleotides at other positions of the antisense strand are 2'-methoxy nucleotides;
(3) the nucleotides at positions 2, 6, 14, and 16 in the direction from the 5' end to the 3' end of the antisense strand and the nucleotide at position 2 in the direction from the 3' end to the 5' end of the antisense strand are 2'-fluoro nucleotides; one or two of the nucleotides at positions 3, 4, 5, 7, 8, 9, 10, 12, and 20 in the direction from the 5' end to the 3' end of the antisense strand may further be 2'-fluoro nucleotides; the nucleotides at other positions of the antisense strand are 2'-methoxy nucleotides; the nucleotide at position 1 in the direction from the 5' end to the 3' end of the antisense strand may further have an (*E*)-VP modification;
(4) the nucleotides at positions 2, 6, 7, 14, and 16 in the direction from the 5' end to the 3' end of the antisense strand and the nucleotide at position 2 in the direction from the 3' end to the 5' end of the antisense strand are 2'-fluoro nucleotides; the nucleotide at position 10 in the direction from the 5' end to the 3' end of the antisense strand may further be a 2'-fluoro nucleotide; the nucleotides at other positions of the antisense strand are 2'-methoxy nucleotides; the nucleotide at position 1 in the direction from the 5' end to the 3' end of the antisense strand further has an (*E*)-VP modification;
(5) the nucleotides at positions 2, 14, and 16 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, and 9 in the direction from the 5' end to the 3' end of the antisense strand may further be 2'-fluoro nucleotides; the nucleotides at other positions of the antisense strand are 2'-methoxy nucleotides;
(6) the nucleotides at positions 2, 6, and 14 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, 9, and 18 in the direction from the 5' end to the 3' end of the antisense strand may further be a 2'-fluoro nucleotide; the nucleotides at other positions of the antisense strand are 2'-methoxy nucleotides; or

(7) the nucleotides at positions 6, 14, and 16 in the direction from the 5' end to the 3' end of the antisense strand and the nucleotide at position 2 in the direction from the 3' end to the 5' end of the antisense strand are 2'-fluoro nucleotides; one of the nucleotides at positions 4, 8, 9, and 18 in the direction from the 5' end to the 3' end of the antisense strand may further be a 2'-fluoro nucleotide; the nucleotides at other positions of the antisense strand are 2'-methoxy nucleotides.

**15.** The siRNA of any one of claims 1-14, wherein the nucleotides at positions 9, 11, 12, 13, and 15 in the direction from the 3' end to the 5' end of the sense strand are 2'-fluoro nucleotides, the nucleotides at other positions of the sense strand are 2'-methoxy nucleotides, and the nucleotides at positions 19 and 20, as well as the nucleotides at positions 20 and 21 in the direction from the 3' end to the 5' end of the sense strand are linked via phosphorothioate groups; and the nucleotides at positions 2, 6, 7, 14, 16, and 22 in the direction from the 5' end to the 3' end of the antisense strand are 2'-fluoro nucleotides, the nucleotides at other positions of the antisense strand are 2'-methoxy nucleotides, the nucleotide at position 1 in the direction from the 5' end to the 3' end of the antisense strand further has an (*E*)-VP modification, and the nucleotides at positions 1 and 2, the nucleotides at positions 2 and 3, the nucleotides at positions 21 and 22, as well as the nucleotides at positions 22 and 23 in the direction from the 5' end to the 3' end of the antisense strand are linked via phosphorothioate groups.

**16.** The siRNA of any one of claims 1-12, wherein the siRNA is at least one selected from modified duplex 46 to modified duplex 50, modified duplex 60 to modified duplex 125, modified duplex 129 to modified duplex 130, modified duplex 133 to modified duplex 149, and modified duplex 152 to modified duplex 201.

**17.** The siRNA of claim 16, wherein the siRNA is at least one selected from modified duplex 46, modified duplex 61 to modified duplex 64 and modified duplex 66 to modified duplex 116, modified duplex 117 to modified duplex 125, modified duplex 129 to modified duplex 130, modified duplex 133 to modified duplex 136, modified duplex 155 to modified duplex 157, modified duplex 162, modified duplex 166, modified duplex 171 to modified duplex 172, and modified duplex 181 to modified duplex 198.

**18.** An siRNA conjugate, wherein the siRNA conjugate is obtained by conjugating the siRNA of any one of claims 1-17 with a conjugate molecule.

**19.** The siRNA conjugate of claim 18, wherein the conjugate molecule comprises:

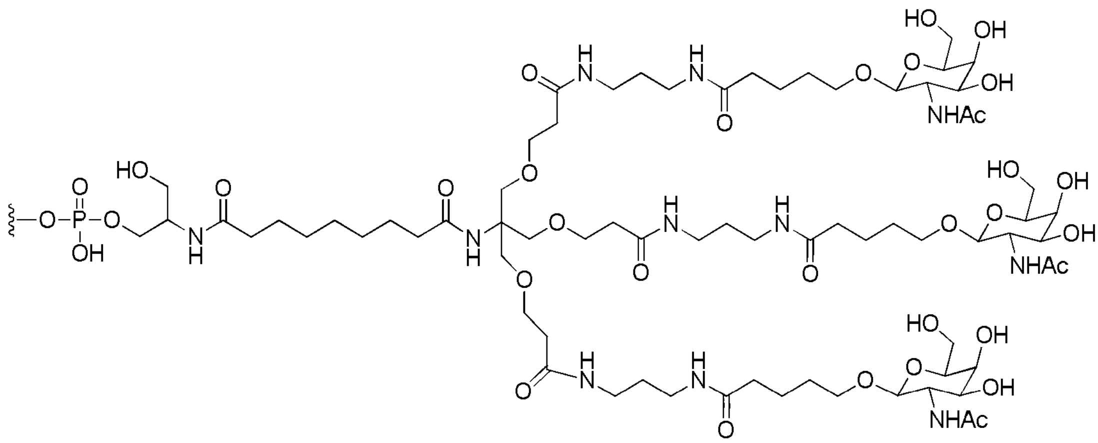

;

or

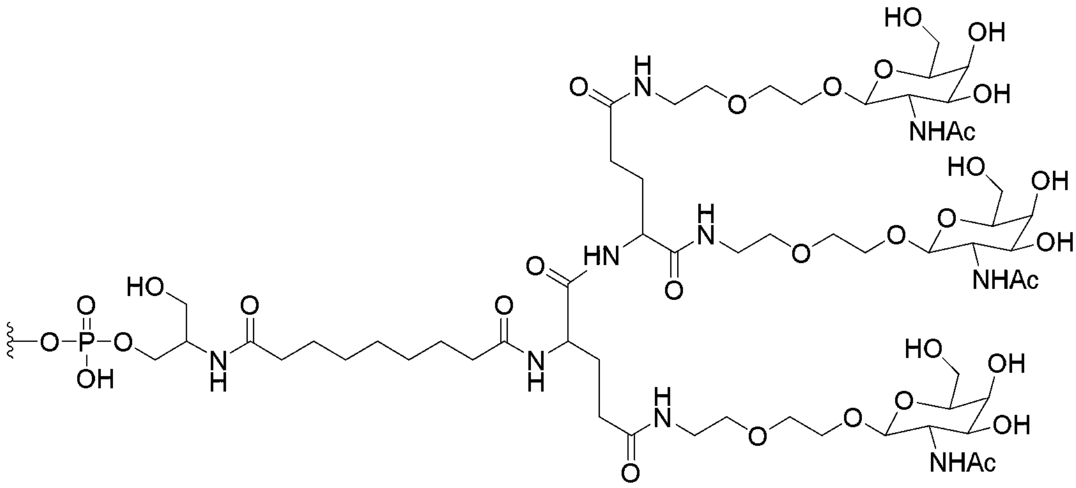

;

or

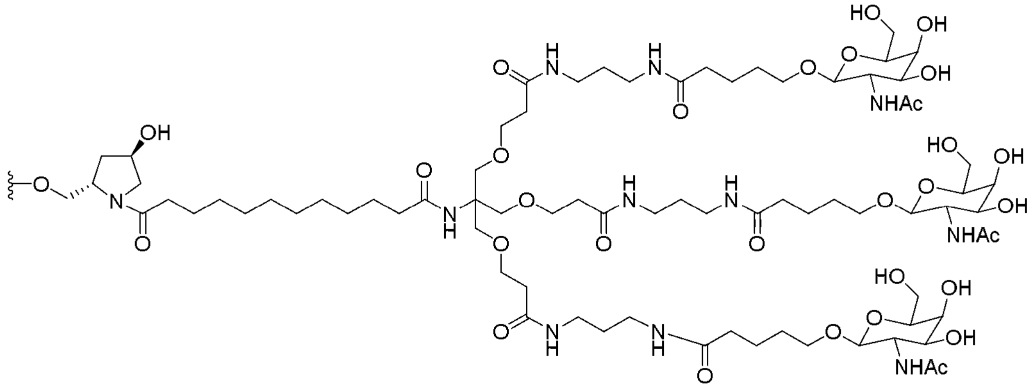

;

or

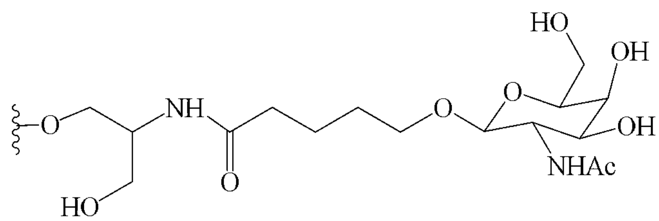

;

preferably, the structure of the conjugate is as follows:

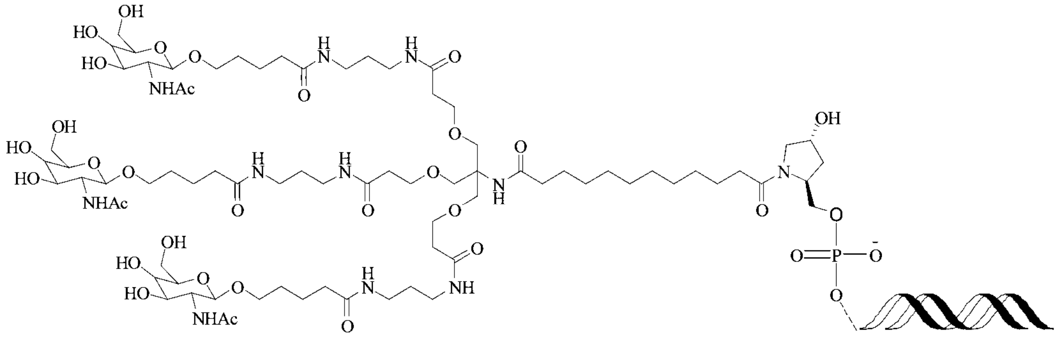

,

wherein the conjugate molecule is covalently conjugated to the 3' end of the sense strand of the siRNA in the manner shown in the above formula.

**20.** The siRNA conjugate of claim 18 or 19, wherein the siRNA conjugate is at least one selected from conjugate 46 to conjugate 50, conjugate 60 to conjugate 125, conjugate 129 to conjugate 130, conjugate 133 to conjugate 136, conjugate 150 to conjugate 151, conjugate 155 to conjugate 204; preferably, the siRNA conjugate is at least one selected from conjugate 150, conjugate 151, conjugate 155, conjugate 156, conjugate 157, conjugate 162, conjugate 166, conjugate 171, conjugate 172, conjugate 175, conjugate 181, conjugate 182, conjugate 183, conjugate 184, conjugate 185; more preferably, the siRNA conjugate is at least one selected from conjugate 155, conjugate 156, conjugate 157, conjugate 162, conjugate 171, conjugate 172, conjugate 175, conjugate 182, conjugate 183 and conjugate 184.

**21.** The siRNA conjugate of claim 18 or 19, wherein the siRNA conjugate comprises a sense strand and an antisense strand, the antisense strand comprises the following sequence in which all nucleotides are modified nucleotides: UACUCAUUAGAAGAAAAGG$X_1$GGG (SEQ ID NO: 396)

wherein, $X_1$ is A or U,
the nucleotides at positions 2, 6, 14, 16, and 22 in the direction from the 5' end to the 3' end of the antisense strand are 2'-fluoro nucleotides; the nucleotide at position 7 or the nucleotides at positions 7 and 10 in the direction from the 5' end to the 3' end of the antisense strand may further be 2'-fluoro nucleotides; the nucleotides at other positions of the antisense strand are 2'-methoxy nucleotides;
the nucleotide at position 1 in the direction from the 5' end to the 3' end of the antisense strand further has an (*E*)-VP modification;
phosphorothioate groups exist between the nucleotides at positions 1 and 2, and between the nucleotides at positions 2 and 3 at the 5' end of the antisense strand, as well as between the nucleotides at positions 1 and 2, and between the nucleotides at positions 2 and 3 at the 3' end of the antisense strand.

**22.** The siRNA conjugate of claim 21, wherein the antisense strand comprises the following sequence in the 5' to 3' direction:

(*E*)-VPmU*i2FA*mCmUmCi2FA$X_2$mUmA$X_3$mAmAmGi2FAmAi2FAmAmGmGm$X_4$mG*i2FG*mG,

wherein $X_2$ is mU or i2FU, $X_3$ is mG or i2FG, and $X_4$ is A or U.

**23.** The siRNA conjugate of claim 22, wherein the sense strand comprises the following sequence in the 5' to 3' direction:

mC*m$X_5$*mCmCmUmUi2FUmUi2FCi2FUi2FUmCi2FUmAmAmUmGmAmGmUmAGalNAc(L96),

wherein $X_5$ is A or U.

**24.** A pharmaceutical composition, comprising the siRNA of any one of claims 1-17 or the siRNA conjugate of any one of claims 18-23 as an active ingredient and a pharmaceutically acceptable carrier.

**25.** A kit, comprising at least one of the siRNA of any one of claims 1-17, the siRNA conjugate of any one of claims 18-23, and the pharmaceutical composition of claim 24.

**26.** A method of preventing and/or treating diseases associated with angiotensinogen dysregulation, comprising administering to a subject in need thereof at least one of the siRNA of any one of claims 1-17, the siRNA conjugate of any one of claims 18-23, and the pharmaceutical composition of claim 24, preferably, the diseases associated with angiotensinogen dysregulation are selected from hypertension or related conditions.

**27.** Use of at least one of the siRNA of any one of claims 1-17, the siRNA conjugate of any one of claims 18-23, and the pharmaceutical composition of claim 24 in preparing a medicament for preventing and/or treating diseases associated with angiotensinogen dysregulation, preferably, the diseases associated with angiotensinogen dysregulation are selected from hypertension or related conditions.

**28.** The method of claim 26 or the use of claim 27, wherein the hypertension or related conditions are selected from at least one of borderline hypertension, essential hypertension, secondary hypertension, hypertensive crisis, hypertensive urgency, isolated systolic and diastolic hypertension, pregnancy-related hypertension, diabetic hypertension, resistant hypertension, refractory hypertension, paroxysmal hypertension, renovascular hypertension, Goldblatt's hypertension, ocular hypertension, pulmonary hypertension, portal hypertension, systemic venous hypertension, systolic hypertension and labile hypertension, hypertensive heart disease, and hypertensive nephropathy.

**INTERNATIONAL SEARCH REPORT**

International application No.
**PCT/CN2024/124919**

**A. CLASSIFICATION OF SUBJECT MATTER**

C12N15/113(2010.01)i

According to International Patent Classification (IPC) or to both national classification and IPC

**B. FIELDS SEARCHED**

Minimum documentation searched (classification system followed by classification symbols)

IPC:C12N

Documentation searched other than minimum documentation to the extent that such documents are included in the fields searched

Electronic data base consulted during the international search (name of data base and, where practicable, search terms used)

CNABS, CNTXT, EPTXT, JPTXT, TWTXT, USTXT, VEN, WOTXT, cnki, 万方, Wanfang, ncbi, 百度, Baidu, 百度学术, Baidu Scholar, 中国专利生物序列检索系统, China Patent Biological Sequence Search System, blast, EBI, STN, Uniprot: 成都国为生物医药有限公司, 黄毅, 魏川来, 周伟, 陈友金, 吴月琪, 吕飞龙, dsDNA, 双链寡核苷酸, siRNA, 小干扰RNA, small interfering RNA, AGT, 血管紧张素原, SERPINA8, ANHU, SEQ ID NOs: 24, 36, 46, 48, 50, 52, 54, 56, 58, 60, 62, 64, 66, 68, 70, 72, 74, 76, 78, 80, 82, 84, 86, 92, 94, 96, 98, 100, 102, 104, 106, 109, 111, 113, 115, 117, 119, 121, 123, 125, 127, 129, 131, 133, 135, 137, 139, 141 and 143

**C. DOCUMENTS CONSIDERED TO BE RELEVANT**

| Category* | Citation of document, with indication, where appropriate, of the relevant passages | Relevant to claim No. |
|---|---|---|
| X | CN 112301031 A (ALNYLAM PHARMACEUTICALS, INC.) 02 February 2021 (2021-02-02)<br>claim 1, description, paragraphs [0148]-[0149], [0158] and [0491], tables 4 and 8, and SEQ ID NOs. 308 and 600 | 1-28 (in part) |
| X | CN 112313335 A (ALNYLAM PHARMACEUTICALS, INC.) 02 February 2021 (2021-02-02)<br>claims 1, 4-12 and 51-57, and description, paragraph [0105], table 5, and SEQ ID NOs. 14, 539, 710, 723 and 959 | 1-28 (in part) |
| X | WO 2022159158 A1 (ALNYLAM PHARMACEUTICALS, INC.) 28 July 2022 (2022-07-28)<br>claim 1, description, paragraph [0385], and SEQ ID NOs. 946, 950, 952, 954 and 955 | 1-28 (in part) |
| A | CN 112852809 A (ALNYLAM PHARMACEUTICALS, INC.) 28 May 2021 (2021-05-28)<br>entire document | 1-28 (in part) |

☑ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 January 2025** | **21 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/124919**

**C. DOCUMENTS CONSIDERED TO BE RELEVANT**

| Category* | Citation of document, with indication, where appropriate, of the relevant passages | Relevant to claim No. |
|---|---|---|
| A | CN 114763547 A (SYNERK BIOTECH LIMITED) 19 July 2022 (2022-07-19) entire document | 1-28 (in part) |
| A | CN 101076590 A (SYLENTIS S.A.) 21 November 2007 (2007-11-21) entire document | 1-28 (in part) |

Form PCT/ISA/210 (second sheet) (July 2022)

**INTERNATIONAL SEARCH REPORT**

International application No.
**PCT/CN2024/124919**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No. **PCT/CN2024/124919**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **26, 28 (in part)**
because they relate to subject matter not required to be searched by this Authority, namely:

Claims 26 and 28 (in part) relate to a disease treatment method as defined in PCT Rule 39.1(iv); however, citations and explanations are still provided on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Invention 1: the technical solutions of claims 1-28 (in part) involving SEQ ID NOs: 6, 8, 10, 18, 22, 30, 88 and 90.

Invention 2: the technical solutions of claims 1-28 (in part) involving SEQ ID NOs: 12, 16, 26, 28 and 34.

Invention 3: the technical solutions of claims 1-28 (in part) involving SEQ ID NO: 14.

Invention 4: the technical solutions of claims 1-28 (in part) involving SEQ ID NOs: 20 and 40.

Invention 5: the technical solutions of claims 1-28 (in part) involving SEQ ID NOs: 24, 36, 46, 48, 50, 52, 54, 56, 58, 60, 62, 64, 66, 68, 70, 72, 74, 76, 78, 80, 82, 84, 86, 92, 94, 96, 98, 100, 102, 104, 106, 109, 111, 113, 115, 117, 119, 121, 123, 125, 127, 129, 131, 133, 135, 137, 139, 141, 143.

Invention 6: the technical solutions of claims 1-28 (in part) involving SEQ ID NO: 32.

Invention 7: the technical solutions of claims 1-28 (in part) involving SEQ ID NO: 38.

Invention 8: the technical solutions of claims 1-28 (in part) involving SEQ ID NOs: 42 and 44.

The same technical features among inventions 1-8 lie in: a siRNA used for inhibiting the expression of angiotensinogen, the siRNA containing a sense strand, an antisense strand, etc. For a person skilled in the art, an AGT gene and a protein sequence are well known in the art, and designing a siRNA on the basis of the gene is also a conventional technique in the art; and the prior art and conventional techniques in the art also disclose a double-stranded oligonucleotide for inhibiting the expression of AGT genes in cells, the double-stranded oligonucleotide containing a sense strand and an antisense strand which form a double-stranded region, etc. (for example, CN 114763547 A, publication date: 19 July 2022, abstract, and claims 1-9; CN 101076590 A, publication date: 21 November 2007, claims 1, 8 and 30). Therefore, inventions 1-8 described above do not share a same or corresponding special technical feature, and do not comply with PCT Rule 13.2.

INTERNATIONAL SEARCH REPORT

International application No.
PCT/CN2024/124919

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **the technical solutions of claims 1-28 (in part) involving SEQ ID NOs: 24, 36, 46, 48, 50, 52, 54, 56, 58, 60, 62, 64, 66, 68, 70, 72, 74, 76, 78, 80, 82, 84, 86, 92, 94, 96, 98, 100, 102, 104, 106, 109, 111, 113, 115, 117, 119, 121, 123, 125, 127, 129, 131, 133, 135, 137, 139, 141, 143**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/124919**

| Patent document cited in search report | Publication date (day/month/year) | Patent family member(s) | Publication date (day/month/year) |
|---|---|---|---|
| CN 112301031 A | 02 February 2021 | WO 2015179724 A1 | 26 November 2015 |
| | | WO 2015179724 A8 | 18 February 2016 |
| | | WO 2015179724 A9 | 10 March 2016 |
| | | SG 11201609376 SA | 29 December 2016 |
| | | IL 281638 A | 31 May 2021 |
| | | IL 281638 B1 | 01 March 2023 |
| | | IL 281638 B2 | 01 July 2023 |
| | | BR 122020023687 B1 | 07 March 2023 |
| | | IL 248816 A0 | 31 January 2017 |
| | | IL 248816 B | 29 July 2021 |
| | | JP 2017517511 A | 29 June 2017 |
| | | JP 6811094 B2 | 13 January 2021 |
| | | US 2023123192 A1 | 20 April 2023 |
| | | US 2019298842 A1 | 03 October 2019 |
| | | US 10814007 B2 | 27 October 2020 |
| | | EP 3739048 A1 | 18 November 2020 |
| | | AU 2015264038 A1 | 01 December 2016 |
| | | AU 2015264038 A2 | 22 December 2016 |
| | | AU 2015264038 B2 | 11 February 2021 |
| | | CA 2948381 A1 | 26 November 2015 |
| | | CA 2948381 C | 07 November 2023 |
| | | AU 2021202552 A1 | 27 May 2021 |
| | | AU 2021202552 B2 | 12 December 2024 |
| | | US 2017189541 A1 | 06 July 2017 |
| | | US 10238749 B2 | 26 March 2019 |
| | | BR 112016026950 A2 | 31 October 2017 |
| | | BR 112016026950 B1 | 07 March 2023 |
| | | KR 20170005130 A | 11 January 2017 |
| | | KR 102410687 B1 | 22 June 2022 |
| | | JP 2022104985 A | 12 July 2022 |
| | | JP 7566814 B2 | 15 October 2024 |
| | | MX 2021006053 A | 06 July 2021 |
| | | KR 20220087576 A | 24 June 2022 |
| | | JP 2021063085 A | 22 April 2021 |
| | | JP 7101748 B2 | 15 July 2022 |
| | | NZ 727615 A | 05 July 2024 |
| | | SG 10202104570 TA | 29 June 2021 |
| | | EA 201692370 A1 | 31 March 2017 |
| | | EP 3146049 A1 | 29 March 2017 |
| | | EP 3146049 B1 | 26 February 2020 |
| | | US 2021046187 A1 | 18 February 2021 |
| | | US 11419942 B2 | 23 August 2022 |
| | | MX 2016015126 A | 23 February 2017 |
| | | CA 3215908 A1 | 26 November 2015 |
| | | ZA 201607666 B | 30 January 2019 |
| CN 112313335 A | 02 February 2021 | PE 20210114 A1 | 19 January 2021 |
| | | ES 2967713 T3 | 03 May 2024 |
| | | US 2021310006 A1 | 07 October 2021 |
| | | US 11834661 B2 | 05 December 2023 |
| | | EP 3794122 A1 | 24 March 2021 |

Form PCT/ISA/210 (patent family annex) (July 2022)

**INTERNATIONAL SEARCH REPORT**
**Information on patent family members**

International application No.
**PCT/CN2024/124919**

| Patent document cited in search report | Publication date (day/month/year) | Patent family member(s) | Publication date (day/month/year) |
|---|---|---|---|
| | | EP 3794122 B1 | 09 August 2023 |
| | | CL 2023002870 A1 | 28 June 2024 |
| | | JP 2021522841 A | 02 September 2021 |
| | | JP 7346460 B2 | 19 September 2023 |
| | | IL 278539 B | 01 June 2022 |
| | | MX 2020012048 A | 29 January 2021 |
| | | ZA 202108348 B | 29 November 2023 |
| | | RS 64812 B1 | 29 December 2023 |
| | | IL 292877 A | 01 July 2022 |
| | | IL 292877 B1 | 01 April 2024 |
| | | IL 292877 B2 | 01 August 2024 |
| | | EP 4324520 A2 | 21 February 2024 |
| | | EP 4324520 A3 | 28 August 2024 |
| | | JP 2023182578 A | 26 December 2023 |
| | | CO 2020015314 A2 | 19 March 2021 |
| | | WO 2019222166 A1 | 21 November 2019 |
| | | KR 20210010529 A | 27 January 2021 |
| | | US 2021095290 A1 | 01 April 2021 |
| | | US 11015201 B2 | 25 May 2021 |
| | | CL 2021002326 A1 | 13 May 2022 |
| | | LT 3794122 T | 27 November 2023 |
| | | SG 11202011144 QA | 30 December 2020 |
| | | BR 112020022943 A2 | 17 February 2021 |
| | | TW 202016304 A | 01 May 2020 |
| | | TWI 851574 B | 11 August 2024 |
| | | PT 3794122 T | 22 November 2023 |
| | | CA 3100003 A1 | 21 November 2019 |
| | | FI 3794122 T3 | 07 November 2023 |
| | | HUE 064073 T2 | 28 February 2024 |
| | | AU 2019269418 A1 | 07 January 2021 |
| | | US 2024191236 A1 | 13 June 2024 |
| | | CL 2020002865 A1 | 30 April 2021 |
| | | SI 3794122 T1 | 29 February 2024 |
| | | PH 12020551904 A1 | 21 June 2021 |
| | | HRP 20231412 T1 | 16 February 2024 |
| | | DK 3794122 T3 | 13 November 2023 |
| | | DK 3794122 T5 | 05 August 2024 |
| | | PL 3794122 T3 | 11 March 2024 |
| WO 2022159158 A1 | 28 July 2022 | AU 2021421624 A1 | 31 August 2023 |
| | | MX 2023008469 A | 22 November 2023 |
| | | EP 4281085 A1 | 29 November 2023 |
| | | JP 2024504694 A | 01 February 2024 |
| | | IL 304334 A | 01 September 2023 |
| | | KR 20230134509 A | 21 September 2023 |
| | | CA 3205809 A1 | 28 July 2022 |
| CN 112852809 A | 28 May 2021 | None | |
| CN 114763547 A | 19 July 2022 | None | |
| CN 101076590 A | 21 November 2007 | GB 0418762 D0 | 22 September 2004 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019222166 A1


### Non-patent literature cited in the description


- **WATTS, J.K.** ; **G.F. DELEAVEY** ; **M.J. DAMHA**. Chemically modified siRNA: tools and applications. *Drug Discov Today*, 2008, vol. 13 (19-20), 842-55
- **ANASTASIA KHVOROVA** ; **JONATHAN K. WATTS**. The chemical evolution of oligonucleotide therapies of clinical utility. *Nature Biotechnology*, 2017, vol. 35 (3), 238-248
- Molecular Cloning. Cold Spring Harbor Laboratory Press, 1989